# EUROPEAN PATENT APPLICATION

(11) **EP 2 407 483 A1**
(43) Date of publication of application: **18.01.2012**
(21) Application number: 11171117.2
(22) Date of filing: 12.04.2007
(51) Int. Cl.: C07K 14/47

(54) **Methods of treating, diagnosing or detecting cancers**

(30) Priority: 13.04.2006 US 791871 P
(62) Divisional of application: 07755364.2
(71) Applicant: Novartis Vaccines and Diagnostics, Inc., Emeryville, CA 94662-8097 (US)
(72) Inventor: Jantapour, Mary J., Emeryville, CA 94602-8097 (US); Yu, Guoying K., Emeryville, CA 94062-8097 (US); To, Robert, Emeryville, CA 94602-8097 (US); Chan, Vivien W., Emeryville, CA 94602-8097 (US); Zimmermann, Deborah, Emeryville, CA 94602-8097 (US)
(74) Representative: UEXKÜLL & STOLBERG

(57) **Abstract**

The invention provides, inter *alia,* methods for treating cancer, compositions for treating cancer, and methods and compositions for diagnosing and/or detecting cancer. In particular, the present invention provides compositions and methods for treating, diagnosing and detecting cancers associated with LIV-1 overexpression.

## Description

**FIELD OF THE INVENTION**

The present invention relates generally to the field of oncology. More particularly, the invention relates to methods for treating cancer, compositions for treating cancer, and methods and compositions for diagnosing and/or detecting cancer.

**BACKGROUND OF THE INVENTION**

Cancer is the second leading cause of death in the United States. Although "cancer" is used to describe many different types of cancer, *i.e.* breast, prostate, lung, colon, pancreas, each type of cancer differs both at the phenotypic level and the genetic level. The unregulated growth characteristic of cancer occurs when the expression of one or more genes becomes dysregulated due to mutations, and cell growth can no longer be controlled.

Genes are often classified in two classes, oncogenes and tumor suppressor genes. Oncogenes are genes whose normal function is to promote cell growth, but only under specific ' conditions. When an oncogene gains a mutation and then loses that control, it promotes growth under all conditions. However, it has been found that for cancer to be truly successful the cancer must also acquire mutations in tumor suppressor genes. The normal function of tumor suppressor genes is to stop cellular growth. Examples of tumor suppressors include, p53, p16, p21, and APC, all of which, when acting normally, stop a cell from dividing and growing uncontrollably. When a tumor suppressor is mutated or lost; that brake on cellular growth is also lost, allowing cells to now grow without restraints.

Zinc is necessary for cell growth and is a co-factor for more than 300 enzymes. Zinc is involved in protein, nucleic acid, carbohydrate and lipid metabolism. Zinc also plays an important role in the control of gene transcription, growth, development and differentiation (Vallee and Falchuk (1993) Physiol. Rev. 73, 79-118). In mammals, zinc deficiency can be detrimental, causing stunted growth and serious metabolic disorders (Truong-Tran et al. (2001) Biometals 14, 315-330). An excess of zinc can also be toxic to cells (Koh et al., (1996) Science 272, 1013-1016). Zinc cannot passively diffuse across cell membranes: Accordingly, specific zinc transporter proteins are required to transport zinc into cells. As zinc is essential for cell growth, zinc transporters have an important role in maintaining the cellular balance between apoptosis and cell growth. Aberrations in the balance could lead to cancer (Taylor et al., Biochem. J. (2003) 375, 51-59).

Eukaryotes have many different zinc transporters. To date, the most studied zinc transporters belonged either to the ZIP, or the CDF (cation diffusion family) transporter families. ZIP transporters are situated either on the plasma membrane and act as zinc influx transporters or are situated on the intracellular membranes allowing them to mobilize stores from these structures. The ZIP family consists of at least 86 members and can be divided into four separate subfamilies; ZIP subfamily I (with 1 known human member), ZIP subfamily II (with 3 known human members), gufa subfamily (with 2 known human members), and the LIV-1 subfamily (with 9 known human members) (Gaither and Eide (2001), Biometals 14, 251-270).

LIV-1, also known, as Zip6, is a zinc transporter which has been identified as a gene whose expression is stimulated by estrogen treatment of certain breast cancer cells (Manning et al., (1988), Mol. Cell. Endocrinol. 59, 205-212). LIV-1 has been shown to belong to a subfamily of ZIP (Zrt-, Irt-like proteins) zinc transporters, named LZT (LIV-1. subfamily of ZIP zinc transporters) (Taylor and Nicholson (2003), Biochim. Biophys. Acta Biomembr. 1611; 16-30). These transporters contain a potential metalloprotease motif similar to the motif present in matrix metalloproteases which have a known role in metastasis (Itoh and Nagase, (2002), Essays Biochem. 38, 21-36). LIV-1 mRNA expression shows an association with the spread of breast cancer to the regional lymph nodes. LIV-1 structure reveals that it is histidine-rich and has at least the potential to bind and/or transport Zn2+ ions. (Taylor, (2000), ZUBMB Life 49:249-253.

Vertebrate gastrulation is a critical step in the establishment of body plan. Epithelial-mesenchymal transition (EMT) occurs during gastrulation. EMT is one of the central events of embryonic development, organ and tissue regeneration, and cancer metastasis. Signal transducers and activators of transcription (STATs) mediate biological actions including cell proliferation, differentiation and survival in response to cytokines and growth factors, in a variety of biological processes. STATs are also important in EMT during gastrulation, organogenesis, wound healing and cancer progression. STAT3 has been shown to be activated during zebrafish gastrulation and its activity is essential for gastrulation movements. The molecular mechanisms of STAT's action in EMT, however, have not been fully elucidated. LIV1 1 is a downstream target of STAT3 in EMT and is also important in the nuclear localization of zinc-finger protein Snail, a master regulator of EMT.

To date, however, the role of LIV-1 in cancer has not been fully elucidated. There is a need to identify compositions and methods that modulate LIV-1. The present invention is directed to these, as well as other, important needs.

**SUMMARY OF THE INVENTION**

In some aspects, the present invention provides compositions comprising a LIV-1 modulator and one or more pharmaceutically acceptable carriers. In some embodiments the LIV-1 modulator is an isolated double-stranded RNA (dsRNA). In some embodiments the LIV-1 modulator is an isolated oligonucleotide comprising at least 10 consecutive nucleotides of a sequence of SEQ ID NO:1. In some embodiments the LIV-1 modulator is an antibody that binds an epitope in a domain of LIV-1 selected from the group consisting of the N-terminal extracellular domain of LIV-1, the extracellular domain of LIV-1 between transmembrane domains (TM) 2&3, the extracellular domain of LIV-1 between TM 4&5, the extracellular domain of LIV-1 between TM 6&7, and the C-terminal extracellular-domain of LIV-1. In some embodiments the LIV-1 modulator is a dsRNA, a siRNA, a shRNA or an antisense oligonucleotide.

In some aspects, the present invention provides methods of treating cancer or a cancer symptom in a patient in need thereof comprising administering to the patient a therapeutically effective amount of a LIV-1 modulator.

In some aspects, the present invention provides methods of modulating a LIV-1-related biological activity in a patient comprising administering to the patient an amount of a LIV-1 modulator effective to modulate the LIV-1-related biological activity.

In some aspects, the present invention provides methods of identifying a patient susceptible to LIV-1 therapy comprising detecting the presence or absence of LIV-1 differential expression in a patient sample, administering a therapeutically effective amount of a LIV-1 modulator to the patient if the patient is a candidate for LIV-1 therapy; and administering a conventional cancer therapeutic to the patient if the patient is not a candidate for LIV-1 therapy.

In some aspects, the present invention provides methods of inhibiting growth of cancer cells that express LIV-1 comprising contacting an amount of a LIV-1 modulator effective to inhibit growth of the cells with the cells.

In some aspects, the present invention provides methods of inhibiting a cancer cell phenotype in a population of cells expressing LIV-1 comprising administering to the cell population an amount of a LIV-1 modulator effective to inhibit the cancer cell phenotype.

In some aspects, the present invention provides methods for detecting one or more cancer cells expressing LIV-1 in a sample comprising contacting the sample with a composition comprising a LIV-1 modulator linked to an imaging agent and detecting the localization of the imaging agent in the sample.

In some aspects, the present invention provides methods for inhibiting the interaction of two or more cells, at least one of which cells expresses LIV-1, comprising administering an effective amount of a LIV-1 modulator to a sample comprising the cells.

In some aspects, the present invention provides methods of expressing an anti-LIV-1 antibody in a CHO or myeloma cell. In some embodiments the anti-LIV-1 antibody inhibits one or more LIV-1-related biological activities. In some embodiments the method comprises expressing a nucleic acid encoding the anti-LIV-1, antibody in a CHO or myeloma cell.

In some, aspects, the present invention provides methods of identifying a cancer inhibitor, comprising contacting a cell expressing LIV-1 with a candidate compound and a LIV-1 ligand, and determining whether a LIV-1-related zinc transport activity is inhibited. In some embodiments inhibition of the LIV-1-related zinc transport activity is indicative of at cancer inhibitor.

In some aspects, the present invention provides methods of identifying a cancer inhibitor comprising contacting a cell expressing LIV-1 with a candidate compound and a LIV-1 ligand, and determining whether a downstream marker of LIV-1 is inhibited. In some embodiments inhibition of the downstream marker is indicative of a cancer inhibitor.

In some aspects, the present invention provides methods for determining the susceptibility of a patient to a LIV-1 modulator comprising detecting evidence of differential expression of LIV-1 in said patient's cancer sample. In some embodiments evidence of differential expression of LIV-1 is indicative of the patient's susceptibility to a LIV-1 modulator.

In some aspects, the present invention provides methods of purifying LIV-1 protein from a sample comprising LIV-1 protein comprising providing an affinity matrix comprising a LIV-1 antibody bound to a solid support, contacting the sample with the affinity matrix to form an affinity matrix-LIV-1 protein complex; separating the affinity matrix-LIV-1 protein complex from the remainder of the sample; and releasing LIV-1 protein from the affinity matrix.

In some aspects, the present invention provides methods of delivering a cytotoxic agent or a diagnostic agent to one or more cells that express LIV-1, the method comprising providing the cytotoxic agent or the diagnostic agent conjugated to a LIV-1 antibody or fragment thereof and exposing the cell to the antibody-agent or fragment-agent conjugate.

In some aspects, the present invention provides methods for determining the prognosis of a cancer patient comprising determining the ratio of LIV-1-delta to LIV-1 in a sample of the patient. In some embodiments the ratio of LIV-1-delta to LIV-1 is used to determine the prognosis of the cancer patient.

In some aspects, the present invention provides methods for determining the prognosis of a cancer patient comprising the presence or absence of LIV-1 bound to the plasma membrane of a cell in a sample of the patient. In some embodiments the absence of LIV-1 bound to the plasma membrane of a cell in a sample of the patient indicates a good prognosis for the patient.

In some aspects, the present invention provides compositions comprising a zinc transport protein modulator and one or more pharmaceutically acceptable carriers. In some embodiments the zinc transport protein is an isolated double-stranded RNA (dsRNA). In some embodiments the zinc transport protein is an isolated oligonucleotide comprising at least 10 consecutive nucleotides of a sequence selected from the group consisting of SEQ ID NOS:383-385. In some embodiments the zinc transport protein is an antibody that binds an epitope in a domain of the zinc transport protein selected from the group consisting of the N-terminal extracellular domain, the extracellular domain between transmembrane domains (TM) 2&3, the extracellular domain between TM 4&5, the extracellular domain between TM 6&7, and the C-terminal extracellular domain. In some embodiments the zinc transport protein is the zinc transport protein is SLC39A10, SLC39A11 or SLC39A13.

In some aspects, the present invention provides methods of treating cancer or a cancer symptom in a patient in need thereof comprising administering to the patient a therapeutically effective amount of a zinc transport protein modulators.

In some aspects, the present invention provides methods of modulating a zinc transport protein-related biological activity in a patient comprising administering to the patient an amount of a zinc transport protein modulator effective to modulate the zinc transport protein-related biological activity.

In some aspects, the present invention provides methods of inhibiting growth of cancer cells that express zinc transport protein comprising contacting an amount of a zinc transport protein modulator effective to inhibit growth of the cells with the cells.

In some aspects, the present invention provides methods for detecting one or more cancer cells expressing a zinc transport protein in a sample comprising the sample with a composition comprising a zinc transport protein modulators linked to an imaging agent, and detecting the localization of the imaging agent in the sample.

In some aspects, the present invention provides methods for inhibiting the interaction of two or more cells, at least one of which cells expresses zinc transport protein, comprising administering an effective amount of a zinc transport protein modulator to a sample comprising the cells.

In some aspects, the present invention provides methods of identifying a cancer inhibitor, the cancer characterized by overexpression of a zinc transport protein compared to a control. In some embodiments the methods comprise contacting a cell expressing a zinc transport protein with a candidate compound and a zinc transport protein ligand, and determining whether zinc transport activity is inhibited. In some embodiments inhibition of the zinc transport activity is indicative of a cancer inhibitor.

In some aspects, the present invention provides methods for identifying a cancer inhibitor, the cancer characterized by overexpression of a zinc transport protein compared to a control. In some embodiments the methods comprise contacting a cell expressing zinc transport protein with a candidate compound and a zinc transport protein ligand, and determining whether a downstream marker of the zinc transport protein is inhibited. In some embodiments inhibition of the downstream marker is indicative of a cancer inhibitor.

In some aspects, the present invention provides methods for determining the susceptibility of a patient to a zinc transport protein modulator comprising detecting evidence of differential expression of zinc transport protein in the patient's cancer sample. In some embodiments evidence of differential expression of zinc transport protein is indicative of the patient's susceptibility to a zinc transport protein modulator.

In some aspects, the present invention provides methods of purifying a zinc transport protein from a sample comprising one or more zinc transport proteins, comprising providing an affinity matrix comprising a zinc transport protein antibody bound to a solid support, contacting the sample with the affinity matrix to form an affinity matrix-zinc transport protein complex, separating the affinity matrix-zinc transport protein complex from the remainder of the sample; and releasing zinc transport protein from the affinity matrix.

In some aspects, the present invention provides methods of delivering a cytotoxic agent or a diagnostic agent to one or more cells that express zinc transport protein, the method comprising providing the cytotoxic agent or the diagnostic agent conjugated to a zinc. transport protein antibody or fragment thereof; and exposing the cell to the antibody-agent or fragment-agent conjugate.

These and other.aspects of the present invention will be elucidated in the following detailed description of the invention.

**BRIEF DESCRIPTION OF THE DRAWINGS**

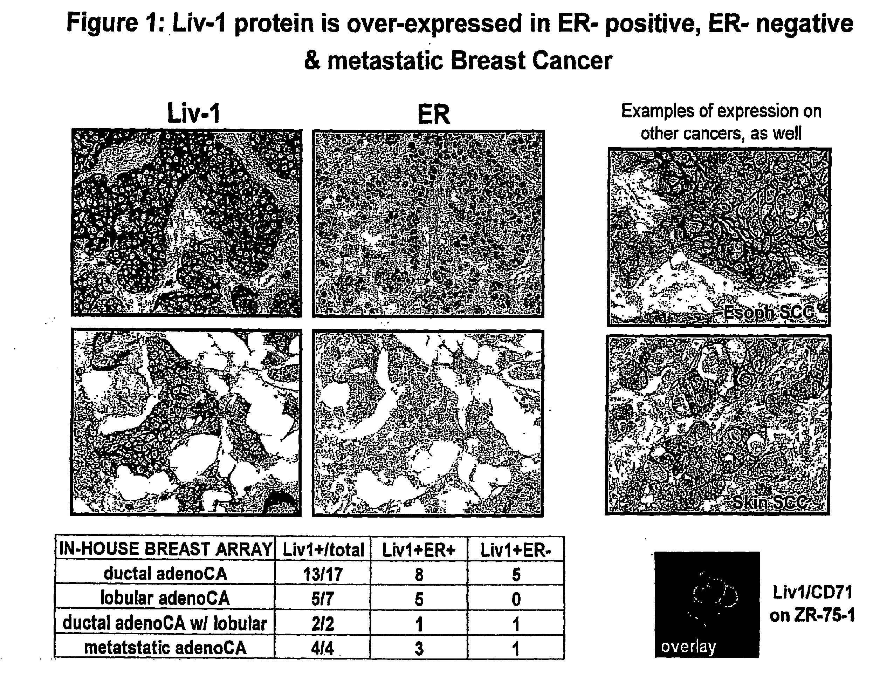

**Figure 1** depicts expression of LIV-1 protein in several cancer samples. Panels on the left depict ER-positive, ER-negative and metastatic breast cancer. Plasma membrane staining is visible in cancer tissues in the panel on the right. The bottom right panel depicts a confocal overlay on non-permeabilized cells.

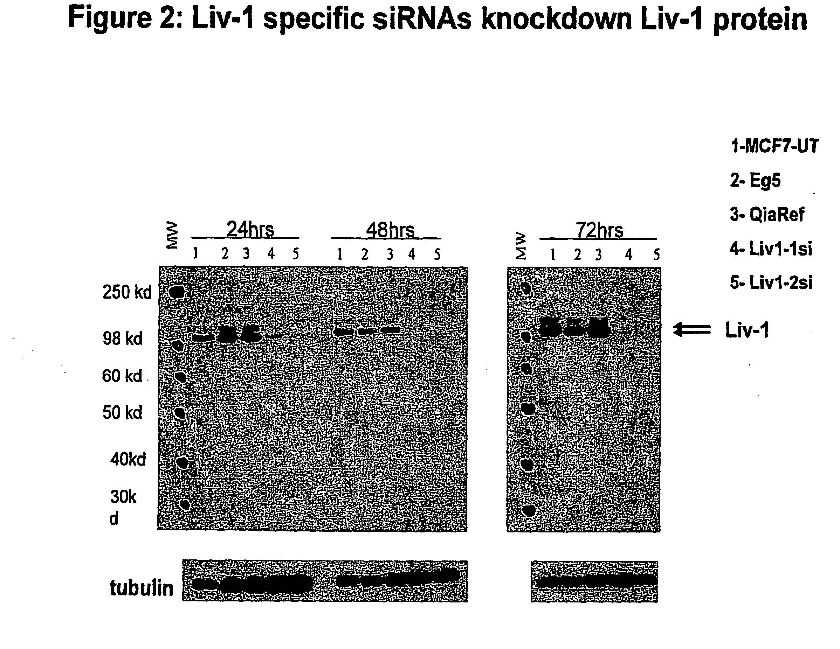

**Figure 2** depicts knockdown of LIV-1 protein by LIV-1 specific siRNAs.

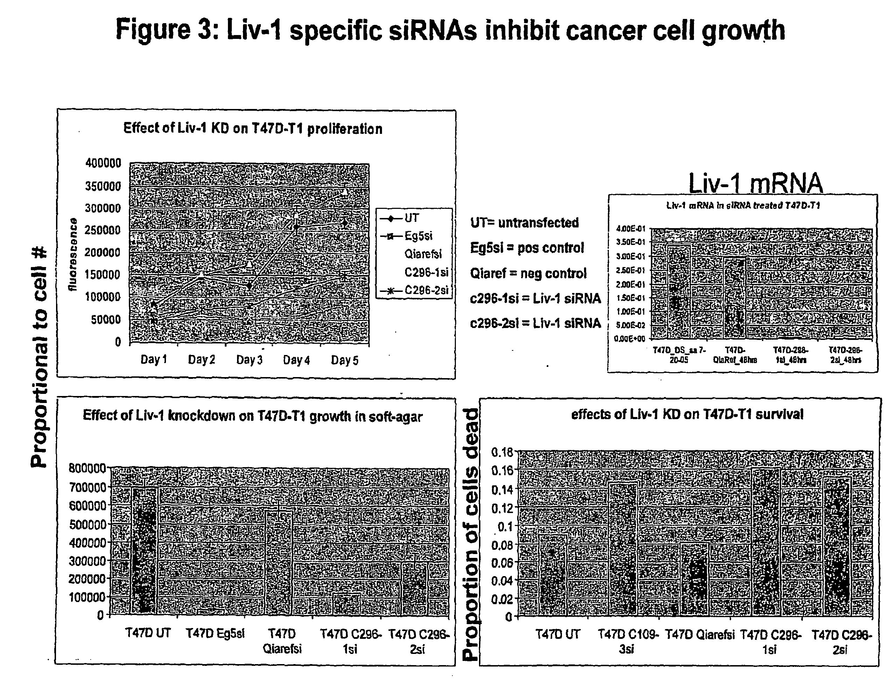

**Figure 3** depicts inhibition of cancer cell growth, proliferation and survival by LIV-1 specific siRNAs in cancer cells.

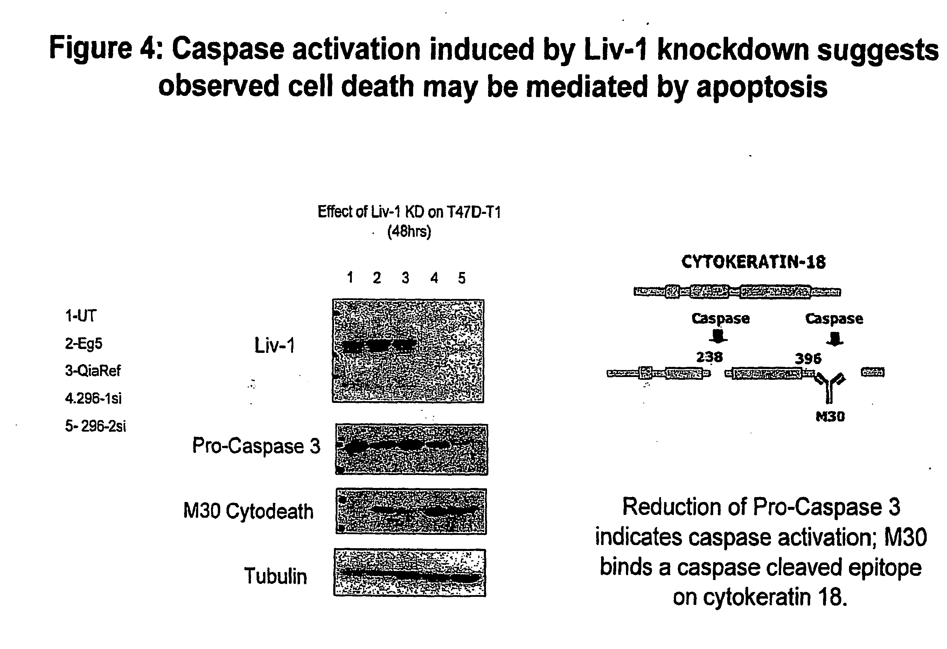

**Figure 4** depicts caspase activation induced by LIV-1 knockdown by LIV-1 specific siRNAs in cancer cells.

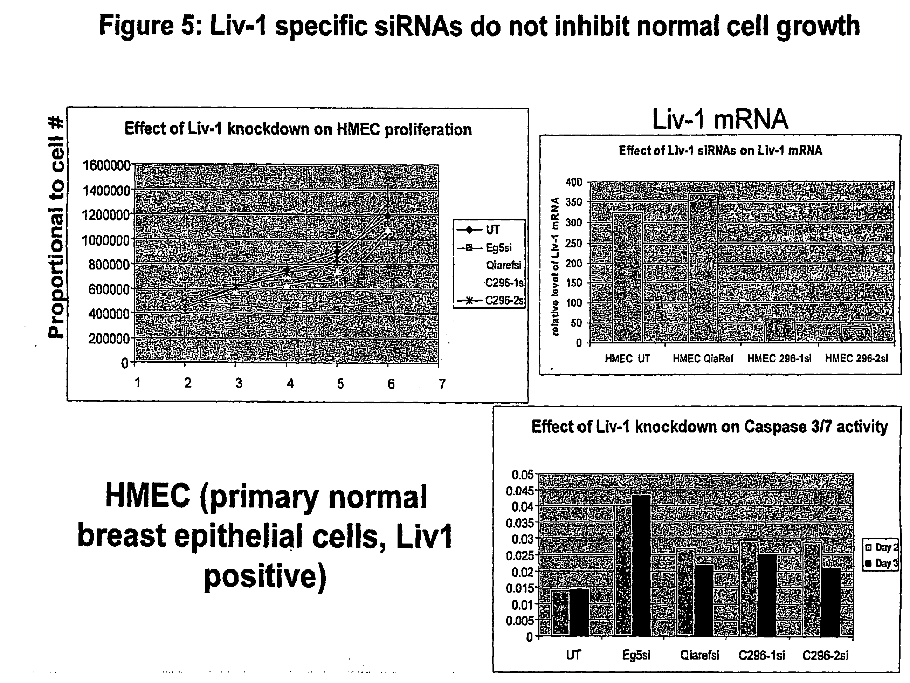

**Figure 5** depicts effect of LIV-1 specific siRNAs on proliferation, caspase activity and levels of LIV-1 mRNA in normal cells.

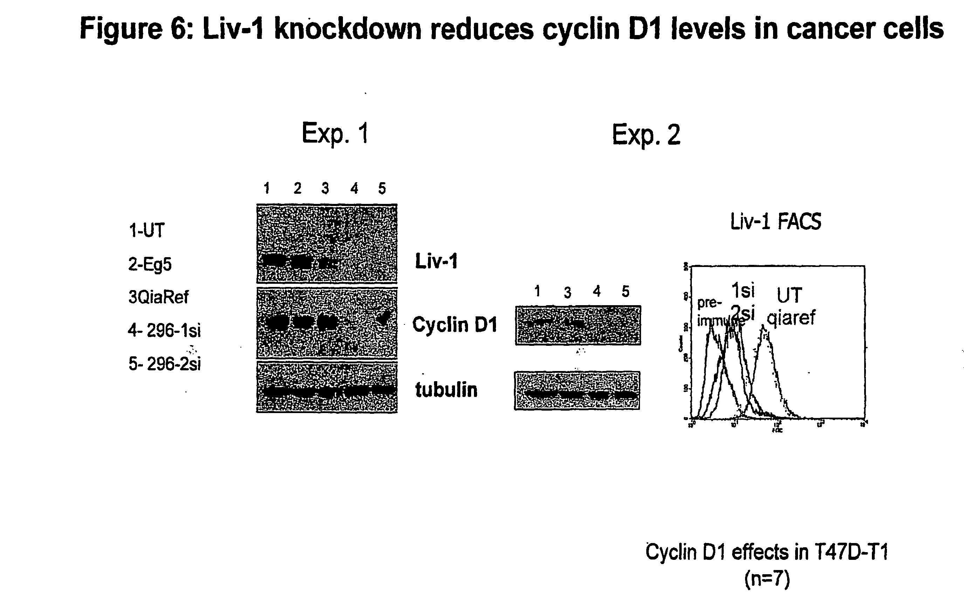

**Figure 6** depicts reduction of cyclin D1 levels in cancer cells by LIV-1 specific siRNAs in cancer cells.

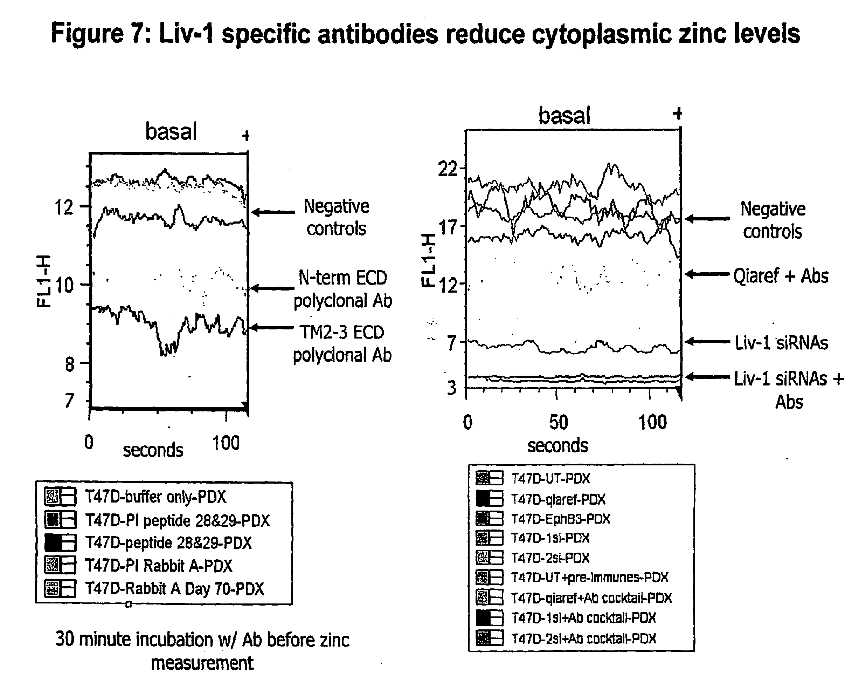

**Figure 7** depicts reduction of cytoplasmic zinc levels by LIV-1 specific antibodies.

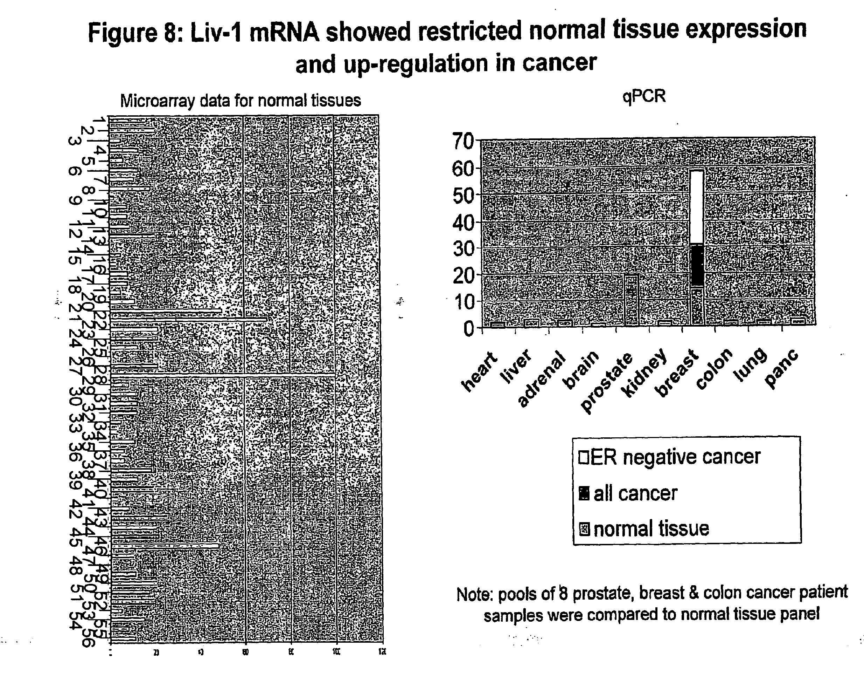

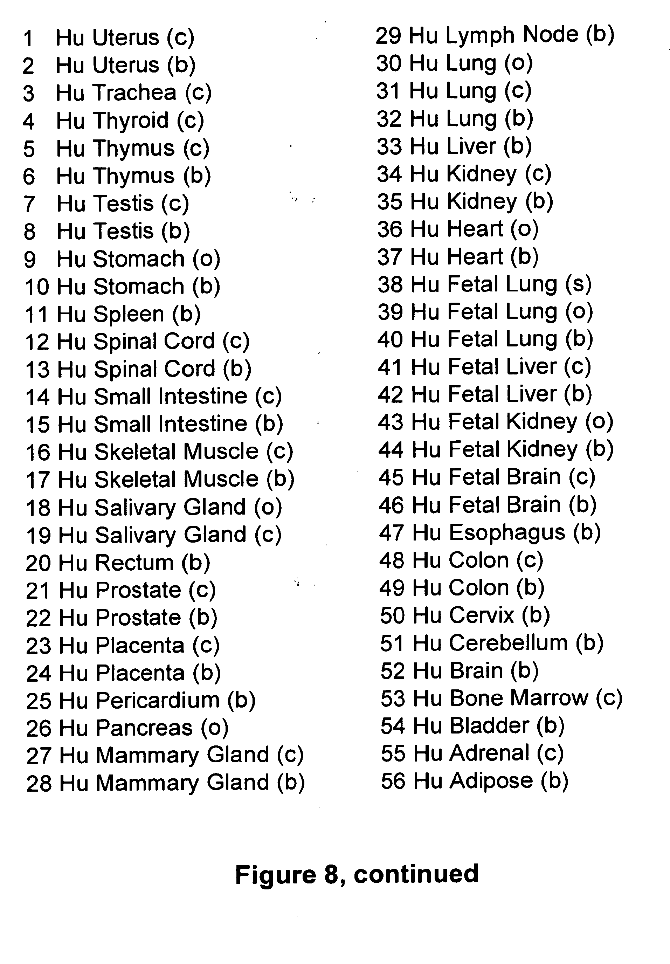

**Figure 8** depicts LIV-1 expression in normal and cancer cells.

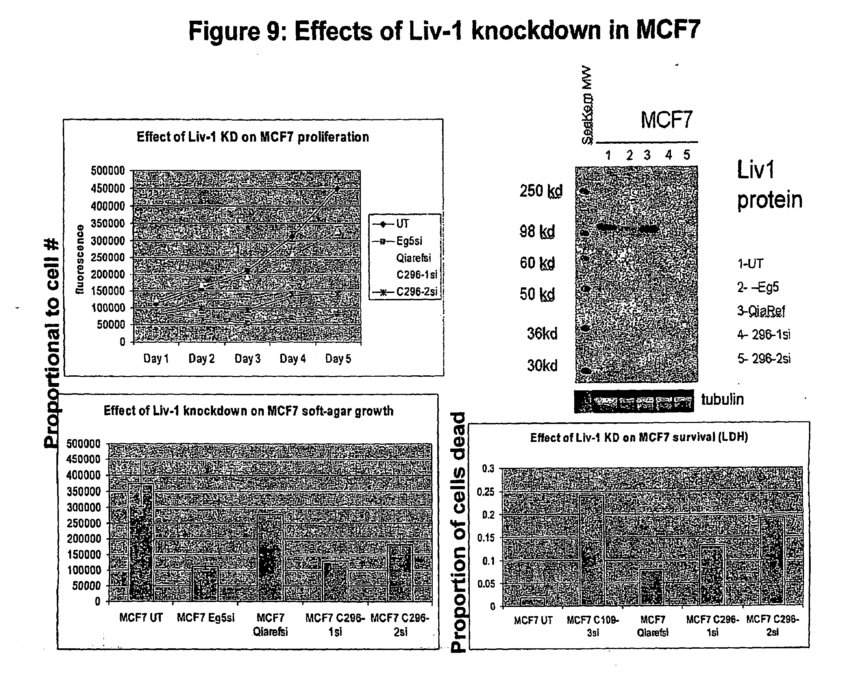

**Figure 9** depicts the effects of LIV-1 knockdown in MCF7 cells including effects on cell proliferation, soft agar growth, protein expression and survival.

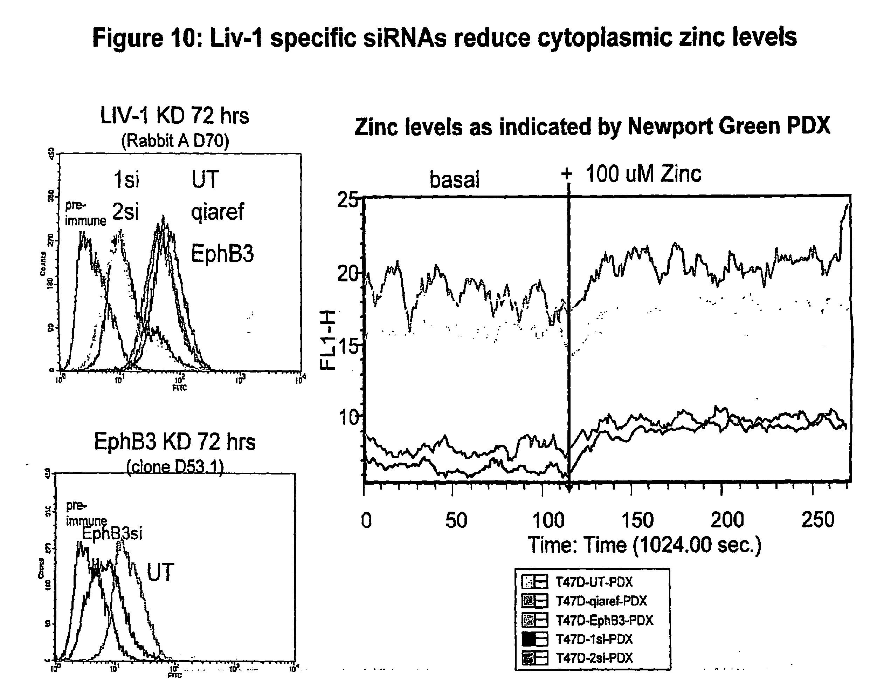

**Figure 10** depicts reduction of cytoplasmic zinc levels by LIV-1 specific siRNAs.

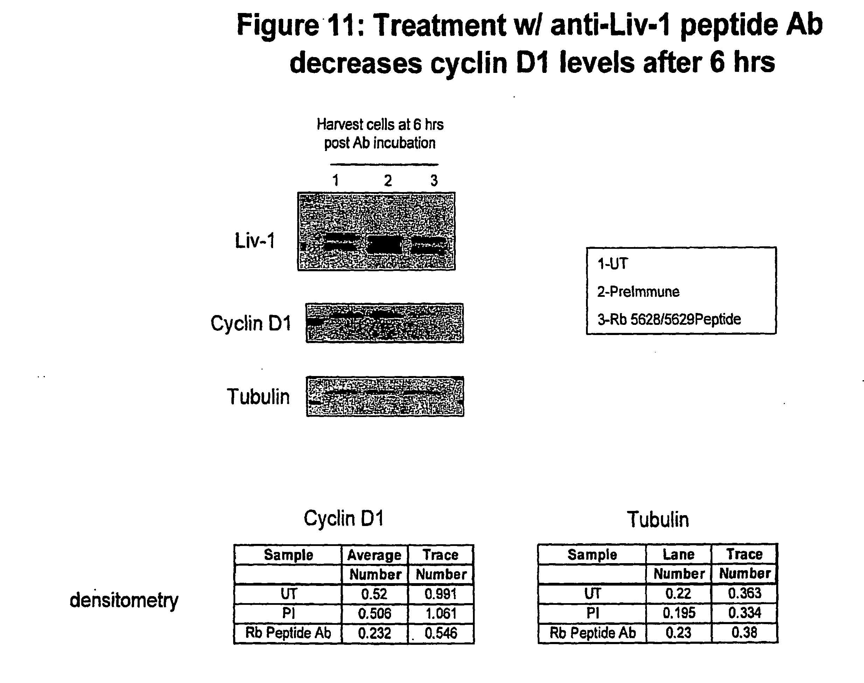

**Figure 11** depicts reduction of cyclin D1 levels following treatment with LIV-1 specific antibodies.

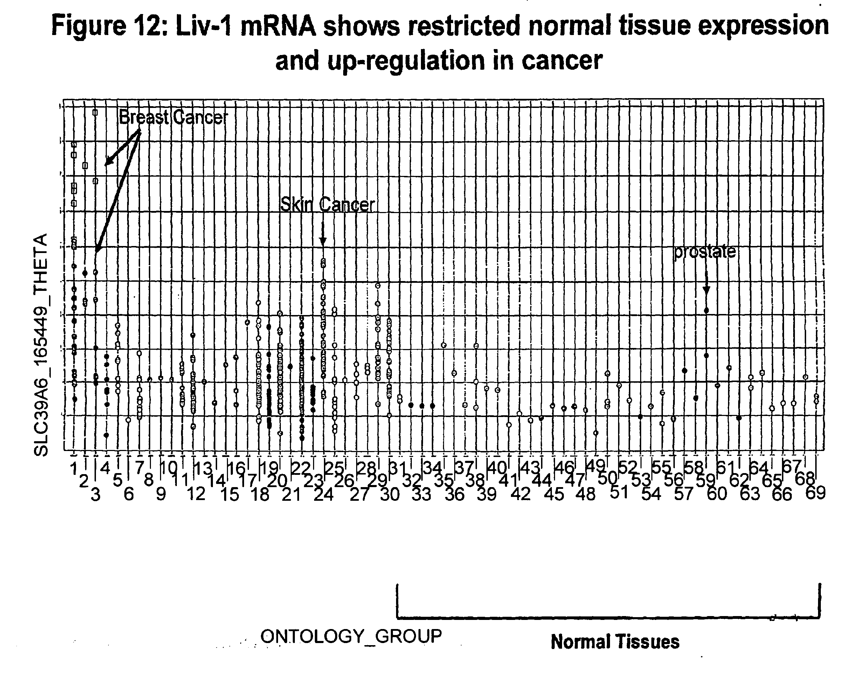

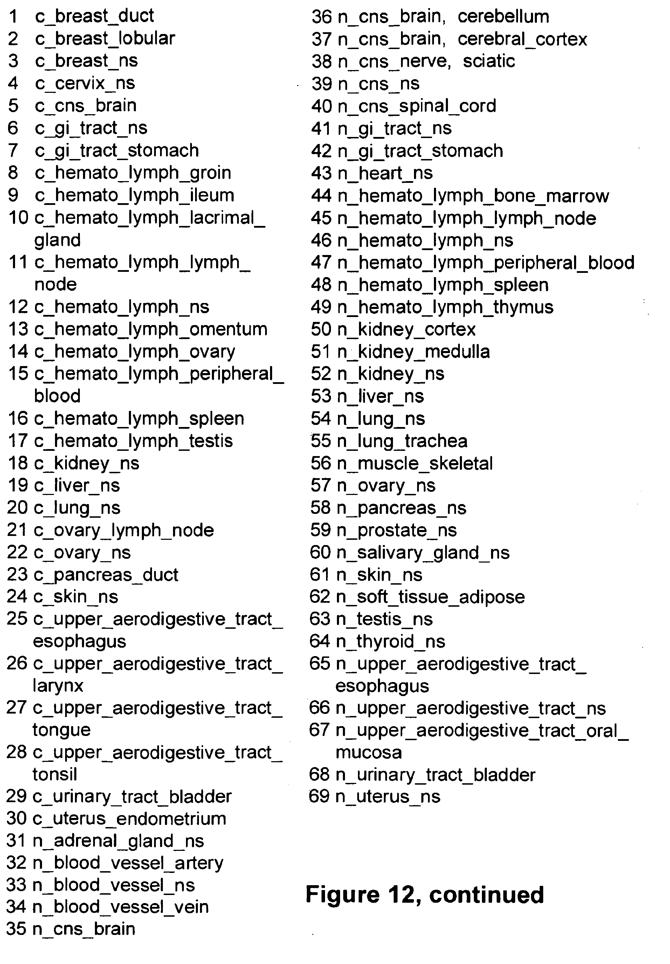

**Figure 12** depicts a graphical representation of the microarray analysis (affy U133 plus 2 chip) of cancerous and normal tissues analyzed using LIV-1. Normal and cancerous tissue types are laid out along the horizontal axis. Cancerous tissues are labeled with a `c', for example, "c_breast_duct" which represents a breast cancer tissue sample and normal tissues are similarly represented with an 'n'. The tissue types are further labeled with respect to the type and subtype of the tissue, if known. For example "c_breast_duct" is a cancerous tissue from a breast cancer that was localized in a breast duct. If the subtype was not clear during surgical removal or was unknown, the label says, 'ns' for 'non-specified'. Each spot on the vertical axes represents a tissue sample from a single patient, and the height of each spot on the vertical axes (log2 based) represents relative expression level of the probeset. Filled circles represent samples with expression levels in the linear detection range. Open circles represent an upper limit on gene expression in samples where the gene was below the probeset's detection limit. Open squares represent a lower limit on gene, expression in samples where the probeset was saturated. (Briefly, before performing an analysis, each probeset is calibrated by analyzing the behavior of its constituent probes across a large, diverse set of samples. This calibration determines the relative sensitivity of each probe, and the range of intensities within which the probeset response is linear between probes. Intensities below this range are called "undetected" while those above it are called "saturated". Because of variation in the hybridization and labeling efficiency between samples, each chip was normalized after applying the calibrations. This causes the upper and lower limits of the range, in terms of gene expression, to vary somewhat from sample to sample.)

**Figure 13** depicts a graphical representation of the microarray analysis (affy U133 plus 2 chip) of cancerous and normal tissues analyzed using SLC39A10. Figure legend information is as provided for Figure 12, above.

**Figure 14** depicts a graphical representation of the microarray analysis (affy U133 plus 2 chip) of cancerous and normal tissues analyzed using SLC39A11. Figure legend information is as provided for Figure 12, above.

**Figure 15** depicts a graphical representation of the microarray analysis (affy U133 plus 2 chip) of cancerous and normal tissues analyzed using SLC39A13. Figure legend information is as provided for Figure 12, above.

**DETAILED DESCRIPTION**

The present invention provides methods and compositions for the treatment, diagnosis and imaging of cancer, in particular for the treatment, diagnosis and imaging of LIV-1 -related cancer.

The inventors of the present application have discovered, *inter alia,* that LIV-1 is overexpressed in several cancers, including breast cancer, and has restricted expression in normal tissues. Inhibition of LIV-1 inhibits proliferation of cancer cells, but not of LIV-1-positive "normal" cells. Further, it has been found that inhibition of LIV-1 modulates, cytoplasmic zinc levels as well as levels of downstream markers including, for example, cyclin D1 and MT1-MMP. These and other aspects of the present invention are provided in the present application.

**Definitions.**

Various definitions are used throughout this document. Most words have the meaning that would be attributed to those Words by one skilled in the art. Words specifically defined either below or elsewhere in this document have the meaning provided in the context of the present invention as a.whole and as are typically understood by those skilled in the art.

The practice of the present invention will employ, unless otherwise indicated, conventional methods of chemistry, biochemistry, molecular biology, immunology and pharmacology, within the skill of the art. Such techniques are explained fully in the literature. See, e.g., Remington's Pharmaceutical Sciences, 18th Edition (Easton, Pennsylvania: Mack Publishing Company, 1990); Methods In Enzymology (S. Colowick and N. Kaplan, eds., Academic Press, Inc.); and Handbook of Experimental Immunology, Vols. I-IV (D.M. Weir and C.C. Blackwell, eds., 1986, Blackwell Scientific Publications); and Sambrook et al., Molecular Cloning: A Laboratory Manual (2nd Edition, 1989).

As used herein, the singular forms "a," "an" and "the" include plural reference unless the content clearly dictates otherwise. Thus, for example, reference to "an antibody includes a mixture of two or more such antibodies.

As used herein, the term "about" refers to +/- 20%, +/- 10%, or +/- 5% of a value.

As used herein, the terms "zinc transport protein" or "zinc transporter" refer to a biological molecule which shows structural characteristics of zinc transporters. In some embodiments zinc transport proteins include LIV-1, SLC39A13, SLC39A11, and SLC39A10.

As used herein, the term "LIV-1", also know as Zip6, refers to a zinc transporter which belongs to a subfamily of ZIP zinc transporters, named LZT. In GeneCard, LIV-1 is also referred to as SLC39A6 (solute carrier family 39 (zinc transporter), member 6). A nucleotide sequence of LIV-1 is set forth as SEQ ID NO:1, and an amino acid sequence of LIV-1 is set forth as SEQ ID NO:2. Although for purposes of brevity; the present invention is exemplified largely in terms of LIV-1, it is understood that definitions and embodiments directed to LIV-1 may also be used for the other zinc transporters disclosed herein.

As used herein, the term "LIV-1-delta" refers to a variant of LIV-1 lacking at least a portion of the amino terminus as compared to LIV-1. A polypeptide sequence of LIV-1-delta is set forth as SEQ ID NO:365.

The terms "polypeptide" and "protein", are used interchangeably and refer to a polymeric form of amino acids of any length, which can include coded and non-coded amino acids, chemically or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues; immunologically tagged proteins; and the like.

The terms "individual", "subject", "host" and "patient" are used interchangeably and refer to any subject for whom diagnosis, treatment, or therapy is desired, particularly humans. Other subjects may include cattle, dogs, cats, guinea pigs, rabbits, rats, mice, horses, and the like. In some preferred embodiments the subject is a human.

As used herein, "cancer" refers to primary or metastatic cancers. The term "cancer cells" refers to cells that are transformed. These cells can be isolated from a patient who has cancer, or be cells that are transformed *in vitro* to become cancerous. Cancer cells can be derived from many types of samples including any tissue or cell culture line. In some embodiments the cancer cells are hyperplasias, tumor cells, or neoplasms. In some embodiments, the cancer cells are isolated from breast cancer, skin cancer, esophageal cancer, liver cancer, pancreatic cancer, prostatic cancer, uterine cancer, cervical cancer, lung cancer, bladder cancer, ovarian cancer, multiple myeloma and melanoma. In some embodiments, the cancer cells are taken from established cell lines that are publicly available. In some embodiments, cancer cells are isolated from pre-existing patient samples or from libraries comprising cancer cells. In some embodiments, cancer cells are isolated and then implanted in a different host, e.g., in a xenograft. In some embodiments cancer cells are transplanted and used in a SCID mouse model. In some embodiments, the cancer is breast cancer.

As used herein, the term "transformed" refers to any alteration in the properties of a cell that is stably inherited by its progeny. In some embodiments, "transformed" refers to the change of normal cell to a cancerous cell, e.g., one that is capable of causing tumors. In some embodiments, a transformed cell is immortalized. Transformation can be caused by a number of factors, including overexpression of a receptor in the absence of receptor phosphorylation, viral infection, mutations in oncogenes and/or tumor suppressor genes, and/or any other technique that changes the growth and/or immortalization properties of a cell.

"Cancerous phenotype" generally refers to any of a variety of biological phenomena that are characteristic of a cancerous cell, which phenomena can vary with the type of cancer. The cancerous phenotype is generally identified by abnormalities in, for example, cell growth or proliferation (e.g., uncontrolled growth or proliferation), regulation of the cell cycle, cell mobility, cell-cell interaction, or metastasis, or the like.

As used herein, the term "metastasis" refers to a cancer which has spread to a site distant from the origin of the cancer, e.g. from the primary tumor. Sites of metastasis include without limitation, the bone, lymph nodes, lung, liver, and brain..

As used herein, the term "angio genesis" refers to the development of blood vessels in a patient.

As used herein, the term "clinical endpoint" refers to a measurable event indicative of cancer. Clinical endpoints include without limitation, time to first metastasis, time to subsequent metastasis, size and/or number of metastases, size and/or number of tumors, location of tumors, aggressiveness of tumors, quality of life, pain and the like. Those skilled in the art are credited with the ability to determine and measure clinical endpoints. Methods of measuring clinical endpoints are known to those of skill in the art.

As used herein, the term "sample" refers to biological material from a patient. The sample assayed by the present invention is not limited to any particular type. Samples include, as non-limiting examples, single cells, multiple cells, tissues, tumors, biological fluids, biological molecules, or supernatants or extracts of any of the foregoing. Examples include tissue removed for biopsy, tissue removed during resection, blood, urine, lymph tissue, lymph fluid, cerebrospinal fluid, mucous, and stool samples. The sample used will vary based on the assay format, the detection method and the nature of the tumors, tissues, cells or extracts to be assayed. Methods for preparing samples are well known in the art and can be readily adapted in order to obtain a sample that is compatible with the method utilized.

As used herein, the term "biological molecule" includes, but is not limited to, polypeptides, nucleic acids, and saccharides.

As used herein, the term "modulating" refers to a change in the quality or quantity of a gene, protein, or any molecule that is inside, outside, or on the surface of a cell. The change can be an increase or decrease in expression or level of the molecule. The term "modulates" also includes changing the quality or quantity of a biological function/activity including, without, limitation, cell proliferation, growth, adhesion, cell survival, apoptosis, intracellular signaling, cell-to-cell signaling, and the like.

As used herein, the term "modulator" refers to a composition that modulates one or more physiological or biochemical events associated with cancer. In some embodiments the modulator inhibits one or more biological activities associated with cancer. In some embodiments the modulator is a small molecule, an antibody, a mimetic, a decoy or an oligonucleotide. In some embodiments the modulator acts by blocking ligand binding or by competing for a ligand-binding site. In some embodiments the modulator acts independently of ligand binding. In some embodiments the modulator does not compete for a ligand binding site. In some embodiments the modulator blocks expression of a gene product involved in cancer. In some embodiments the modulator blocks a physical interaction of two or more biomolecules involved in cancer. In some embodiments modulators of the invention inhibit one or more LIV-1 biological activities selected from the group consisting of cancer cell growth, tumor formation, cancer cell proliferation, cancer cell survival, cancer cell metastasis, cell migration, angiogenesis, LIV-1 signaling, LIV-1-mediated cell-cell adhesion, cell-cell interaction, LIV-1-mediated cell-cell membrane interaction, LIV-1-mediated cell-extracellular matrix interaction, integrin mediated activities, LIV-1 surface expression, LIV-1-mediated cell-extracellular matrix degradation, EGFR phosphorylation, and Snail nuclear localization. In some embodiments the LIV-1 modulator inhibits LIV-1 expression.

A "gene product" is a biopolymeric product that is expressed or produced by a gene. A gene product may be, for example, an unspliced RNA, an mRNA, a splice variant mRNA, a polypeptide, a post-translationally modified polypeptide, a splice variant polypeptide etc. Also encompassed by this term are biopolymeric products that are made using an RNA gene product as a template (i.e. cDNA of the RNA). A gene product may be made enzymatically, recombinantly, chemically, or within a cell to which the gene is native. In some embodiments, if the gene product is proteinaceous, it exhibits a biological activity. In some embodiments, if the gene product is a nucleic acid, it can be translated into a proteinaceous gene product that exhibits a biological activity.

"Modulation of LIV-1 activity", as used herein, refers to an increase or decrease in LIV-1 activity that can be a result of, for example, interaction of an agent with a LIV-1 polynucleotide or polypeptide, inhibition of LIV-1 transcription and/or translation (e.g., through antisense or siRNA interaction with the LIV-1 gene or LIV-1 transcript, through modulation of transcription factors that facilitate LIV-1 expression), and the like. For example, modulation of a biological activity refers to an increase in a biological activity or a decrease in a biological activity). Modulation of LIV-1 activity that results in a decrease of LIV-1 activity is of particular interest in the present invention. LIV-1 activity can be assessed by means including, without limitation, assaying zinc transport activity, assessing LIV-1 polypeptide levels, or by assessing LIV-1 transcription levels. Comparisons of LIV-1 activity can also be accomplished by measuring levels of a LIV-1 downstream marker, measuring inhibition of LIV-1 signaling, measuring inhibition of LIV-1 mediated cell adhesion, measuring activation of LIV-1 mediated cancer cell apoptosis, measuring inhibition of cancer cell growth, measuring inhibition of tumor formation, measuring inhibition of cyclin production, measuring inhibition of fibronectin production, and measuring inhibition of zinc transport

As used herein, the term "inhibit" refers to a reduction, decrease, inactivation or down-regulation of an activity or quantity. For example, in the context of the present invention, Liv-1 modulators may inhibit one or more of cancer cell growth, tumor formation, cancer cell proliferation, cancer cell survival, cancer cell metastasis, cell migration, angiogenesis, LIV-1 signaling, LIV-1-mediated cell-cell adhesion, cell-cell interaction, LIV-1-mediated cell-cell membrane interaction, LIV-1-mediated cell-extracellular matrix interaction, integrin mediated activities, LIV-1 surface expression, LIV-1-mediated cell-extracellular matrix degradation, Snail nuclear localization, and LIV-1 expression. LIV-1 modulators may also inhibit cyclin D1, fibronectin, RhoB, MT1-MMP, FGF, CDK4, VEGF, EGFR, EGFR phosphorylation, one or more genes in the SNAIL pathway. LIV-1 modulators may also inhibit zinc transport and, in some embodiments can reduce cytoplasmic zinc levels. Inhibition may be at least 25%, at least 50%, at least 60%, at least 70%, at least 75%, at least 80%, at least 90%, at least 95%, at least 97%, at least 98%, at least 99%, or 100%, as compared to a control.

As used herein, the term "differentially expressed in a cancer cell" and "a polynucleotide that is differentially expressed in a cancer cell" are used interchangeably herein, and refer to a polynucleotide that represents or corresponds to a gene that is differentially expressed in a cancerous cell when compared with a cell of the same cell type that is not cancerous, e.g., mRNA is found at levels at least about 25%, at least about 50% to about 75%, at least about 90%, at least about 1.5-fold, at least about 2-fold, at least about 5-fold, at least about 10-fold, or at least about 50-fold or more, different (e.g., higher or lower). The comparison can be made in tissue, for example, if one is using *in situ* hybridization or another assay method that allows some degree of discrimination among cell types in the tissue. The comparison may also or alternatively be made between cells removed from their tissue source, or between one cell *in situ* and a second cell removed from its tissue source. In some embodiments, the gene is upregulated in the cancer gene as compared to the normal cell.

A LIV-1 associated-cancer is "inhibited" if at least one symptom of the cancer is alleviated, terminated, slowed, or prevented. As used herein, a LIV-1 associated-cancer is also "inhibited" if recurrence or metastasis of the cancer is reduced, slowed, delayed, or prevented.

As used herein, the phrase "inhibits LIV-1 mediated cell adhesion" refers to inhibition or abolition of cell-to-cell adhesion in the presence of a LIV-1 inhibitor wherein at least one cell differentially expresses LIV-1. In this context, LIV-1 mediated cell adhesion can be decreased by LIV-1 inhibitor at least 25%, at least 50%, at least 75%, at least 85%, at least 90%, at least 95%, up to 100% relative to LIV-1 mediated cell adhesion in the absence of a LIV-1 inhibitor. Comparisons of LIV-1 mediated cell adhesion can be accomplished by measuring, for example, by labeling the cells of interest, incubating them with a population of unlabeled cells adhering to a substrate, and washing to separate the adherent from the non-adherent populations. In this manner, cell adhesion is determined by measuring the amount of label retained on the substrate. Examples of assay systems include, but are not limited to labeling with fluorescent probes such as calcein AM, CFMDA (5-chloromethylfluorescein diacetate), 5(6)-CFDA-SE [5-(and-6)-carboxyfluorescein diacetate, succinimidyl ester] and measuring fluorescence in fluorescence plate reader or via flow cytometry.

As used herein, the phrase "increasing cancer cell apoptosis" refers to increasing apoptosis of cancer cells that differentially express LIV-1 in the presence of a LIV-1 inhibitor. In this context, cancer cell apoptosis can be increased by LIV-1 inhibitor at least 25%, at least 50%, at least 75%, at least 85%, at least 90%, at least 95%, up to 100% relative to cancer cell apoptosis in the absence of a LIV-1 inhibitor. Comparisons of cancer cell apoptosis can be accomplished by measuring, for example, DNA fragmentation, caspase activity, loss of mitochondrial membrane potential, increased production of reactive oxygen species (ROS), intracellular acidification, chromatin condensation, phosphatidyl serine (PS) levels at the cell surface, and increased cell membrane permeability.

DNA fragmentation can be measured, for example, with the TUNEL assay (terminal deoxynucleotide transferase dUTP nick end labeling). Commercial versions of the assay are widely available, for example, APO-BrdU™ TUNEL Assay Kit (Invitrogen), APO-DIRECT™ Kit (BD Biosciences Pharmingen) and ApoAlert™ DNA Fragmentation Assay Kit (Clontech, a Takara Bio Company).

Caspase activity can be monitored via fluorogenic, chromogenic and luminescent substrates specific for particular caspases. Commercial assay kits are available for at least caspases 1, 2, 3, 6, 7, 8 and 9. (See, for example, Invitrogen, Chemicon, CalBiochem, BioSource International, Biovision).

Loss of mitochondrial membrane potential can be measured with fluorescent dyes that differentially accumulate in healthy active mitochondria. One non-limiting example is the MitoTracker Red system from Invitrogen.

Production of reactive oxygen species (ROS) can be measured with fluorescent dyes including, for example, H2DCFDA (Invitrogen).

Intracellular acidification can be measured with fluorescent or chromogenic dyes.

Chromatin condensation can be measure with fluorescent dyes including, for example, Hoechst 33342.

Phosphatidyl serine (PS) levels can be measured at the cell surface. For example, Annexin V has a high affinity for PS. Numerous commercially available assays are suitable to monitor the binding of labeled Annexin V to the cell surface.

Cell membrane permeability can be measured using dyes, such as the fluorescent dye, YO-PRO-1 (Invitrogen) which can enter apoptotic, but not necrotic cells.

As used herein, the phrase "inhibits cancer cell growth" refers to inhibition or abolition of cancer cell growth in the presence of a LIV-1 inhibitor wherein the cell differentially expresses LIV-1. In this context, cancer cell growth can be decreased by LIV-1 inhibitor at least 25%, at least 50%, at least 75%, at least 85%, at least 90%, at least 95%, up to 100% relative to cancer cell growth in the absence of a LIV-1 inhibitor. Comparisons of cancer cell growth can be accomplished using, for example, MTT assay (for example, the Vybrant® MTT Cell Proliferation Assay Kit (Invitrogen)); BrdU incorporation (for example, the Absolute-S SBIP assay (Invitrogen)); measuring intracellular ATP levels (for example using ATPLite™-M, 1,000 Assay Kit (PerkinElmer) or ATP Cell Viability Assay Kit (BioVision)); DiOc18 assay, a membrane permeable dye (Invitrogen);Glucose-6-phosphate dehydrogenase activity assay (for example, the Vibrant cytotoxicity assay (Invitrogen)); or measuring cellular LDH activity.

As used herein, the phrase "inhibits tumor formation" refers to inhibition or abolition of tumor formation in the presence of a LIV-1 inhibitor wherein the tumor comprises cells that differentially express LIV-1. In this context, tumor formation can be decreased by a LIV-1 inhibitor at least 25%, at least 50%, at least 75%, at least 85%, at least 90%, at least 95%, and up to 100% relative to tumor formation in the absence of a LIV-1 inhibitor. Comparisons of tumor formation can be accomplished using, for example, cell based assays (for example colony formation in soft agar); *in vivo* models of tumor formation typically relying upon injecting the cells of interest into animals (for example, athymic mice or rats, irradiated mice or rats; inoculation into immunologically privileged sites such as brain, cheek pouch or eye; inoculation of syngeneic animals), and monitoring the size of the mass after a defined time period.

As used herein, the phrase "inhibits cyclin D1" refers to the inhibition or abolition of LIV-1 mediated cyclin production. In this context, LIV-1 mediated cyclin production can be decreased by an inhibitory agent at least 25%, at least 50%, at least 75%, at least 85%, at least 90%, at least 95%, up to 100% relative to LIV-1 mediated cyclin production in the absence of a LIV-1 inhibitor. Comparisons of cyclin production can be accomplished by measuring, for example, cyclin mRNA levels via RT-PCR or northern blotting; cyclin polypeptide levels via immunoblotting, immunoprecipitation or ELISA; or using functional assays, including co-immunoprecipitation assays to measure levels of cyclin that are complexed with cyclin regulators such as cyclin-dependent kinases (CDK's) using for example antibodies that target CDK, p21WAF1, p27 KIP-1; and measuring phosphorylation of cyclins by the CDK's can be assayed through radiolabeling and immunoprecipitation analysis or FRET-based methods, for example, CDK2/Cyclin A Assay Kit (Molecular Devices).

As used herein, the phrase "inhibits EGFR phosphorylation" refers to the inhibition or abolition of LIV-1 mediated EGFR phosphorylation. In this context, LIV-1 mediated EGFR phosphorylation can be decreased by an inhibitory agent at least 25%, at least 50%, at least 75%, at least 85%, at least 90%, at least 95%, up to 100% relative to LIV-1 mediated EGFR phosphorylation in the absence of a LIV-1 inhibitor. Comparisons of EGFR phosphorylation can be assessed using phosphorylation assays known to those of skill in the art.

As used herein, the phrase "inhibits cancer cell survival" refers to the inhibition of survival of cancer cells that express LIV-1. In some embodiments the term refers to effecting apopotosis of cancer cells that express LIV-1. In this context, LIV-1 expressing cancer cell survival can be decreased by an inhibitory agent at least 25%, at least 50%, at least 75%, at least 85%, at least 90%, at least 95%, up to 100% relative to cancer cell survival in the absence of a LIV-1 inhibitor and/or in a normal cell.

As used herein, the phrase "inhibits integrin mediated activities" refers to the inhibition or abolition of activities related to integrins. Integrin mediated activities include, without limitation, cell adhesion, chemotaxis, proliferation, survival, and tubule formation. In this context, LIV-1 mediated integrin activities can be decreased by an inhibitory agent at least 25%, at least 50%, at least 75%, at least 85%, at least 90%, at least 95%, up to 100% relative to LIV-1 mediated integrin activities in the absence of a LIV-1 inhibitor.

As used herein, the phrase "inhibits fibronectin" refers to the inhibition or abolition of LIV-1 mediated fibronectin production. In this context, LIV-1 mediated fibronectin production can be decreased by an inhibitory agent at least 25%, at least 50%, at least 75%, at least 85%, at least 90%, at least 95%, up to 100% relative to LIV-1 mediated fibronectin production in the absence of a LIV-1 inhibitor. Comparisons of fibronectin production can be accomplished by measuring, for example, fibronectin mRNA levels via RT-PCR or northern blotting; fibronectin polypeptide levels via immunoblotting, immunoprecipitation or ELISA (for example the Fibronectin ELISA kit (AMERICAN DIAGNOSTICA; using functional assays to measure cell adhesion to fibronectin coated substrates. See, for example, InnoCyte™ ECM Cell Adhesion Assay, Fibronectin (Calbiochem) and QCM™-FN [Quantitative Cell Migration Assay- Fibronectin (CHEMICON)].

As used herein, the phrase "inhibits zinc transport" refers to the inhibition or abolition of LIV-1 mediated Zinc transport. In this context, LIV-1 mediated Zinc transport can be decreased by an inhibitory agent at least 25%, at least 50%, at least 75%, at least 85%, at least 90%, at least 95%, up to 100% relative to LIV-1 mediated Zinc transport in the absence of a LIV-1 inhibitor. In some embodiments LIV-1 inhibitors decrease cytoplasmic zinc levels. Comparisons of zinc transport and determination of zinc levels can be accomplished using methods known to the art-skilled including, for example, measuring uptake of ⁶⁵Zn into cells or vesicles. (See, Kambe et al., (2002) J. Biol. Chem., 277: 19049-19055); or measuring, uptake of the cell-permeant fluorescent zinc indicator, Newport Green Diacetate (Molecular Probes). Pools of zinc in body fluids and cell-conditioned media can be measured, for example, following the discussions set forth in Zalewski et al. (BioTechniques, . 2006, Volume 40, Number 4: pp 509-520).

As used herein, the phrase "inhibits LIV-1 signaling" refers to decreasing the effect of LIV-1 on downstream members of cellular signaling cascades that include LIV-1. Cellular signaling cascades that include LIV-1 include the SNAIL pathway, among others. In some embodiments, inhibition of LIV-1 signaling up-regulates one or more epithelial markers in the SNAIL pathway. In some embodiments, inhibition of LIV-1 signaling down-regulates one or more mesenchymal markers in the SNAIL pathway. In some embodiments the epithelial markers and/or mesenchymal markers are involved in motility and cell survival. In some embodiments modulation of LIV-1 signaling, modulates Stat3-associated signaling cascades. In some embodiments the elements of the Stat3-associated signaling cascade are distinct than those of the SNAIL-associated signaling cascade. Inhibition of LIV-1 signaling can be determined by measuring polypeptide or polynucleotide levels of downstream members of the cellular signaling pathway. Those of skill in the art are credited with the ability of measuring LIV-1 polypeptide and/or polynucleotide levels. The art-skilled can also measure levels of . LIV-1 downstream markers.

As used herein, the phrase "inhibits cell-cell interaction" refers to reducing or eliminating an interaction between two or more cells that express LIV-1. In some embodiments, the interaction between the cells leads to a cell signal. Cell-cell interaction can be detected via a number of methods known to those of skill in the art, including, without limitation, the observation of membrane exchange between co-cultured, pre-labeled cells, labeled, for example, with different fluorescent membrane stains including PKH26 and PKH67 (Sigma).

A "LIV-1 downstream marker", as used herein, is a gene or activity which exhibits altered level of expression in a cancer tissue or cancer cell compared to its expression level in normal or healthy tissue, or is a property altered in the presence of a LIV-1 modulator (e.g. cytoplasmic zinc levels). In some embodiments, the downstream markers exhibit altered levels of expression when LIV-1 is perturbed with a LIV-1 modulator of the present invention. LIV-1 downstream markers include; without limitation, cyclin D1, fibronectin, RhoB, MT1-MMP, FGF, CDK4, VEGF, EGFR, one or more genes in the SNAIL pathway, E-cadherin, VE-cadherin, Muc-1, claudin, occludin, desmoplakin, caspase, p21, p53, BID (bcl-interacting death agonist), DFF40 (DNA fragmentation factor), and cytokeratin. As discussed above, in some embodiments, a LIV-1 downstream marker is a gene in the Stat3 pathway.

As used herein, the term "up-regulates" refers to an increase, activation or stimulation of an activity or quantity. For example, in the context of the present invention, Liv-1 modulators may increase one or more of E-cadherin, VE-cadherin, Muc-1, claudin, occludin, desmoplakin, caspase, p21, p53, BID (bcl-interacting death agonist), DFF40 (DNA fragmentation factor), and cytokeratin. Up-regulation may be at least 25%, at least 50%, at least 75%, at least 100%, at least 150%, at least 200%, at least 250%, at least 400%, or at least . 500% as compared to a control.

As used herein, the term "N-terminus" refers to the first 10 amino acids of a protein.

As used herein, the term "C-terminus" refers to the last 10 amino acids of a protein.

The term "domain" as used herein refers to a structural part of a biomolecule that contributes to a known or suspected function of the biomolecule. Domains may be coextensive with regions or portions thereof and may also incorporate a portion of a biomolecule that is distinct from a particular region, in addition to all or part of that region.

As used herein, the term "extracellular domain" refers to the portion of a molecule that is outside or external to a cell. In the context of the present invention, an N-terminal extracellular domain refers to the extracellular domain that is present at the N-terminal of the molecule immediately before the first transmembrane domain. In the context of extracellular domain between two transmembrane (TM) domains, for example between TM 2&3, extracellular domain refers to that portion of LIV-1 external to the cell membrane between the second and third transmembrane domains of LIV-1.

As used herein, the term "ligand binding domain" refers to any portion or region of a receptor retaining at least one qualitative binding activity of a corresponding native sequence of LIV-1.

The term "region" refers to a physically contiguous portion of the primary structure of a biomolecule. In the case of proteins, a region is defined by a contiguous portion of the amino acid sequence of that protein. In some embodiments a "region" is associated with a function of the biomolecule.

The term "fragment" as used herein refers to a physically contiguous portion of the primary structure of a biomolecule. In the case of proteins, a portion is defined by a contiguous portion of the amino acid sequence of that protein and refers to at least 3-5 amino acids, at least 8-10 amino acids, at least 11-15 amino acids, at least 17-24 amino acids, at least 25-30 amino acids, and at least 30-45 amino acids. In the case of oligonucleotides, a portion is defined by a contiguous portion of the nucleic acid sequence of that oligonucleotide and refers to at least 9-15 nucleotides, at least 18-30 nucleotides; at least 33-45 nucleotides, at least 48-72 nucleotides, at least 75-90 nucleotides, and at least 90-130 nucleotides. In some embodiments, portions of biomolecules have a biological activity. In the context of the present invention, LIV-1 polypeptide fragments do not comprises the entire LIV-1 polypeptide sequence set forth in SEQ ID NO:2.

As used herein, the phrase "LIV-1-related cells/tumors/samples" and the like refers to cells, samples, tumors or other pathologies that are characterized by differential expression of LIV-1 relative to non-cancerous and/or non-metastatic cells, samples, tumors, or other pathologies. In some embodiments, LIV-1-related cells, samples, tumors or other pathologies are characterized by increased evidence of LIV-1 expression relative to non-metastatic cells, samples, tumors, or other pathologies.

As used herein, the term "antibody" refers to monoclonal and polyclonal antibodies, single chain antibodies, chimeric antibodies, bifunctional/bispecific antibodies; humanized antibodies, human antibodies, and complementary determining region (CDR)-grafted antibodies, that are specific for the target protein or fragments thereof. The term "antibody" further includes *in vivo* therapeutic antibody gene transfer. Antibody fragments, including Fab, Fab', F(ab')2, scFv, and Fv are also provided by the invention.

As used herein, the term "epitope" refers to an antigenic determinant of a polypeptide. In some embodiments an epitope may comprise 3 or more amino acids in a spatial conformation which is unique to the epitope. In some embodiments epitopes are linear or conformational epitopes. Generally an epitope consists of at least 4, at least 6, at least 8, at least 10, and at least 12 such amino acids, and more usually, consists of at least 8-10 such amino acids. Methods of determining the spatial conformation of amino acids are known in the art, and include, for example, x-ray crystallography and 2-dimensional nuclear magnetic resonance.

As used herein, the term "oligonucleotide" refers to a series of linked nucleotide residues. Oligonucleotides include without limitation, antisense and siRNA oligonucleotides. Oligonucleotides comprise portions of a DNA sequence and have at least about 10 nucleotides and as many as about 500 nucleotides. In some embodiments oligonucleotides comprise from about 10 nucleotides to about 50 nucleotides, from about 15 nucleotides to about 30 nucleotides, and from about 20 nucleotides to about 25 nucleotides. Oligonucleotides may be chemically synthesized and can also be used as probes. In some embodiments oligonucleotides are single stranded. In some embodiments oligonucleotides comprise at least one portion which is double stranded. In some embodiments the oligonucleotides are antisense oligonucleotides (ASO). In some embodiments the oligonucleotides are RNAi oligonucleotides, siRNAs or shRNAs.

As used herein, the term "antisense oligonucleotide" refers to an unmodified or modified nucleic acid having a nucleotide sequence complementary to a LIV-1 polynucleotide sequence including polynucleotide sequences associated with the transcription or translation of LIV-1 (e.g., a promoter of a LIV-1 polynucleotide), where the antisense polynucleotide is capable of hybridizing to a LIV-1 polynucleotide sequence. Of particular interest are antisense polynucleotides capable of inhibiting transcription and/or translation of LIV-1 polypeptide-encoding polynucleotide either *in vitro* or *in vivo.*

As used herein, the terms "siRNA oligonucleotides", "RNAi oligonucleotides", "short interfering RNA", or "siRNA" are used interchangeably and refer to oligonucleotides that work through post-transcriptional gene silencing, also known as RNA interference (RNAi). The terms refer to a double stranded nucleic acid molecule capable of RNA interference "RNAi", (see Kreutzer et al., WO 00/44895; Zernicka-Goetz et al. WO 01/36646; Fire, WO 99/32619; Mello and Fire, WO 01/29058). SiRNA molecules are generally RNA molecules but further encompass chemically modified nucleotides and non-nucleotides. SiRNA gene-targeting experiments have been carried out by transient siRNA transfer into cells (achieved by such classic methods as liposome-mediated transfection, electroporation, or microinjection). Molecules of siRNA are 21- to 23-nucleotide RNAs, with characteristic 2- to 3-nucleotide 3'-overhanging ends resembling the RNase III processing products of long double-stranded RNAs (dsRNAs) that normally initiate RNAi.

Effective exploitation of the siRNA pathway to mediate gene silencing depends, in part, on efficient methods of intracellular delivery of siRNA. siRNA molecules tend to be short-lived in the cell, not readily deliverable to cell types that are difficult to transfect and relatively expensive to produce via chemical syntheses. (Jacks et al., (2005) Biotechniques 39: 215-224; Bernards et al., (2006) Nature Methods 3: 701-706)

One method for efficient intracellular delivery of siRNA is the use of short hairpin RNAs, or "shRNAs". shRNAs are single stranded RNA molecules that include two complementary sequences joined by a non-complementary region. In vivo, the complementary sequences anneal to create a double-stranded helix with an unpaired loop at one end. The resulting lollypop-shaped shaped structure is called a stem loop and can be recognized by the RNAi machinery and processed intracellularly into short duplex RNAs having siRNA-like properties.

shRNA can be synthesized in a cell by transcription from a DNA template that has been inserted into a appropriate vector. Useful shRNAs are typically 50-70 nucleotides in length, with two complementary sequences of 19-29 nucleotides separated by a 5-10 nucleotide loop. shRNA construction is generally effected by one of three methods: annealing of complementary oligonucleotides; promoter-based polymerase chain reaction (PCR); or primer extension. Many vector systems employ RNA Pol III promoters; Pol III-mediated transcription is advantageous because it initiates at a well-defined start-site, produces a non-poly (A) containing transcript and Pol III promoters are active in all cell types. (Brummelkamp et al., (2002) Science 296: 550-553; McIntyre, G. and Fanning, G. (2006) BMC Biotechnology 6: 1-8)

shRNA-encoding vector systems provide a renewable intracellular source of gene-silencing reagents that can mediate persistent gene silencing after stable integration of the vector into the host genome. Moreover, the shRNA cassette can be readily inserted into retroviral, lentiviral or adenoviral vectors to facility delivery of shRNA into a broad range of cell types, including nondividing primary cultures. Regulatable versions of shRNA vectors are particularly useful for genetic screens.

As used herein, the term "decoy" refers to a polyeptide comprising at least a portion of a LIV-1 polypeptide capable of binding zinc or a zinc carrier. In some embodiments the decoy is capable of binding a zinc carrier complexed with zinc. In some embodiments the decoy binds a zinc carrier uncomplexed with zinc.

As used herein, the term "therapeutically effective amount" is meant to refer to an amount of a medicament which produces a medicinal effect observed as reduction or reverse in one or more clinical endpoints, growth and/or survival of cancer cell, or metastasis of cancer cells in an individual when a therapeutically effective amount of the medicament is administered to the individual. Therapeutically effective amounts are typically determined by the effect they have compared to the effect observed when a composition which includes no active ingredient is administered to a similarly situated individual. The precise effective amount for a subject will depend upon the subject's size and health, the nature and extent of the condition, and the therapeutics or combination of therapeutics selected for administration. However, the effective amount for a given situation is determined by routine experimentation and is within the judgment of the clinician.

As used herein, the terms "in combination with" or "in conjunction with" refer to administration of the LIV-1 modulators of the invention with other therapeutic regimens.

As used herein, the term "susceptible" refers to patients for whom LIV-1 therapy is an acceptable method of treatment, i.e., patients who are likely to respond positively. Cancer patients susceptible to LIV-1 therapy express high levels of LIV-1 relative to those patients not susceptible to LIV-1 therapy. Cancer patients who are not good candidates for LIV-1 therapy include cancer patients with tumor samples that lack or have lower levels of LIV-1 in or on their cancer cells.

As used herein, the term "detecting" means to establish, discover, or ascertain evidence of an activity (for example, gene expression) or biomolecule (for example, a polypeptide).

As used herein, the phrase "homologous nucleotide sequence or "homologous amino acid sequence," or variations thereof, refers to sequences characterized by a homology, at the nucleotide level or amino acid level, of at least a specified percentage and is used interchangeably with "sequence identity". Homologous nucleotide sequences include those sequences coding for isoforms of proteins. Such isoforms can be expressed in different tissues of the same organism as a result of, for example, alternative splicing of RNA. Alternatively, isoforms can be encoded by different genes. Homologous nucleotide sequences include nucleotide sequences encoding for a protein of a species other than humans, including, but not limited to, mammals. Homologous nucleotide sequences also include, but are not limited to, naturally occurring allelic variations and mutations of the nucleotide sequences set forth herein. Homologous amino acid sequences include those amino acid sequences which contain conservative amino acid substitutions and which polypeptides have the same binding and/or activity.

Percent homology or identity can be determined by, for example, the Gap program (Wisconsin Sequence Analysis Package, Version 8 for UNIX, Genetics Computer Group, University Research Park, Madison WI), using default settings, which uses the algorithm of Smith and Waterman (Adv. Appl. Math., 1981, 2, 482-489). In some embodiments, homology between the probe and target is between about 50% to about 60%. In some embodiments, nucleic acids have nucleotides that are about 60%, about 70%, about 80%, about 85%, about 90%, about 92%, about 94%, about 95%, about 97%, about 98%, about 99% and about 100% homologous to SEQ ID NO:1, or a portion thereof. The present invention further provides partial of full complements of SEQ ID NO:1 or its homologs.

Homology may also be at the polypeptide level. In some embodiments, polypeptides are about 60%, about 70%, about 80%, about 85%, about 90%, about 92%, about 94%, about 95%, about 97%, about 98%, about 99% and about 100% homologous to SEQ ID NO:2, or a portion thereof.

As used herein, the term "probe" refers to nucleic acid sequences of variable length. In some embodiments probes comprise at least about 10 and as many as about 6,000 nucleotides. In some embodiments probes comprise at least 12, at least 14, at least 16, at least 18, at least 20, at least 25, at least 50 or at least 75 consecutive nucleotides. Probes are used in the detection of identical, similar, or complementary nucleic acid sequences. Longer length probes are usually obtained from natural or recombinant sources, are highly specific to the target sequence, and are much slower to hybridize to the target than are oligomers. Probes may be single- or double-stranded and are designed to have specificity in PCR, hybridization membrane-based, in situ hybridization (ISH), fluorescent in situ hybridization (FISH), or ELISA-like technologies.

As used herein, the term "mixing" refers to the process of combining one or more compounds, cells, molecules, and the like together in the same area. This may be performed, for example, in a test tube, petri dish, or any container that allows the one or more compounds, cells, or molecules, to be mixed.

As used herein the term "isolated" refers to a polynucleotide, a polypeptide, an antibody, or a host cell that is in an environment different from that in which the polynucleotide, the polypeptide, or the antibody naturally occurs. Methods of isolating cells are well known to those skilled in the art. A polynucleotide, a polypeptide, or an antibody which is isolated is generally substantially purified.

As used herein, the term "substantially purified" refers to a compound (e.g., either a polynucleotide or a polypeptide or an antibody) that is removed from its natural environment and is at least 60% free, at least 75% free, and at least 90% free from other components with which it is naturally associated.

As used herein, the term "binding" means the physical or chemical interaction between two or more biomolecules or compounds. Binding includes ionic, non-ionic, hydrogen bonds, Van der Waals, hydrophobic interactions, etc. Binding can be either direct or indirect; indirect being through or due to the effects of another biomolecule or compound. Direct binding refers to interactions that do not take place through or due to the effect of another molecule or compound but instead are without other substantial chemical intermediates.

As used herein, the term "contacting" means bringing together, either directly or indirectly, one molecule into physical proximity to a second molecule. The molecule can be in any number of buffers, salts, solutions, etc. "Contacting" includes, for example, placing a polynucleotide into a beaker, microtiter plate, cell culture flask, or a microarray, or the like, which contains a nucleic acid molecule. Contacting also includes, for example, placing an antibody into a beaker, microtiter plate, cell culture flask, or microarray, or the like, which contains a polypeptide. Contacting may take place *in vivo*, *ex vivo,* or *in vitro.*

As used herein, the phrase "stringent hybridization conditions" or "stringent conditions" refers to conditions under which a probe, primer, or oligonucleotide will hybridize to its target sequence, but to a minimal number of other sequences. Stringent conditions are sequence-dependent and will be different in different circumstances. Longer sequences will hybridize with specificity to their proper complements at higher temperatures. Generally, stringent conditions are selected to be about 5°C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present in excess, at Tₘ, 50% of the probes are hybridized to their complements at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (e.g., 10 to 50 nucleotides) and at least about 60°C for longer probes, primers or oligonucleotides. Stringent conditions may also be achieved with the addition of destabilizing agents, such as formamide.

As used herein, the term "moderate stringency conditions" refers to conditions under which a probe, primer, or oligonucleotide.will hybridize to its target sequence, but to a limited number of other sequences. Moderate conditions are sequence-dependent and will be different in different circumstances. Moderate conditions are well-known to the art skilled and are described in, *inter alia,* Manitatis et al. (Molecular Cloning: A Laboratory Manual, Cold Spring Harbor Laboratory; 2nd Edition (December 1989)).

The nucleic acid compositions described herein can be used, for example, to produce polypeptides, as probes for the detection of mRNA in biological samples (e.g., extracts of human cells) or cDNA produced from such samples, to generate additional copies of the polynucleotides, to generate ribozymes or oligonucleotides (single and double stranded), and as single stranded DNA probes or as triple-strand forming oligonucleotides. The probes described herein can be used to, for example, determine the presence or absence of the polynucleotide provided herein in a sample. The polypeptides can be used to generate antibodies specific for a polypeptide associated with cancer, which antibodies are in turn useful in diagnostic methods, prognostic methods, and the like as discussed in more detail herein. Polypeptides are also useful as targets for therapeutic intervention, as discussed in more detail herein. Antibodies of the present invention may also be used, for example, to purify, detect, and target the polypeptides of the present invention, including both *in vitro* and *in vivo* diagnostic and therapeutic methods. For example, the antibodies are useful in immunoassays for qualitatively and quantitatively measuring levels of the polypeptides of the present invention in biological samples. See, e.g., Harlow et al., Antibodies: A Laboratory . Manual, (Cold Spring Harbor Laboratory Press, 2nd ed. 1988). These and other uses are described in more detail below.

As used herein the term "imaging agent" refers to a composition linked to an antibody, small molecule, or probe of the invention that can be detected using techniques known to the art-skilled. As used herein, the term "evidence of gene expression" refers to any measurable indicia that a gene is expressed.

The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which can be administered without undue toxicity. Suitable carriers can be large, slowly metabolized macromolecules such as proteins, polysaccharides, polylactic acids, polyglycolic acids, polymeric amino acids, amino acid copolymers, lipid aggregates and inactive virus particles. Such carriers are well known to those of ordinary skill in the art. Pharmaceutically acceptable carriers in therapeutic compositions can include liquids such as water, saline, glycerol and ethanol. Auxiliary substances, such as wetting or emulsifying agents, pH buffering substances, and the like, can also be present in such vehicles.

Specific examples of cancers that can be treated by the methods and compositions of the present invention include, but are not limited to, LIV-1 associated cancers. As used herein, "LIV-1 associated cancer" refers to a cancer characterized by cells that differentially express LIV-1 relative to non-cancerous cells. The present invention is also applicable to any tumor cell-type where LIV-1 plays a role in cancer cell growth, tumor formation, cancer cell proliferation, cancer cell metastasis, cell migration, angiogenesis, LIV-1 signaling, LIV-1-mediated cell-cell adhesion, cell-cell interaction, LIV-1-mediated cell-cell membrane interaction, LIV-1-mediated cell-extracellular matrix interaction, integrin mediated activities, LIV-1 surface expression, LIV-1-mediated cell-extracellular matrix degradation, Snail nuclear localization, and LIV-1 expression.. In some embodiments, the cancer is breast cancer, skin cancer, esophageal cancer, liver cancer, pancreatic cancer, prostatic cancer, uterine cancer, cervical cancer, lung cancer, bladder cancer, ovarian cancer, multiple myeloma and melanoma. In some embodiments, the cancer is ER-positive breast cancer. In some embodiments, the cancer is ER-negative breast cancer. In some embodiments, such cancers exhibit differential expression of LIV-1 of at least about 25%, at least about 50%, at least about 75%, at least about 100%, at least about 150%, at least about 200%, or at least about 300% as compared to a control.

The present invention provides methods and compositions that provide for the treatment, inhibition, and management of diseases and disorders associated with LIV-1 overexpression as well as the treatment, inhibition, and management of symptoms of such diseases and disorders. Some embodiments of the invention relate to methods and compositions comprising compositions that treat, inhibit or manage cancer including, without limitation, cancer metastases, cancer cell proliferation, cancer cell growth and cancer cell invasion.

The present invention further provides methods including other active ingredients in combination with the LIV-1 modulators of the present invention. In some embodiments, the methods further comprise administering one or more conventional cancer therapeutics to the patient. In some embodiments the methods of the present invention further comprise treating the patient with one or more of chemotherapy, radiation therapy or surgery.

The present invention also provides methods and compositions for the treatment, inhibition, and management of cancer or other hyperproliferative cell disorder or disease that has become partially or completely refractory to current or standard cancer treatment, such as surgery, chemotherapy, radiation therapy, hormonal therapy, and biological therapy.

The invention also provides diagnostic and/or imaging methods using the LIV-1 modulators of the invention, particularly LIV-1 antibodies, to diagnose cancer and/or predict cancer progression. In some embodiments, the methods of the invention provide methods of imaging and localizing tumors and/or metastases and methods of diagnosis and prognosis. In some embodiments, the methods of the invention provide methods to evaluate the appropriateness of LIV-1-related therapy.

**LIV-1 Modulators**

The present invention provides LIV-1 modulators for, *inter alia,* the treatment, diagnosis, detection or imaging of cancer. LIV-1 modulators are also useful in the preparation of medicaments for the treatment of cancer.

In some embodiments, the LIV-1 modulator is an oligonucleotide, a small molecule, a mimetic, a decoy, or an antibody. In some embodiments, the LIV-1 modulator inhibits a LIV-1 biological activity by 25%, 50%, 60%, 70%, 75%, 80%, 90%, 95%, 97%, 98%, 99% or 100%, as compared to a control. In some embodiments, the LIV-1 modulator inhibits LIV-1 expression by at least 25%, 50%, 60%, 70%, 75%, 80%, 90%, 95%, 97%, 98%, 99% or 100%, as compared to a control.

**Antibodies**

In some embodiments the LIV-1 modulator is a monoclonal antibody, a polyclonal antibody, a chimeric antibody, a human antibody, a humanized antibody, a single-chain antibody, or a Fab fragment. The antibody may be labeled with, for example, an enzyme, radioisotope, or fluorophore. In some embodiments the antibody has a binding affinity less than about 1x10⁵Ka for a polypeptide other than LIV-1. In some embodiments, the LIV-1 ' modulator is a monoclonal antibody which binds to LIV-1 with an affinity of at least 1x10⁸Ka.

The invention also provides antibodies that competitively inhibit binding of an antibody to an epitope of the invention as determined by any method known in the art for determining competitive binding using, for example, immunoassays. In some embodiments, the antibody competitively inhibits binding to the epitope by at least 95%, at least 90%,' at least 85%, at least 80%, at least 75%, at least 70%, at least 60%, or at least 50%.

In some embodiments the antibody is a humanized antibody. Humanized antibodies may be achieved by a variety of methods including, for example: (1) grafting the non-human complementarity determining regions (CDRs) onto a human framework and constant region (a process referred to in the art as "humanizing"), or, alternatively, (2) transplanting the entire non-human variable domains, but "cloaking" them with a human-like surface by replacement of surface residues (a process referred to in the art as "veneering"). In the present invention, humanized antibodies will include both "humanized" and "veneered" antibodies. Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995); Jones et al., Nature 321:522-525 (1986); Morrison et al., Proc. Natl. Acad. Sci, U.S.A., 81:6851-6855 (1984); Morrison and Oi, Adv. Immunol., 44:65-92 (1988); Verhoeyer et al., Science 239:1534-1536 (1988); Padlan, Molec. Immun. 28:489-498 (1991); Padlan, Molec. Immunol. 31(3):169-217 (1994); and Kettleborough, C.A. et al., Protein Eng. 4(7):773-83 (1991) each of which is incorporated herein by reference.

Antibodies of the present invention may function through different mechanisms. In some embodiments, antibodies trigger antibody-dependent cellular cytotoxicity (ADCC), a lytic attack on antibody-targeted cells. In some embodiments, antibodies have multiple therapeutic functions, including, for example, antigen-binding, induction of apoptosis, and complement-dependent cellular cytotoxicity (CDC).

In some embodiments, antibodies of the present invention may act as agonists or antagonists of the polypeptides of the present invention. For example, in some embodiments the present invention provides antibodies which disrupt the receptor/ligand interactions with the polypeptides of the invention either partially or fully. In some embodiments antibodies of the present invention bind an epitope disclosed herein, or a portion thereof. In some embodiments, antibodies are provided that modulate ligand activity or receptor activity by at least 95%, at least 90%, at least 85%, at least 80%, at least 75%, at least 70%, at least 60%, or at least 50% compared to the activity in the absence of the antibody.

In some embodiments, LIV-1 antibodies inhibit the Snail pathway and/or inhibit one or more of cyclin D1, fibronectin, RhoB, MT1-MMP, FGF, CDK4, VEGF, EGFR, and EGFR phosphorylation. In some embodiments, LIV-1 antibodies inhibit integrin mediated activities. In some embodiments, LIV-1 antibodies inhibit Snail nuclear localization.

In some embodiments, the LIV-1 antibodies up-regulates one or more of E-cadherin, VE-cadherin, Muc-1, claudin, occludin, desmoplakin, caspase, p21, p53, BID (bcl-interacting death agonist), DFF40 (DNA fragmentation factor), and cytokeratin. In some embodiments, LIV-1 antibodies down-regulate one or more mesenchymal markers in the SNAIL pathway.

In some embodiments the present invention provides neutralizing antibodies. In some embodiments the neutralizing antibodies act as receptor antagonists, i.e., inhibiting either all or a subset of the biological activities of the ligand-mediated receptor activation. In some embodiments the antibodies may be specified as agonists, antagonists or inverse agonists for biological activities comprising the specific biological activities of the peptides of the invention disclosed herein.

The antibodies of the present invention may be used either alone or in combination with other compositions. The antibodies may further be recombinantly fused to a heterologous polypeptide at the N- or C-terminus or chemically conjugated (including covalently and non-covalently conjugations) to polypeptides or other compositions. For example, antibodies of the present invention may be recombinantly fused or conjugated to molecules useful as labels in detection assays and effector molecules such as heterologous polypeptides, drugs; radionuclides, or toxins. See, e.g., PCT. publications WO 92/08495; WO 91/14438; WO 89/12624; U.S. Patent No. 5,314,995; and EP 396,387.

In addition to chimeric and humanized antibodies, fully human antibodies can be derived from transgenic mice having human immunoglobulin genes (see, e.g., U.S. Patent Nos. 6,075,181, 6,091,001, and 6,114,598, all of which are incorporated herein by reference), or from phage display libraries of human immunoglobulin genes (see, e.g. McCafferty et al., Nature, 348:552-554 (1990). Clackson et al., Nature, 352:624-628 (1991), and Marks et al., J. Mol. Biol., 222:581-597 (1991)).

Monoclonal antibodies can be prepared using the method of Kohler et al. (1975) Nature 256:495-496, or a modification thereof. Typically, a mouse is immunized with a solution containing an antigen. Immunization can be performed by mixing or emulsifying the antigen-containing solution in saline, preferably in an adjuvant such as Freund's complete, adjuvant, and injecting the mixture or emulsion parenterally. Any method of immunization known in the art may be used to obtain the monoclonal antibodies of the invention. After immunization of the animal, the spleen (and optionally, several large lymph nodes) are removed and dissociated into single cells. The spleen cells may be screened by applying a cell suspension to a plate or well coated with the antigen of interest. The B cells expressing membrane bound immunoglobulin specific for the antigen bind to the plate and are not rinsed away. Resulting B cells, or all dissociated spleen cells, are then induced to fuse with myeloma cells to form hybridomas, and are cultured in a selective medium. The resulting cells are plated by serial or limiting dilution and are assayed for the production of antibodies that specifically bind the antigen of interest (and that do not bind to unrelated antigens). The selected monoclonal antibody (mAb)-secreting hybridomas are then cultured *in vitro* (e.g., in tissue culture bottles or hollow fiber reactors), or *in vivo* (as ascites in mice).

As an alternative to the use of hybridomas for expression, antibodies can be produced in a cell line such as a CHO or myeloma cell lines, as disclosed in U.S. Patent Nos. 5,545,403; 5,545,405; and 5,998,144; each incorporated herein by reference. Briefly the cell line is transfected with vectors capable of expressing a light chain and a heavy chain, respectively. By transfecting the two proteins on separate vectors, chimeric antibodies can be produced. Immunol. 147:8; Banchereau et al. (1991) Clin. Immunol. Spectrum 3:8; and Banchereau et al. (1991) Science 251:70; all of which are herein incorporated by reference.

Human antibodies can also be produced using techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner, et al. are also available for the preparation of human monoclonal antibodies [Cole et al., Monoclonal Antibodies and Cancer Therapy, Alan R. Liss, p. 77 (1985) and Boerner et al., J. Immunol., 147(1):86 95 (1991)]. Humanized antibodies may be achieved by a variety of methods including, for example: (1) grafting the non-human complementarity determining regions (CDRs) onto a human framework and constant region (a process referred to in the art as "humanizing"), or, alternatively, (2) transplanting the entire non-human variable domains, but "cloaking" them with a human-like surface by replacement of surface residues (a process referred to in the art as "veneering"). In the present invention, humanized antibodies will include both "humanized" and "veneered" antibodies. Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10, 779 783 (1992); Lonberg et al., Nature 368 856 859 (1994); Morrison, Nature 368, 812 13 (1994); Fishwild et al., Nature Biotechnology 14, 845 51 (1996); Neuberger, Nature Biotechnology 14, 826 (1996); Lonberg and Huszar, Intern. Rev. Immunol. 13 65 93 (1995); Jones et al., Nature 321:522-525 (1986); Morrison et al.; Proc. Natl. Acad. Sci, U.S.A., 81:6851-6855 (1984); Morrison and Oi, Adv. Immunol., 44:65-92 (1988); Verhoeyer et al., Science 239:1534-1536 (1988); Padlan, Molec. Immun. 28:489-498 (1991); Padlan, Molec. Immunol. 31(3):169-217 (1994); and Kettleborough, C.A. et al., Protein Eng. 4(7):773-83 (1991) each of which is incorporated herein by reference.

The phrase "complementarity determining region" refers to amino acid sequences which together define the binding affinity and specificity of the natural Fv region of a native immunoglobulin binding site. See, e.g., Chothia et al., J. Mol. Biol. 196:901=917 (1987); Kabat et al., U.S. Dept. of Health and Human Services NIH Publication No. 91-3242 (1991). The phrase "constant region" refers to the portion of the antibody molecule that confers effector functions. In the present invention, mouse constant regions are substituted by human constant regions. The constant regions of the subject humanized antibodies are derived from human immunoglobulins. The heavy chain constant region can be selected from any of the five isotypes: alpha, delta, epsilon, gamma or mu. One method of humanizing antibodies comprises aligning the non-human heavy and light chain sequences to human heavy and light chain sequences, selecting and replacing the non-human framework with a human framework based on such alignment, molecular modeling to predict the conformation of the humanized sequence and comparing to the conformation of the parent antibody. This process is followed by repeated back mutation of residues in the CDR region that disturb the structure of the CDRs until the predicted conformation of the humanized sequence model closely approximates the conformation of the non-human CDRs of the parent non-human antibody. Such humanized antibodies may be further derivatized to facilitate uptake and clearance, e.g, via Ashwell receptors. See, e.g., U.S. Patent Nos. 5,530,101 and 5,585,089 which are incorporated herein by reference.

Humanized antibodies can also be produced using transgenic animals that are engineered to contain human immunoglobulin loci. For example, WO 98/24893 discloses transgenic animals having a human Ig locus wherein the animals do not produce functional endogenous immunoglobulins due to the inactivation of endogenous heavy and light chain loci. WO 91/10741 also discloses transgenic non-primate mammalian hosts capable of mounting an immune response to an immunogen, wherein the antibodies have primate constant and/or variable regions, and wherein the endogenous immunoglobulin-encoding loci are substituted or inactivated. WO 96/30498 discloses the use of the Cre/Lox system to modify the immunoglobulin locus in a mammal, such as to replace all or a portion of the constant or variable region to form a modified antibody molecule. WO 94/02602 discloses non-human mammalian hosts having inactivated endogenous Ig loci and functional human Ig loci. U.S. Patent No. 5,939,598 discloses methods of making transgenic mice in which the mice lack endogenous heavy chains, and express an exogenous immunoglobulin locus comprising one or more xenogeneic constant regions. Antibodies of the present invention can also be produced using human engineering techniques as discussed in U.S. Patent 5,766,886, which is incorporated herein by reference.

Using a transgenic animal described above, an immune response can be produced to a selected antigenic molecule, and antibody-producing cells can be removed from the animal and used to produce hybridomas that secrete human monoclonal antibodies. Immunization protocols, adjuvants, and the like are known in the art, and are used in immunization of, for example, a transgenic mouse as described in WO 96/33735. The monoclonal antibodies can be tested for the ability to inhibit or neutralize the biological activity or physiological effect of the corresponding protein.

Antibodies of the present invention may be administered to a subject via *in vivo* therapeutic antibody gene transfer as discussed by Fang et al. (2005), Nat. Biotechnol. 23, 584-590. For example recombinant vectors can be generated to deliver a multicistronic expression cassette comprising a peptide that mediates enzyme independent, cotranslational self cleavage of polypeptides placed between MAb heavy and light chain encoding sequences. Expression leads to stochiometric amounts of both MAb chains. A preferred example of the peptide that mediates enzyme independent, cotranslational self cleavage is the foot-and-mouth-disease derived 2A peptide.

Fragments of the antibodies are suitable for use in the methods of the invention so long as they retain the desired affinity of the full-length antibody. Thus, a fragment of an anti-LIV-1 antibody will retain the ability to bind to LIV-1. Such fragments are characterized by properties similar to the corresponding full-length anti-LIV-1 antibody, that is, the fragments will specifically bind a human LIV-1 antigen expressed on the surface of a human cell.

In some embodiments, the antibodies bind to one or more epitopes in an extracellular domain of LIV-1. In some embodiments, the antibodies modulate one or more LIV-1 related biological activities. In some embodiments the antibodies inhibit one or more of cancer cell growth, tumor formation, and cancer cell proliferation.

In some embodiments the antibody is a monoclonal antibody which binds to one or more LIV-1 epitopes in a domain selected from the group consisting of the N-terminal extracellular domain of LIV-1, the extracellular domain of LIV-1 between transmembrane domains (TM) 2&3, the extracellular domain of LIV-1 between TM 4&5, the extracellular domain of LIV-1 between TM 6&7, and the C-terminal extracellular domain of LIV-1.

In some embodiments the monoclonal antibody binds to an LIV-1 epitope in the extracellular domain of LIV-1 between TM 2&3. In some embodiments the monoclonal antibody binds to one or more epitopes of SEQ ID NO:388.

In some embodiments the antibodies the monoclonal antibody binds to an LIV-1 epitope in the N-terminal extracellular domain of LIV-1. In some embodiments the monoclonal antibody binds to one or more epitopes of SEQ ID NO:387.

Suitable antibodies according to the present invention can recognize linear or conformational epitopes, or combinations thereof. In some embodiments the antibodies of the present invention bind to epitopes of antigenic regions of LIV-1 selected from the group consisting of SEQ ID NOS:3-6. In some embodiments the antibody is specific for an epitope having a sequence selected from the group consisting of SEQ ID NOS:3-360. In some embodiments the antibody is specific for an epitope having a sequence selected from the group consisting of SEQ ID NOS:361-364 or 387-391. It is to be understood that these peptides may not necessarily precisely map one epitope, but may also contain LIV-1 sequence that is not immunogenic.

Methods of predicting other potential epitopes to which an antibody of the invention can bind are well-known to those of skill in the art and include without limitation, Kyte-Doolittle Analysis (Kyte, J. and Dolittle, R.F., J. Mol. Biol. (1982) 157:105-132), Hopp and Woods Analysis (Hopp, T.P. and Woods, K.R., Proc. Natl. Acad. Sci. USA (1981) 78:3824-3828; Hopp, T.J. and Woods, K.R., Mol. Immunol. (1983) 20:483-489; Hopp, T.J., J. Immunol. Methods (1986) 88:1-18.), Jameson-Wolf Analysis (Jameson, B.A. and Wolf, H., Comput. Appl. Biosci. (1988) 4:181-186.), and Emini Analysis (Emini, E.A., Schlief, W.A., Colonno, R.J. and Wimmer, E., Virology (1985) 140:13-20.).

Antibodies are defined to be "specifically binding" if: 1) they exhibit a threshold level of binding activity, and/or 2) they do not significantly cross-react with known related polypeptide molecules. The binding affinity of an antibody can be readily determined by one of ordinary skill in the art, for example, by Scatchard analysis (Scatchard, Ann. NY Acad. Sci. 51: 660-672, 1949). In some embodiments the antibodies of the present invention bind to their target epitopes or mimetic decoys at least 1.5-fold, 2-fold, 5-fold 10-fold, 100-fold, 10³-fold, 10⁴-fold, 10⁵-fold, 10⁶-fold or greater for the target cancer-associated polypeptide higher than to other known members of the ZIP (Zrt-, Irt-like proteins) zinc transporters.

In some embodiments the antibodies bind with high affinity of 10⁻⁴M or less, 10⁻⁷M or less, 10⁻⁹M or less or with subnanomolar affinity (0.9, 0.8, 0.7, 0.6, 0.5, 0.4, 0.3, 0.2, 0.1 nM or even less). In some embodiments the binding affinity of the antibodies for LIV-1 is at least 1 x 10⁶ Ka. In some embodiments the binding affinity of the antibodies for LIV-1 is at least 5 x 10⁶ Ka, at least 1 x 10⁷ Ka, at least 2 x 10⁷ Ka, at least 1 x 10⁸ Ka, or greater. Antibodies of the present invention may also be described or specified in terms of their binding affinity to a polypeptide of the invention. In some embodiments binding affinities include those with a Kd less than 5 x 10⁻² M, 10⁻² M, 5 x 10⁻³ M, 10⁻³ M, 5 X 10⁻⁴ M, 10⁻⁴ M, 5 x 10⁻⁵ M, 10⁻⁵ M, 5 x 10⁻⁶ M, 10⁻⁶ M, 5 x 10⁻⁷ M, 10⁻⁷ M, 5 x 10⁻⁸ M, 10⁻⁸ M, 5 x 10⁻⁹ M, 10⁻⁹ M, 5 x 10⁻¹⁰ M, 10⁻¹⁰ M, 5 x 10⁻¹¹ M, 10⁻¹¹ M, 5 x 10⁻¹² M, 10⁻¹² M, 5 x 10⁻¹³ M, 10⁻¹³ M, 5 x 10⁻¹⁴ M, 10⁻¹⁴ M, 5 x 10⁻¹⁵ M, or 10⁻¹⁵ M, or less.

In some embodiments, the antibodies of the present invention do not bind to known related polypeptide molecules, for example, if they bind LIV-1 polypeptide but not known related polypeptides using a standard Western blot analysis (Ausubel et al.). Examples of known related polypeptides include, without limitation, other members of the ZIP (Zrt-, Irt-like proteins) zinc transporters protein family, and the like, including, without limitation, SEQ ID NO:365, SEQ ID NO: 366, and SEQ ID NO:386.

In some embodiments, the antibodies of the present invention bind to orthologs, homologs, paralogs or variants, or combinations and subcombinations thereof, of LIV-1 or zinc transport protein polypeptides. In some embodiments, the antibodies of the present invention bind to orthologs of LIV-1- or zinc transport protein polypeptides. In some embodiments, the antibodies of the present invention bind to homologs of LIV-1 or zinc transport protein polypeptides. In some embodiments, the antibodies of the present invention bind to paralogs of LIV-1 or zinc transport protein polypeptides. In some embodiments, the antibodies of the present invention bind to variants of LIV-1 or zinc transport protein polypeptides. In some embodiments, the antibodies of the present invention do not bind to orthologs, homologs, paralogs or variants, or combinations and subcombinations thereof, of LIV-1 or zinc transport protein polypeptides.

In some embodiments, antibodies may be screened against known related polypeptides to isolate an antibody population that specifically binds to LIV-1 polypeptides. For example, antibodies specific to human LIV-1 polypeptides will flow through a column comprising ZIP (Zrt-, Irt-like proteins) zinc transporters proteins (with the exception of LIV-1) adhered to insoluble matrix under appropriate buffer conditions. Such screening allows isolation of polyclonal and monoclonal antibodies non-crossreactive to closely related polypeptides (Antibodies: A Laboratory Manual, Harlow and Lane (eds.), Cold Spring Harbor Laboratory Press, 1988; Current Protocols in Immunology, Cooligan et al. (eds.), National Institutes of Health, John Wiley and Sons, Inc., 1995). Screening and isolation of specific antibodies is well known in the art (see, Fundamental Immunology, Paul (eds.), Raven Press, 1993; Getzoff et al., Adv. in Immunol. 43: 1-98, 1988; Monoclonal Antibodies: Principles and Practice, Goding, J. W. (eds.), Academic Press Ltd., 1996; Benjamin et al., Ann. Rev. Immunol. 2: 67-101, 1984). Representative examples of such assays include: concurrent immunoelectrophoresis, radioimmunoassay (RIA), radioimmunoprecipitation, enzyme-linked immunosorbent assay (ELISA), dot blot or Western blot assay, inhibition or competition assay, and sandwich assay.

In some embodiments the antibodies of the present invention do not specifically bind to SEQ ID NO: 365, SEQ ID NO: 366, or SEQ ID NO:386. In some embodiments, the antibodies of the present invention do not specifically bind to epitopes consisting of residues 125-138 of SEQ ID NO:386, residues 252-265 of SEQ ID NO:386, or residues 418-431 of SEQ ID NO:386. In some embodiments the antibodies do not cross-react with ZnT1 or Zip1.

The invention also provides antibodies that are SMIPs or binding domain immunoglobulin fusion proteins specific for target protein. These constructs are single-chain polypeptides comprising antigen binding domains fused to immunoglobulin domains necessary to carry out antibody effector functions. See e.g., WO03/041600, U.S. Patent publication 20030133939 and US Patent Publication 20030118592.

In some embodiments the antibodies of the present invention are neutralizing antibodies. In some embodiments the antibodies are targeting antibodies. In some embodiments, the antibodies are internalized upon binding a target. In some embodiments the antibodies do not become internalized upon binding a target and istead remain on the surface.

The antibodies of the present invention can be screened for the ability to either be rapidly internalized upon binding to the tumor-cell antigen in question, or for the ability to remain on the cell surface following binding. In some embodiments, for example in the construction of some types of immunoconjugates, the ability of an antibody to be internalized may be desired if internalization is required to release the toxin moiety. Alternatively, if the antibody is being used to promote ADCC or CDC, it may be more desirable for the antibody to remain on the cell surface. A screening method can be used to differentiate these type behaviors. For example, a tumor cell antigen bearing cell may be used where the cells are incubated with human IgG1 (control antibody) or one of the antibodies of the invention at a concentration of approximately 1 µg/mL on ice (with 0.1% sodium azide to block internalization) or 37°C (without sodium azide) for 3 hours. The cells are then washed with cold staining buffer (PBS + 1% BSA + 0.1% sodium azide), and are stained with goat anti-human IgG-FITC for 30 minutes on ice. Geometric mean fluorescent intensity (MFI) is recorded by FACS Calibur. If no difference in MFI is observed between cells incubated with the antibody of the invention on ice in the presence of sodium azide and cells observed at 37°C in the absence of sodium azide, the antibody will be suspected to be one that remains bound to the cell surface, rather than being internalized If however, a decrease in surface stainable antibody is found when the cells are incubated at 37°C in the absence of sodium azide, the antibody will be suspected to be one which is capable of internalization.

**Antibody Conjugates**

In some embodiments, the antibodies of the invention are conjugated. In some embodiments, the conjugated antibodies are useful for cancer therapeutics, cancer diagnosis, or imaging of cancerous cells.

For diagnostic applications, the antibody typically will be labeled with a detectable moiety. Numerous labels are available which can be generally grouped into the following categories:
(a) Radionuclides such as those discussed infra. The antibody can be labeled, for example, with the radioisotope using the techniques described in Current Protocols in Immunology, Volumes 1 and 2, Coligen et al., Ed. Wiley-Interscience, New York, N.Y., Pubs. (1991) for example and radioactivity can be measured using scintillation counting.
(b) Fluorescent labels such as rare earth chelates (europium chelates) or fluorescein and its derivatives, rhodamine and its derivatives, dansyl, Lissamine, phycoerythrin and Texas Red are available. The fluorescent labels can be conjugated to the antibody using the techniques disclosed in Current Protocols in Immunology, supra, for example. Fluorescence can be quantified using a fluorimeter.
(c) Various enzyme-substrate labels are available and U.S. Pat. No. 4,275,149 provides a review of some of these. The enzyme generally catalyzes a chemical alteration of the chromogenic substrate which can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (e.g., firefly luciferase and bacterial luciferase; U.S. Pat. No. 4,737,456), luciferin, 2,3-dihydrophthalazinediones, malate dehydrogenase, urease, peroxidase such as horseradish peroxidase (HRPO), alkaline phosphatase, beta.-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed J. Langone & H. Van Vunakis), Academic press, New York, 73:147-166 (1981).

The antibodies may also be used for in vivo diagnostic assays. In some embodiments, the antibody is labeled with a radionuclide so that the tumor can be localized using immunoscintiography. As a matter of convenience, the antibodies of the present invention can be provided in a kit, i. e., a packaged combination of reagents in predetermined amounts with instructions for performing the diagnostic assay. Where the antibody is labeled with an enzyme, the kit may include substrates and cofactors required by the enzyme (e.g., a substrate precursor which provides the detectable chromophore or fluorophore). In addition, other additives may be included such as stabilizers, buffers (e.g., a block buffer or lysis buffer) and the like. The relative amounts of the various reagents may be varied widely to provide for concentrations in solution of the reagents which substantially optimize the sensitivity of the assay. Particularly, the reagents may be provided as dry powders, usually lyophilized, including excipients which on dissolution will provide a reagent solution having the appropriate concentration.

In some embodiments, antibodies are conjugated to one or more maytansine molecules (e.g. about 1 to about 10 maytansine molecules per antibody molecule). Maytansine may, for example, be converted to May-SS-Me which may be reduced to May-SH3 and reacted with modified antibody (Chari et al. Cancer Research 52: 127-131 (1992)) to generate a maytansinoid-antibody immunoconjugate. In some embodiments, the conjugate may be the highly potent maytansine derivative DM1 (N2'-deacetyl-N2'-(3-mercapto-1-oxopropyl)-maytansine) (see for example WO02/098883 published Dec. 12, 2002) which has an IC50 of approximately 10-11 M (review, see Payne (2003) Cancer Cell 3:207- 212) or DM4 (N2'-deacetyl-N2'(4-methyl- -4-mercapto-1-oxopentyl)-maytansine) (see for example WO2004/103272 published Dec. 2, 2004).

In some embodiments the antibody conjugate comprises an anti-tumor cell antigen antibody conjugated to one or more calicheamicin molecules. The calicheamicin family of antibiotics is capable of producing double-stranded DNA breaks at sub-picomolar concentrations. Structural analogues of calicheamicin which may be used include, but are not limited to, gamma1I, alpha2I, alpha3I, N-acetyl-gamma1I, PSAG and thetaI1 (Hinman et al. Cancer Research 53: 3336-3342 (1993) and Lode et al. Cancer Research 58: 2925-2928 (1998)). See, also, U.S. Pat. Nos. 5,714,586; 5,712,374; 5,264,586; and 5,773,001, each of which is expressly incorporated herein by reference.

In some embodiments the antibody is conjugated to a prodrug capable of being release in its active form by enzymes overproduced in many cancers. For example, antibody conjugates can be made with a prodrug form of doxorubicin wherein the active component is released from the conjugate by plasmin. Plasmin is known to be over produced in many cancerous tissues (see Decy et al, (2004) FASEB Journal 18(3): 565-567).

In some embodiments the antibodies are conjugated to enzymatically active toxins and fragments thereof. In some embodiments the toxins include, without limitation, diphtheria A chain, nonbinding active fragments of diphtheria toxin, exotoxin A chain (from Pseudomonas aeruginosa),Pseudomonas endotoxin, ricin A chain, abrin A chain, modeccin A chain, alpha-sarcin, Aleurites fordii proteins, dianthin proteins, Phytolaca americana proteins (PAPI, PAPII, and PAP-S), Ribonuclease (Rnase), Deoxyribonuclease (Dnase), pokeweed antiviral protein, momordica charantia inhibitor, curcin, crotin, sapaonaria officinalis inhibitor, gelonin, mitogellin, restrictocin, phenomycin, neomycin and the tricothecenes. See, for example, WO 93/21232 published Oct. 28, 1993. In some embodiments the toxins have low intrinsic immunogenicity and a mechanism of action (e.g. a cytotoxic mechanism versus a cytostatic mechanism) that reduces the opportunity for the cancerous cells to become resistant to the toxin.

In some embodiments conjugates are made between the antibodies of the invention and immunomodulators. For example, in some embodiments immunostimulatory oligonucleotides can be used. These molecules are potent immunogens that can elicit antigen-specific antibody responses (see Datta et al, (2003) Ann N.Y. Acad. Sci 1002: 105-111). Additional immunomodulatory compounds can include stem cell growth factor such as "S1 factor", lymphotoxins such as tumor necrosis factor (TNF), hematopoietic factor such as an interleukin, colony stimulating factor (CSF) such as granulocyte-colony stimulating factor (G-CSF) or granulocyte macrophage-stimulating factor (GM-CSF), interferon (IFN) such as interferon alpha, beta or gamma, erythropoietin, and thrombopoietin.

In some embodiments radioconjugated antibodies are provided. In some embodiments such antibodies can be made using ³²P, ³³P, ⁴⁷Sc, ⁵⁹Fe, ⁶⁴Cu, ⁶⁷Cu, ⁷⁵Se, ⁷⁷As, ⁸⁹Sr, ⁹⁰Y, ⁹⁹Mo, ¹⁰⁵Rh, ¹⁰⁹Pd, ¹²⁵I, ¹³¹I, ¹⁴²Pr, ¹⁴³Pr, ¹⁴⁹Pm, ¹⁵³Sm, ¹⁶¹Th, ¹⁶⁶Ho, ¹⁶⁹Er, ¹⁷⁷Lu, ¹⁸⁶Re, ¹⁸⁸Re, ¹⁸⁹Re, ¹⁹⁴Ir, ¹⁹⁸Au, ¹⁹⁹Au, ²¹¹Pb, ²¹²Pb, ²¹³Bi, ⁵⁸Co, ⁶⁷Ga, ^{80m}Br, ^{99m}Tc, ^{103m}Rh, ¹⁰⁹Pt, ¹⁶¹Ho, ^{189m}Os, ¹⁹²Ir, ¹⁵²Dy, ²¹¹At, ²¹²Bi, ²²³Ra, ²¹⁹Rn, ²¹⁵Po, ²¹¹Bi, ²²⁵Ac, ²²¹Fr, ²¹⁷At, ²¹³Bi, ²⁵⁵Fm and combinations and subcombinations thereof. In some embodiments, boron, gadolinium or uranium atoms are conjugated to the antibodies. In some embodiments the boron atom is ¹⁰B, the gadolinium atom is ¹⁵⁷Gd and the uranium atom is ²³⁵U.

In some embodiments the radionuclide conjugate has a radionuclide with an energy between 20 and 10,000 keV. The radionuclide can be'an Auger emitter, with an energy of less than 1000 keV, a P emitter with an energy between 20 and 5000 keV, or an alpha or 'a' emitter with an energy between 2000 and 10,000 keV.

In some embodiments diagnostic radioconjugates are provided which comprise a radionuclide that is a gamma-, beta-, or positron-emitting isotope. In some embodiments the radionuclide has an energy between 20 and 10,000 keV. In some embodiments the radionuclide is selected from the group of ¹⁸F, ⁵¹Mn, ^{52m}Mn, ⁵²Fe, ⁵⁵Co, ⁶²Cu, ⁶⁴Cu, ⁶⁸Ga, ⁷²As, ⁷⁵Br, ⁷⁶Br, ^{82m}Rb, ⁸³Sr, ⁸⁹Zr, ^{94m}Tc, ⁵¹Cr, ⁵⁷Co, ⁵⁸Co, ⁵⁹Fe, ⁶⁷Ga, ⁷⁵Se, ⁹⁷Ru, ^{99m}Tc, ^{114m}In, ¹²³I, ¹²⁵I ¹³Li and ¹⁹⁷Hg_{.}

In some embodiments the antibodies of the invention are conjugated to diagnostic agents that are photoactive or contrast agents. Photoactive compounds can comprise compounds such as chromagens or dyes. Contrast agents may be, for example a paramagnetic ion, wherein the ion comprises a metal selected from the group of chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) and erbium (III). The contrast agent may also be a radio-opaque compound used in X-ray techniques or computed tomography, such as an iodine, iridium, barium, gallium and thallium compound. Radio-opaque compounds may be selected from the group of barium, diatrizoate, ethiodized oil, gallium citrate, iocarmic acid, iocetamic acid, iodamide, iodipamide, iodoxamic acid, iogulamide, iohexol, iopamidol, iopanoic acid, ioprocemic acid, iosefamic acid, ioseric acids, iosulamide meglumine, iosemetic acid, iotasul, iotetric acid, iothalamic acid, iotroxic acid, ioxaglic acid, ioxotrizoic acid, ipodate, meglumine, metrizamide, metrizoate, propyliodone, and thallous chloride. In some embodiments, the diagnostic immunoconjugates may contain ultrasound-enhancing agents such as a gas filled liposome that is conjugated to an antibody of the invention. Diagnostic immunoconjugates may be used for a variety of procedures including, but not limited to, intraoperative, endoscopic or intravascular methods of tumor or cancer diagnosis and detection.

In some embodiments antibody conjugates are made using a variety of bifunctional protein coupling agents such as N-succinimidyl-3-(2-pyridyldithiol) propionate (SPDP), succininiidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al. Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, dimethyl linker or disulfide-containing linker (Chari et al. Cancer Research 52: 127-131 (1992)) may be used. Agents may additionally be linked to the antibodies of the invention through a carbohydrate moiety.

In some embodiments fusion proteins comprising the antibodies of the invention and cytotoxic agents may be made, e.g. by recombinant techniques or peptide synthesis. In some embodiments such immunoconjugates comprising the anti-tumor antigen antibody conjugated with a cytotoxic agent are administered to the patient. In some embodiments the immunoconjugate and/or tumor cell antigen protein to which it is bound is/are internalized by the cell, resulting in increased therapeutic efficacy of the immunoconjugate in killing the cancer cell to which it binds. In some embodiments, the cytotoxic agent targets or interferes with nucleic acid in the cancer cell. Examples of such cytotoxic agents include maytansinoids, calicheamicins, ribonucleases and DNA endonucleases.

In some embodiments the antibodies are conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (e.g. avidin) which is conjugated to a cytotoxic agent (e.g. a radionucleotide).

In some embodiments the antibodies are conjugated conjugated to a cytotoxic molecule which is released inside a target cell lysozome. For example, the drug monomethyl auristatin E (MMAE) can be conjugated via a valine-citrulline linkage which will be cleaved by the proteolytic lysozomal enzyme cathepsin B following internalization of the antibody conjugate (see for example WO03/026577 published April 3, 2003). In some embodiments, the MMAE can be attached to the antibody using an acid-labile linker containing a hydrazone functionality as the cleavable moiety (see for example WO02/088172 published Nov. 11, 2002).

**Antibody Dependent Enzyme Mediated Prodrug Therapy (ADEPT)**

In some embodiments the antibodies of the present invention may be used in ADEPT by conjugating the antibody to a prodrug-Activating enzyme which converts a prodrug (e.g. a peptidyl chemotherapeutic agent, see WO81/01145) to an active anti-cancer drug. See, for example, WO 88/07378 and U.S. Pat. No. 4,975,278.

In some embodiments the enzyme component of the immunoconjugate useful for ADEPT includes any enzyme capable of acting on a prodrug in such a way so as to covert it into its more active, cytotoxic form.

Enzymes that are useful in ADEPT include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; .beta.-lactamase useful for converting drugs derivatized with beta.-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. In some embodiments antibodies with enzymatic activity, also known in the art as "abzymes", can be used to convert the prodrugs of the invention into free active drugs (see, e.g., Massey, Nature 328: 457-458 (1987)). Antibody-abzyme conjugates can be prepared as described herein for delivery of the abzyme to a tumor cell population.

In some embodiments the ADEPT enzymes can be covalently bound to the antibodies by techniques well known in the art such as the use of the heterobifunctional crosslinking reagents discussed above. In some embodiments, fusion proteins comprising at least the antigen binding region of an antibody of the invention linked to at least a functionally active portion of an enzyme of the invention can be constructed using recombinant DNA techniques well known in the art (see, e.g., Neuberger et al., Nature, 312: 604-608 (1984).

In some embodiments identification of an antibody that acts in a cytostatic manner rather than a cytotoxic manner can be accomplished by measuring viability of a treated target cell culture in comparison with a non-treated control culture. Viability can be detected using methods known in the art such as the CellTiter-Blue® Cell Viability Assay or the CellTiter-Glo® Luminescent Cell Viability Assay (Promega, catalog numbers G8080 and G5750 respectively). In some embodiments an antibody is considered as potentially cytostatic if treatment causes a decrease in cell number in comparison to the control culture without any evidence of cell death as measured by the means described above.

In some embodiments an in vitro screening assay can be performed to identify an antibody that promotes ADCC using assays known in the art. One exemplary assay is the In Vitro ADCC Assay. To prepare chromium 51-labeled target cells, tumor cell lines are grown in tissue culture plates and harvested using sterile 10 mM EDTA in PBS. The detached cells are washed twice with cell culture medium. Cells (5×10⁶) are labeled with 200 µCi of chromium 51 (New England Nuclear/DuPont) at 37° C. for one hour with occasional mixing. Labeled cells were washed three times with cell culture medium, then are resuspended to a concentration of 1×10⁵ cells/mL. Cells are used either without opsonization, or are opsonized prior to the assay by incubation with test antibody at 100 ng/mL and 1.25 ng/mL in PBMC assay or 20 ng/mL and 1 ng/mL in NK assay. Peripheral blood mononuclear cells are prepared by collecting blood on heparin from normal healthy donors and diluted with an equal volume of phosphate buffered saline (PBS). The blood is then layered over LYMPHOCYTE SEPARATION MEDIUM® (LSM: Organon Teknika) and centrifuged according to the manufacturer's instructions. Mononuclear cells are collected from the LSM-plasma interface and are washed three times with PBS. Effector cells are suspended in cell culture medium to a final concentration of 1×10⁷ cells/mL. After purification through LSM, natural killer (NK) cells are isolated from PBMCs by negative selection using an NK cell isolation kit and a magnetic column (Miltenyi Biotech) according to the manufacturer's instructions. Isolated NK cells are collected, washed and resuspended in cell culture medium to a concentration of 2×10⁶ cells/mL. The identity of the NK cells is confirmed by flow cytometric analysis. Varying effector:target ratios are prepared by serially diluting the effector (either PBMC or NK) cells two-fold along the rows of a microtiter plate (100 µL final volume) in cell culture medium. The concentration of effector cells ranges from 1.0×10⁷/mL to 2.0×10⁴ /mL for PBMC and from 2.0×10⁶/mL to 3.9×10³/mL for NK. After titration of effector cells, 100 µL of chromium 51-labeled target cells (opsonized or nonoponsonized) at 1×10⁵ cells/mL are added to each well of the plate. This results in an initial effector:target ratio of 100:1 for PBMC and 20:1 for NK cells. All assays are run in duplicate, and each plate contains controls for both spontaneous lysis (no effector cells) and total lysis (target cells plus 100 µL 1% sodium dodecyl sulfate, 1 N sodium hydroxide). The plates are incubated at 37° C. for 18 hours, after which the cell culture supernatants are harvested using a supernatant collection system (Skatron Instrument, Inc.) and counted in a Minaxi auto-gamma 5000 series gamma counter (Packard) for one minute. Results are then expressed as percent cytotoxicity using the formula: % Cytotoxicity = (sample cpm-spontaneous lysis)/(total lysis-spontaneous lysis)×100.

To identify an antibody that promotes CDC, the skilled artisan may perform an assay known in the art. One exemplary assay is the In Vitro CDC assay. In vitro, CDC activity can be measured by incubating tumor cell antigen expressing cells with human (or alternate source) complement-containing serum in the absence or presence of different concentrations of test antibody. Cytotoxicity is then measured by quantifying live cells using ALAMAR BLUE® (Gazzano-Santoro et al., J. Immunol. Methods 202 163-171 (1997)). Control assays are performed without antibody, and with antibody, but using heat inactivated serum and/or using cells which do not express the tumor cell antigen in question. Alternatively, red blood cells can be coated with tumor antigen or peptides derived from tumor antigen, and then CDC may be assayed by observing red cell lysis (see for example Karjalainen and Mantyjarvi, Acta Pathol Microbiol Scand [C]. 1981 Oct; 89(5):315-9).

To select for antibodies that induce cell death, loss of membrane integrity as indicated by, e.g., PI, trypan blue or 7AAD uptake may be assessed relative to control. One exemplary assay is the PI uptake assay using tumor antigen expressing cells. According to this assay, tumor cell antigen expressing cells are cultured in Dulbecco's Modified Eagle Medium (D-MEM):Ham's F-12 (50:50) supplemented with 10% heat-inactivated FBS (Hyclone) and 2 mM L-glutamine. (Thus, the assay is performed in the absence of complement and immune effector cells). The tumor cells are seeded at a density of 3 x 10⁶ per dish in 100 x 20 mm dishes and allowed to attach overnight. The medium is then removed and replaced with fresh medium alone or medium containing 10 µg/mL of the appropriate monoclonal antibody. The cells are incubated for a 3 day time period. Following each treatment, monolayers are washed with PBS and detached by trypsinization. Cells are then centrifuged at 1200 rpm for 5 minutes at 4°C., the pellet resuspended in 3 mL ice cold Ca²⁺ binding buffer (10 mM Hepes, pH 7.4, 140 mM NaCl, 2.5 mM CaCl₂) and aliquoted into 35 mm strainer-capped 12 x 75 tubes (1 mL per tube, 3 tubes per treatment group) for removal of cell clumps. Tubes then receive PI (10 µg/mL). Samples may be analyzed using a FACSCAN™ flow cytometer and FACSCONVERT™. CellQuest software (Becton Dickinson). Those antibodies that induce statistically significant levels of cell death as determined by PI uptake may be selected as cell death-inducing antibodies.

Antibodies can also be screened in vivo for apoptotic activity using ¹⁸F-annexin as a PET imaging agent. In this procedure, Annexin V is radiolabeled with ¹⁸F and given to the test animal following dosage with the antibody under investigations One of the earliest events to occur in the apoptotic process in the eversion of phosphatidylserine from the inner side of the cell membrane to the outer cell surface, where it is accessible to annexin. The animals are then subjected to PET imaging (see Yagle et al, J Nucl Med. 2005 Apr;46(4):658-66). Animals can also be sacrificed and individual organs or tumors removed and analyzed for apoptotic markers following standard protocols.

While in some embodiments cancer may be characterized by overexpression of a gene expression product, the present application further provides methods for treating cancer which is not considered to be a tumor antigen-overexpressing cancer. To determine tumor antigen expression in the cancer, various diagnostic/prognostic assays are available. In some embodiments, gene expression product overexpression can be analyzed by IHC. Paraffin embedded tissue sections from a tumor biopsy may be subjected to the IHC assay and accorded a tumor antigen protein staining intensity criteria as follows:
Score 0: no staining is observed or membrane staining is observed in less than 10% of tumor cells.
Score 1+: a faint/barely perceptible membrane staining is detected in more than 10% of the tumor cells. The cells are only stained in part of their membrane.
Score 2+: a weak to moderate complete membrane staining is observed in more than 10% of the tumor cells.
Score 3+: a moderate to strong complete membrane staining is observed in more than 10% of the tumor cells.

Those tumors with 0 or 1+ scores for tumor antigen overexpression assessment may be characterized as not overexpressing the tumor antigen, whereas those tumors with 2+ or 3+ scores may be characterized as overexpressing the tumor antigen.

Alternatively, or additionally, FISH assays such as the INFORM™ (sold by Ventana; Ariz.) or PATHVISION™ (Vysis, Ill.) may be carried out on formalin-fixed,
paraffin-embedded tumor tissue to determine the extent (if any) of tumor antigen overexpression in the tumor.

Additionally, antibodies can be chemically modified by covalent conjugation to a polymer to increase their circulating half-life, for example. Each antibody molecule may be attached to one or more (i.e. 1, 2, 3, 4, 5 or more) polymer molecules. Polymer molecules are preferably attached to antibodies by linker molecules. The polymer may, in general, be a synthetic or naturally occurring polymer, for example an optionally substituted straight or branched chain polyalkene, polyalkenylene or polyoxyalkylene polymer or a branched or unbranched polysaccharide, e.g. homo- or hetero-polysaccharide. In some embodiments the polymers are polyoxyethylene polyols and polyethylene glycol (PEG). PEG is soluble in water at room temperature and has the general formula: R(O--CH₂--CH₂)ₙ O--R where R can be hydrogen, or a protective group such as an alkyl or alkanol group. In some embodiments, the protective group has between 1 and 8 carbons. In some embodiments the protective groupis methyl. The symbol n is a positive integer, between 1 and 1,000, or 2 and 500. In some embodiments the PEG has an average molecular weight between 1000 and 40,000, between 2000 and 20,000, or between 3,000 and 12, 000. In some embodiments, PEG has at least one hydroxy group. In some embodiments the hydroxy is a terminal hydroxy group. In some embodiments it is this hydroxy group which is activated to react with a free amino group on the inhibitor. However, it will be understood that the type and amount of the reactive groups may be varied to achieve a covalently conjugated PEG/antibody of the present invention. Polymers, and methods to attach them to peptides, are shown in U. S. Patent Nnumbers 4,766,106; 4,179,337; 4,495,285; and 4,609,546 each of which is hereby incorporated by reference in its entirety.

**Oligonucleotides**

In some embodiments, the LIV-1 modulator is an oligonucleotide. In some embodiments, the LIV-1 modulator is an oligonucleotide comprising a sequence selected from the group consisting of SEQ ID NO:367-382.

In some embodiments the oligonucleotide is an antisense or RNAi oligonucleotide including siRNAs and shRNAs. In some embodiments the oligonucleotide is complementary to a region, domain, portion, or segment of the LIV-1 gene or gene product: In some embodiments, the oligonucleotide comprises from about 5 to about 100 nucleotides, from about 10 to about 50 nucleotides, from about 12 to about 35, and from about 18 to about 25 nucleotides. In some embodiments, the oligonucleotide is at least 50%, at least 60%, at least 70%, at least 80%, at least 90%, at least 95%, at least 96%, at least 97%, at least 98%, at least 99%, or 100% homologous to a region, portion, domain, or segment of the LIV-1 gene or gene product. In some embodiments there is substantial sequence homology over at least 15, 20, 25, 30, 35, 40, 50, or 100 consecutive nucleotides of the LIV-1 gene or gene product. In some embodiments there is substantial sequence homology over the entire length of the LIV-1 gene or gene product. In some embodiments, the oligonucleotide binds under moderate or stringent hybridization conditions to a nucleic acid molecule having a nucleotide sequence of SEQ ID NO: 1.

In some embodiments, the LIV-1 modulator is a double stranded RNA (dsRNA) molecule and works via RNAi (RNA interference). In some embodiments, one strand of the dsRNA is at least 50%, at least 60%, at least 70%, at least 80%, at least 90%, at least 95%, at least 96%, at least 97%, at least 98%, at least 99%, or 100% homologous to a region, portion, domain, or segment of the LIV-1 gene. In some embodiments there is substantial sequence homology over at least 15, 20, 25, 30, 35, 40, 50, 100, 200, 300, 400, 500, or 1000 consecutive nucleotides of the LIV-1 gene. In some embodiments there is substantial sequence homology over the entire length of the LIV-1 gene.

In some embodiments oligonucleotides of the invention are used in a polymerase chain reaction (PCR). This sequence may be based on (or designed from) a genomic sequence or cDNA sequence and is used to amplify, confirm, or detect the presence of an identical, similar, or complementary DNA or RNA in a particular cell or tissue.

**Small molecules**

In some embodiments, the LIV-1 modulator is a small molecule. As used herein, the term "small molecule" refers to an organic or inorganic non-polymer compound that has a molecular weight that is less than about 10 kilodaltons. Examples of small molecules include peptides, oligonucleotides, organic compounds, inorganic compounds, and the like. In some embodiments, the small molecule has a molecular weight that is less than about 9, about 8, about 7, about 6, about 5, about 4, about 3, about 2, or about 1 kilodalton.

**Mimetics**

In some embodiments, the LIV-1 modulator is a mimetic. As used herein, the term "mimetic" is used to refer to compounds which mimic the activity of a peptide. Mimetics are non-peptides but may comprise amino acids linked by non-peptide bonds. U.S. Patent No. 5,637,677, issued on June 10, 1997, and parent applications thereof, all of which are incorporated herein by reference, contain detailed guidance on the production of mimetics. Briefly, the three-dimensional structure of the peptide which specifically interacts with the three dimensional structure of the LIV-1 is duplicated by a molecule that is not a peptide. In some embodiments the LIV-1 mimetic is a mimetic of LIV-1 or mimetic of a ligand-of LIV-1.

**Decoys**

In some embodiments, the LIV-1 modulator is a decoy comprising at least a portion of a LIV-1 polypeptide. In some embodiments the decoy competes with natural LIV-1 polypeptides for zinc or zinc carrier-zinc complexes. In some embodiments, the decoy is labeled to facilitate quantification, qualification, and/or visualization. In other embodiments, the decoy further comprises a moiety to facilitate isolation and/or separation of the decoy or the decoy-zinc or decoy-zinc carrier complex. In some embodiments, the decoy functions by capturing zinc and/or a zinc carrier (complexed or uncomplexed with zinc) and preventing it from interacting with the signaling LIV-1 polypeptide. In some embodiments the decoy comprises at least a portion of a LIV-1 polypeptide fused to an antibody or antibody fragment.

**Methods of treating/Preventing Cancer**

The present invention provides methods for treating and/or preventing cancer or symptoms of cancer in a subject comprising administering to the subject a therapeutically effective amount of one or more LIV-1 modulators of the present invention. In some embodiments the cancer is a cancer associated with overexpression of LIV-1. In some embodiments, the cancer is breast cancer, skin cancer, esophageal cancer, liver cancer, pancreatic cancer, prostatic cancer, uterine cancer, cervical cancer, lung cancer, bladder cancer, ovarian cancer, multiple myeloma or melanoma. In some embodiments, the cancer is in a non-hormonally regulated tissue. In some embodiments the breast cancer is an ER-positive breast cancer, an ER-negative breast cancer, or a metastatic breast cancer. In some embodiments the breast cancer is ductal adenocarcinoma, lobular adenocarcinoma, or metastatic adenocarcinoma. In some embodiments the subject has been diagnosed as having a cancer or as being predisposed to cancer.

Symptoms of cancer are well-known to those of skill in the art and include, without limitation, breast lumps, nipple changes, breast cysts, breast pain, death, weight loss, weakness, excessive fatigue, difficulty eating, loss of appetite, chronic cough, worsening breathlessness, coughing up blood, blood in the urine, blood in stool, nausea, vomiting, liver metastases, lung metastases, bone metastases, abdominal fullness, bloating, fluid in peritoneal cavity, vaginal bleeding, constipation, abdominal distension, perforation of colon, acute peritonitis (infection, fever, pain), pain, vomiting blood, heavy sweating, fever, high blood pressure; anemia, diarrhea, jaundice, dizziness, chills, muscle spasms, colon metastases, lung metastases, bladder metastases, liver metastases, bone metastases, kidney metastases, and pancreas metastases, difficulty swallowing, and the like.

A therapeutically effective amount of the modulating compound can be determined empirically, according to procedures well known to medicinal chemists, and will depend, inter alia, on the age of the patient, severity of the condition, and on the ultimate pharmaceutical formulation desired. Administration of the modulators of the present invention can be carried out, for example, by inhalation or suppository or to mucosal tissue such as by lavage to vaginal, rectal, urethral, buccal and sublingual tissue, orally, topically, intranasally, intraperitoneally, parenterally, intravenously, intralymphatically, intratumorly, intramuscularly, interstitially, intra-arterially, subcutaneously, intraoccularly, intrasynovial, transepithelial, and transdermally. In some embodiments, the inhibitors are administered by lavage, orally or inter-arterially. Other suitable methods of introduction can also include rechargeable or biodegradable devices and slow or sustained release polymeric devices. As discussed above, the therapeutic compositions of this invention can also be administered as part of a combinatorial therapy with other known anti-cancer agents or other known anti-bone disease treatment regimen.

The present invention further provides methods of modulating a LIV-1-related biological activity in a patient. The methods comprise administering to the patient an amount of a LIV-1 modulator effective to modulate one or more LIV-1 biological activities. Suitable assays for measuring LIV-1 biological activities are set forth *supra* and *infra*.

The present invention also provides methods of inhibiting cancer cell growth in a patient in need thereof comprising administering a therapeutically effective amount of one or more LIV-1 modulators to the patient. Suitable assays for measuring LIV-1-related cell growth are known to those skilled in the art and are set forth *supra* and *infra.*

The present invention further provides methods of inhibiting cancer in a patient in need thereof. The methods comprise determining if the patient is a candidate for LIV-1 therapy as described herein and administering a therapeutically effective amount of one or more LIV-1 modulators to the patient if the patient is a candidate for LIV-1 therapy. If the patient is not a candidate for LIV-1 therapy, the patient is treated with conventional cancer treatment.

The present invention further provides methods of inhibiting cancer in a patient diagnosed or suspected of having a cancer. The methods comprise administering a therapeutically effective amount of one or more LIV-1 modulators to the patient.

The present invention also provides methods for inhibiting the interaction of two or more cells in a patient comprising administering a therapeutically effective amount of a LIV-1 modulator to said patient. Suitable assays for measuring LIV-1-related cell interaction are known to those skilled in the art and are set forth *supra* and *infra.*

The present invention also provides methods of modulating one or more symptoms of cancer in a patient comprising administering to said patient a therapeutically effective amount of the LIV-1 compositions described herein.

The present invention further provides methods for inhibiting cell growth in a patient in need thereof comprising administering to the patient a therapeutically effective amount of a LIV-1 modulator. Suitable assays for measuring LIV-1-related anchorage-independent cell growth are set forth *supra* and *infra.*

The present invention also provides methods for inhibiting migration of cancer cells in a patient in need thereof comprising administering to the patient a therapeutically effective amount of a LIV-1 modulator. Suitable assays for measuring LIV-1-related cell migration are known to those skilled in the art.

The present invention further provides methods for inhibiting adhesion of cancer cells in a patient in need thereof comprising administering to the patient a therapeutically effective amount of a LIV-1 modulator. Suitable assays for measuring LIV-1-related cell adhesion are known to those skilled in the art.

The present invention also provides methods for prophylactically treating a patient who is predisposed to develop cancer, a cancer metastasis or who has had a metastasis and is therefore susceptible to a relapse or recurrence. The methods are particularly useful in high-risk individuals who, for example, have a family history of cancer or of metastasizing tumors, or show a genetic predisposition for a cancer metastasis. In some embodiments the tumors are LIV-1-related tumors. Additionally, the methods are useful to prevent patients from having recurrences of LIV-1-related tumors who have had LIV-1-related tumors removed by surgical resection or treated with a conventional cancer treatment.

The present invention also provides methods of inhibiting cancer progression and/or causing cancer regression comprising administering to the patient a therapeutically effective amount of a LIV-1 modulator.

In some embodiments, the patient in need of anti-cancer treatment is treated with the LIV-1 modulators of the present invention in conjunction with chemotherapy and/or radiation therapy. For example, following administration of the LIV-1 modulators, the patient may also be treated with a therapeutically effective amount of anti-cancer radiation. In some embodiments chemotherapeutic treatment is provided in combination with LIV-1 modulators. In some embodiments LIV-1 modulators are administered in combination with chemotherapy and radiation therapy.

Methods of treatment comprise administering single or multiple doses of one or more LIV-1 modulators to the patient. In some embodiments the LIV-1 modulators are administered as injectable pharmaceutical compositions that are sterile, pyrogen free and comprise the LIV-1 modulators in combination with a pharmaceutically acceptable carrier or diluent.

In some embodiments, the therapeutic regimens of the present invention are used with conventional treatment regimens for cancer including, without limitation, surgery, radiation therapy, hormone ablation and/or chemotherapy. Administration of the LIV-1 modulators of the present invention may take place prior to, simultaneously with, or after conventional cancer treatment. In some embodiments, two or more different LIV-1 modulators are administered to the patient.

In some embodiments the amount of LIV-1 modulator administered to the patient is effective to inhibit one or more of cancer cell growth, tumor formation, cancer cell proliferation, cancer cell metastasis, cell migration, angiogenesis, LIV-1 signaling, inhibit LIV-1-mediated cell-cell adhesion, LIV-1-mediated cell-cell membrane interaction, LIV-1-mediated cell-extracellular matrix interaction, integrin mediated activities, LIV-1-mediated cell-extracellular matrix degradation, and LIV-1 expression. In some embodiments the amount of LIV-1 modulator administered to the patient is effective to increase cancer cell death through apoptosis.

**Combination Therapy**

In some embodiments the invention provides compositions comprising two or more LIV-1 modulators to provide still improved efficacy against cancer. In some embodiments the LIV-1 modulators are monoclonal antibodies. Compositions comprising two or more LIV-1 antibodies may be administered to persons or mammals suffering from, or predisposed to suffer from, cancer. One or more antibodies may also be administered with another therapeutic agent, such as a cytotoxic agent, or cancer chemotherapeutic. Concurrent administration of two or more therapeutic agents does not require that the agents be administered at the same time or by the same route, as long as there is an overlap in the time period during which the agents are exerting their therapeutic effect. Simultaneous or sequential administration is contemplated, as is administration on different days or weeks.

In some embodiments the methods provide of the invention contemplate the Administration of combinations, or "cocktails", of different antibodies. Such antibody cocktails may have certain advantages inasmuch as they contain antibodies which exploit different effector mechanisms or combine directly cytotoxic antibodies with antibodies that rely on immune effector functionality. Such antibodies in combination may exhibit synergistic therapeutic effects.

A cytotoxic agent refers to a substance that inhibits or prevents the function of cells and/or causes destruction of cells. The term is intended to include radioactive isotopes (e.g., ¹³¹I, ¹²⁵I, ⁹⁰Y and ¹⁸⁶Re), chemotherapeutic agents, and toxins such as enzymatically active toxins of bacterial, fungal, plant or animal origin or synthetic toxins, or fragments thereof. A non-cytotoxic agent refers to a substance that does not inhibit or prevent the function of cells and/or does not cause destruction of cells. A non-cytotoxic agent may include an agent that can be activated to be cytotoxic. A non-cytotoxic agent may include a bead, liposome, matrix or particle (see, e.g., U.S. Patent Publications 2003/0028071 and 2003/0032995 which are incorporated by reference herein). Such agents may be conjugated, coupled, linked or associated with an antibody according to the invention.

In some embodiments, conventional cancer medicaments are administered with the compositions of the present invention. Conventional cancer medicaments include:
a) cancer chemotherapeutic agents;
b) additional agents;
c) prodrugs.

Cancer chemotherapeutic agents include, without limitation, alkylating agents, such as carboplatin and cisplatin; nitrogen mustard alkylating agents; nitrosourea alkylating agents, such as carmustine (BCNU); antimetabolites, such as methotrexate; folinic acid; purine analog antimetabolites, mercaptopurine; pyrimidine analog antimetabolites, such as fluorouracil (5-FU) and gemcitabine (Gemzar®); hormonal antineoplastics, such as goserelin, leuprolide, and tamoxifen; natural antineoplastics, such as aldesleukin, interleukin-2, docetaxel, etoposide (VP-16), interferon alfa, paclitaxel (Taxol®), and tretinoin (ATRA); antibiotic natural antineoplastics, such as bleomycin, dactinomycin, daunorubicin, doxorubicin, daunomycin and mitomycins including mitomycin C; and vinca alkaloid natural antineoplastics, such as vinblastine, vincristine, vindesine; hydroxyurea; aceglatone, adriamycin, ifosfamide, enocitabine, epitiostanol, aclarubicin, ancitabine, nimustine, procarbazine hydrochloride, carboquone, carboplatin, carmofur, chromomycin A3, antitumor polysaccharides, antitumor platelet factors, cyclophosphamide (Cytoxin®), Schizophyllan, cytarabine (cytosine arabinoside), dacarbazine, thioinosine, thiotepa, tegafur, dolastatins, dolastatin analogs such as auristatin, CPT-11 (irinotecan), mitozantrone, vinorelbine, teniposide, aminopterin, carminomycin, esperamicins (See, e.g., U.S. Patent No. 4,675,187), neocarzinostatin, OK-432, bleomycin, furtulon, broxuridine, busulfan, honvan, peplomycin, bestatin (Ubenimex®), interferon-β, mepitiostane, mitobronitol, melphalan, laminin peptides, lentinan, Coriolus versicolor extract, tegafur/uracil, estramustine (estrogen/mechlorethamine).

Additonal agents which may be used as therapy for cancer patients include EPO, G-CSF, ganciclovir; antibiotics, leuprolide; meperidine; zidovudine (AZT); interleukins 1 through 18, including mutants and analogues; interferons or cytokines, such as interferons α, β, and γ hormones, such as luteinizing hormone releasing hormone (LHRH) and analogues and, gonadotropin releasing hormone (GnRH); growth factors, (such as transforming growth factor-β (TGF-β), fibroblast growth factor (FGF), nerve growth factor (NGF), growth hormone releasing factor (GHRF), epidermal growth factor (EGF), fibroblast growth factor homologous factor (FGFHF), hepatocyte growth factor (HGF), and insulin growth factor (IGF); tumor necrosis factor-α & β (TNF-α & β); invasion inhibiting factor-2 (IIF-2); bone morphogenetic proteins 1-7 (BMP 1-7); somatostatin; thymosin-α-1; γ-globulin; superoxide dismutase (SOD); complement factors; anti-angiogenesis factors; antigenic materials; and prodrugs.

Prodrug refers to a precursor or derivative form of a pharmaceutically active substance that is less cytotoxic or non-cytotoxic to tumor cells compared to the parent drug and is capable of being enzymatically activated or converted into an active or the more active parent form. See, e.g., Wilman, "Prodrugs in Cancer Chemotherapy." Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). Prodrugs include, but are not limited to, phosphate-containing prodrugs, thiophosphate-containing prodrugs, sulfate-containing prodrugs, peptide-containing prodrugs, D-amino acid-modified prodrugs, glycosylated prodrugs, b-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use herein include, but are not limited to, those chemotherapeutic agents described above.

**Clinical Aspects**

In some embodiments, the methods and compositions of the present invention are particularly useful in breast cancer, skin cancer, esophageal cancer, liver cancer, pancreatic cancer, prostatic cancer, uterine cancer, cervical cancer, lung cancer, bladder cancer, ovarian cancer, multiple myeloma and melanoma. In some embodiments, the cancer is ductal adenocarcinoma, lobular adenocarcinoma, or metastatic adenocarcinoma.

**Pharmaceutical Compositions**

The present invention also provides pharmaceutical compositions comprising one or more of the LIV-1 modulators described herein and a pharmaceutically acceptable carrier. In some embodiments the pharmaceutical compositions are prepared as injectables, either as liquid solutions or suspensions; solid forms suitable for solution in, or suspension in, liquid vehicles prior to injection can also be prepared. Liposomes are included within the definition of a pharmaceutically acceptable carrier. Pharmaceutically acceptable salts can also be present in the pharmaceutical composition, e.g., mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington: The Science and Practice of Pharmacy (1995) Alfonso Gennaro, Lippincott, Williams, & Wilkins.

**Methods of Detecting LIV-1**

The present invention also provides methods for detecting LIV-1. In some embodiments the LIV-1 is present in a patient or in a patient sample. In some embodiments the method comprises administering a composition comprising one or more LIV-1 modulators to the patient and detecting the localization of the imaging agent in the patient. In some embodiments the patient sample comprises cancer cells. In some embodiments the LIV-1 modulator is linked to an imaging agent or is detectably labeled. In some embodiments, the LIV-1 modulator is a LIV-1 antibody conjugated to an imaging agent and is administered to a patient to detect one or more tumors or to determine susceptibility of the patient to LIV-1 therapy. The labeled antibodies will bind to the high density of receptors on cells and thereby accumulate on the tumor cells. Using standard imaging techniques, the site of the tumors can be detected.

The present invention also provides methods of imaging/detecting cells or tumors expressing or overexpressing LIV-1 comprising contacting a composition comprising an LIV-1 modulator to a sample and detecting the presence of the LIV-1 modulator in the sample. In some embodiments the sample is a patient sample. In some embodiments the patient sample comprises cancer cells. In some embodiments the LIV-1 modulator is linked to an imaging agent or is detectably labeled.

The present invention also provides methods for quantifying the amount of LIV-1 present in a patient, cell or sample. The methods comprise administering one or more of antibodies, probes, or small molecules to a patient or sample and detecting the amount of LIV-1 present in the sample. In some embodiments the antibodies, probes, or small molecules are linked to an imaging agent or are detectably labeled. Such information indicates, for example, whether or not a tumor is related to LIV-1, and, therefore, whether specific treatments should be used or avoided. In some embodiments, using standard techniques well known to the art-skilled, samples believed to include tumor cells are obtained and contacted with labeled antibodies, probes, oligonucleotides, and small molecules. After removing any unbound, labeled antibodies, probes, oligonucleotides or small molecules, the quantity of labeled antibodies, peptides, oligonucleotides or mimetics bound to the cell, or the quantity of antibodies, peptides, oligonucleotides or mimetics removed as unbound is determined. The information directly relates to the amount of LIV-1 present.

Imaging can be performed using procedures well known to those of ordinary skill in the art. Imaging can be performed, for example, by radioscintigraphy, nuclear magnetic resonance imaging (MRI) or computed tomography (CT scan). The most commonly employed radiolabels for imaging agents include radioactive iodine and indium. Imaging by CT scan may employ a heavy metal such as an iron chelate. MRI scanning may employ chelates of gadolinium or manganese. Additionally, positron emission tomography (PET) may be possible using positron emitters of oxygen, nitrogen, iron, carbon, or gallium. Imaging may also be performed using zinc-sensitive dyes to monitor Liv-1 activity in vivo. Such methods are known to those skilled in the art. Examples of such methods are discussed by A. Takeda et al, Cancer Research 61, 5065-5069, July 1, 2001; and C. Frederickson, Sci STKE. 2003 May 13;2003(182); each of which is incorporated by reference in its entirety.

In some embodiments the LIV-1 modulator is a LIV-1 antibody. In some embodiments the modulator is linked to an imaging agent or is detectably labeled. In some embodiments the imaging agent is ¹⁸F, ⁴³K, ⁵²Fe, ⁵⁷Co, ⁶⁷Cu, ⁶⁷Ga, ⁷⁷Br, ⁸⁷MSr, ⁸⁶Y, ⁹⁰Y, ⁹⁹MTc, ¹¹¹In, ¹²³I, ¹²⁵I, ¹²⁷Cs, ¹²⁹Cs, ¹³¹I, ¹³²I, ¹⁹⁷Hg, ²⁰³Pb, or ²⁰⁶Bi.

Methods of detection are well known to those of skill in the art. For example, methods of detecting polynucleotides include, but are not limited to PCR, Northern blotting, Southern blotting, RNA protection, and DNA hybridization (including in situ hybridization). Methods of detecting polypeptides include, but are not limited to, Western blotting, ELISA, enzyme activity assays, slot blotting, peptide mass fingerprinting, electrophoresis, immunochemistry and immunohistochemistry. Other examples, of detection methods include, but are not limited to, radioimmunoassay (RIA), chemiluminescence immunoassay, fluoroimmunoassay, time-resolved fluoroimmunoassay (TR-FIA), two color fluorescent microscopy, or immunochromatographic assay (ICA), all well known by those of skill in the art. In some preferred embodiments of the present invention, polynucleotide expression is detected using PCR methodologies and polypeptide production is detected using ELISA technology.

**Methods for delivering a cytotoxic agent or a diagnostic agent to a cell**

The present invention also provides methods for delivering a cytotoxic agent or a diagnostic agent to one or more cells that express LIV-1. In some embodiments the methods comprise contacting a LIV-1 modulator of the present invention conjugated to a cytotoxic agent or diagnostic agent with the cell.

**Methods for determining the prognosis of a cancer patient**

The present invention also provides methods for determining the prognosis of a patient with a LIV-1-associated cancer. The methods comprise determining the ratio of LIV-1-delta to LIV-1 in a patient sample. Although not wishing to be bound by theory, the present inventors have discovered that higher levels of LIV-1-delta relative to LIV-1 correlates with less aggressive cancer and/or, a cancer more amenable to treatment. Accordingly, in some embodiments, high levels of LIV-1-delta relative to LIV-1 are indicative of a patient with a good prognosis for extended survival and/or successful treatments with a LIV-1 modulator of the present invention and/or a conventional cancer medicament. In some embodiments, a good prognosis is indicated by a LIV-1-delta:LIV-1 ratio of at least 2:1, at least 3:1, at least 4:1 or at least 5:1. In some embodiments ratios of LIV-1-delta:LIV-1 are determined by measuring mRNA or protein levels.

In some embodiments, methods for determining the prognosis of a patient with a LIV-1 associated cancer comprise detecting LIV-1 bound to the plasma membrane of a cell in a patient sample. In some embodiments, detection of LIV-1 bound to the plasma membrane of a cell in a patient sample is not indicative of a good prognosis for extended survival and or successful treatment with a LIV-1 modulator of the present invention and/or a conventional cancer medicament.

In some embodiments LIV-1 is encoded for by a nucleic acid having a sequence of SEQ ID NO:1. In some embodiments LIV-1 has a sequence of SEQ ID NO:2. In some embodiments LIV-1-delta has a sequence of SEQ ID NO: 365.

**Methods for determining susceptibility to LIV-1 therapy**

The present invention also provides methods for determining the susceptibility of a patient to LIV-1 therapy. The methods comprise detecting the presence or absence of evidence of differential expression of LIV-1 in a patient or patient sample. The presence of evidence of differential expression of LIV-1 in the patient or sample is indicative of a patient who is susceptible to LIV-1 therapy. In some embodiments, the absence of evidence of differential expression of LIV-1 in the patient or patient sample is indicative of a patient who is not a candidate for LIV-1 therapy.

In some embodiments the therapeutic methods comprise first identifying patients susceptible to LIV-1 therapy comprising administering to the patient in need thereof a composition comprising a LIV-1 modulator linked to an imaging agent and detecting the presence or absence of evidence of the gene or gene product in the patient. In some embodiments, the therapeutic methods further comprise administering one or more LIV-1 modulators to the patient if the patient is a candidate for LIV-1 therapy and treating the patient with conventional cancer treatment if the patient is not a candidate LIV-1 therapy.

In some therapeutic methods, one or more LIV-1 modulators are administered to the patients alone or in combination with other anti-cancer medicaments when the patient is identified as having a cancer or being susceptible to a cancer.

**Methods for assessing the progression of cancer**

The invention also provides methods for assessing the progression of cancer in a patient comprising comparing the level of an expression product of LIV-1 in a biological sample at a first time point to a level of the same expression product at a second time point. A change in the level of the expression product at the second time point relative to the first time point is indicative of the progression of the cancer.

**Methods for Screening**

The present invention also provides methods of screening for anti-cancer agents. The methods comprise contacting a cell expressing LIV-1 with a candidate compound and determining whether an LIV-1-related biological activity is modulated. In some embodiments, inhibition of one or more of cancer cell growth, integrin mediated activities, tumor formation, cancer cell proliferation, cancer cell metastasis, cell migration, angiogenesis, LIV-1 signaling, LIV-1-mediated cell-cell adhesion, LIV-1-mediated cell-cell membrane interaction, LIV-1-mediated cell-extracellular matrix interaction, LIV-1-mediated cell-extracellular matrix degradation, and LIV-1 expression is indicative of an anti-cancer agent.

The present invention further provides methods of identifying a cancer inhibitor. The methods comprise contacting a cell expressing LIV-1 with a candidate compound and an LIV-1 ligand, and determining whether an LIV-1-related biological activity is modulated. In some embodiments, inhibition of one or more of cancer cell growth, integrin mediated activities, tumor formation, cancer cell proliferation, cancer cell metastasis, cell migration, angiogenesis, LIV-1 signaling, LIV-1-mediated cell-cell adhesion, LIV-1-mediated cell-cell membrane interaction, LIV-1-mediated cell-extracellular matrix interaction, LIV-1-mediated cell-extracellular matrix degradation, and LIV-1 expression is indicative of a cancer inhibitor. In some embodiments the amount of LIV-1 modulator administered to the patient is effective to increase cancer cell apoptosis.

In some embodiments, the invention provides methods of screening for anti-cancer agents, particularly anti-metastatic cancer agents, by, for example, screening putative modulators for an ability to modulate the activity or level of a downstream marker. In some embodiments candidate agents that decrease cyclin D1 levels, reduce MT1-MMP levels, or reduce cytoplasmic zinc levels are identified as anti-cancer agents.

**Methods for purifying LIV-1**

In some embodiments, the invention provides methods of purifying LIV-1 protein from a sample comprising LIV-1. The methods comprise providing an affinity matrix comprising a LIV-1 antibody of the present invention bound to a solid support, contacting the sample with the affinity matrix to form an affinity matrix-LIV-1 protein complex, separating the affinity matrix-LIV-1 protein complex from the remainder of the sample; and releasing LIV-1 protein from the affinity matrix.

**Kits**

In some embodiments, the present invention provides kits for imaging and/or detecting a gene or gene product correlated with LIV-1 overexpression. Kits of the invention comprise detectable antibodies, small molecules, oligonucleotides, decoys, mimetics or probes as well as instructions for performing the methods of the invention. Optionally, kits may also contain one or more of the following: controls (positive and/or negative), containers for controls, photographs or depictions of representative examples of positive and/or negative results.

Each of the patents, patent applications, accession numbers and publications described herein is hereby incorporated by reference in its entirety.

Various modifications of the invention, in addition to those described herein, will be apparent to those of skill in the art in view of the foregoing description. Such
modifications are also intended to fall within the scope of the appended embodiments. The present invention is further demonstrated in the following examples that are for purposes of illustration and are not intended to limit the scope of the present invention.

### EXAMPLES

**Example 1: Immunoprecipitation**

Immunoprecipitation (IP) buffer was prepared containing 50 mM Tris-HCl pH 7.5, 150 mM NaCl, 1% TritonX-100 and 1 protease inhibitor tablet (Roche Diagnostic Corp., Indianapolis, IN) per 10 mL total volume. A rabbit polyclonal antibody (Ab) generated in-house, anti-LIV-1, was combined at 1:100 dilution with IP buffer and added to cell lysate in a screw-top tube, which was allowed to mix on a rocker platform for 1-2 hours at 4°C. For immunoprecipitation, either 40 µl of anti-rabbit IgG-conjugated beads or 120 µl of streptavidin beads were added to each tube and incubation was continued overnight at 4°C on the rocking platform. Subsequently, the tubes were centrifuged at 7000 x g for 2 minutes at 4°C, and the supernatant removed. Bead pellets were washed 4 times with cold wash buffer, and then 30 µl of 2X SDS Tris/Glycine sample buffer containing reducing agent was added to each tube. The beads in sample buffer were then twice boiled at 95°C for 5 minutes each time to release the immunoprecipitate from the beads. The boiled bead solution was then centrifuged at 14,000 x g for 5 minutes at room temperature, and the supernatant then removed and transferred to a new tube. Immunoprecipitates were immediately analyzed by electrophoresis on an SDS-PAGE gel or stored at -20°C. Western blot analysis was performed using standard methods. Electrophoresed immunoprecipitates were transferred from the polyacrylamide gel to membrane and the membrane was then probed for 1 hour at room temperature with gentle rocking using a second LIV-1 antibody (at 1:1000). After several washes with PBS containing 0.05% Tween20 (PBST), the appropriate species-specific secondary antibody conjugated to horseradish peroxidase (HRP) was added and incubated on a rocker platform for 30 minutes at room temperature. After several washes, reactive bands on the membrane were then visualized using the ECL detection system (Amersham Biosciences UK).

**Example 2: FACS analysis**

Non-permeabilized cells were used for the analysis. FACS buffer was prepared containing (cold) PBS, 1% bovine serum albumin (BSA), 2% fetal bovine serum (FBS) and 0.1% sodium azide. Cells were harvested by detaching adherent cells using dissociation buffer (Invitrogen Corp., Carlsbad, CA). To neutralize the dissociation buffer, an equal volume of growth media was added. Cells were then aliquotted into a 5 mL polystyrene round-bottom tube. For each staining, one million cells were centrifuged at 1000 rpm for 5 minutes at 4°C, and then primary antibody (up to 6 µg in 100 µL FACS buffer) was added to the cell pellet, mixed by vortexing and incubated on ice for 30 minutes to one hour. Cells were then washed twice with 3 mL FACS buffer after pelleting by centrifugation at 1000 rpm for 5 minutes at 4°C. After the second wash and centrifugation, secondary antibody (1 µg in 50 µL FACS buffer) was then added to cell pellet, mixed by vortexing and incubated on ice for 30 minutes in the dark. Cells were then washed twice with 3 mL FACS buffer after pelleting by centrifugation at 1000 rpm for 5 minutes at 4°C. After the second wash and centrifugation, cells were resuspended in 500 µg in 50 µL FACS buffer containing propidium iodide (PI). (PI was prepared as a 1 µg/µL stock and used at 1:100). FACS / flow cytometry analysis was performed within an hour.

**Example 3: LIV-1 Oligonucleotides Inhibit Soft Agar Growth**

MDA231, MCF-7, ZR75-1 and T47D1 cells were treated with oligonucleotides to LIV-1 (SEQ ID NOS: 369 and 370). The cells were plated in 0.35% soft agar and growth quantitated using Alamar Blue after 7 days in culture.

The effect of LIV-1 gene expression upon anchorage-independent cell growth of T47D 1 cells was measured by colony formation in soft agar. Soft agar assays were performed by first coating a non-tissue culture treated plate with Poly-HEMA to prevent cells from attaching to the plate. Non-transfected cells were harvested using trypsin and washing twice in media. The cells were counted using a hemacytometer and resuspended to 10⁴ cells per ml in media. Fifty µl aliquots were placed in polyHEMA coated 96-well plates and transfected. For each transfection mixture, a carrier molecule, preferably a lipitoid or cholesteroid, was prepared to a working concentration of 0.5 nM in water, sonicated to yield a uniform solution, and filtered through a 0.45 µm PVDF membrane. The antisense or control oligonucleotide was then prepared to a working concentration of 100 µM in sterile Millipore water. The oligonucleotides were further diluted in OptiMEM™ (Gibco/BRL) in a microfuge tube to 2 µM, or approximately 20 µg oligo/ml of OptiMEM™. In a separate microfuge tube, lipitoid or cholesteroid, typically in the amount of about 1.5-2 nmol lipitoid/µg antisense oligonucleotide, was diluted in the same volume of OptiMEM™ used to dilute the oligonucleotide. The diluted antisense oligonucleotide was immediately added to the diluted lipitoid and mixed by pipetting up and down. Oligonucleotide was added to the cells to a final concentration of about 300 nM. Following transfection at 37° C for about 30 minutes, 3% GTG agarose was added to the cells for a final concentration of 0.35% agarose by pipetting up and down. After the cell layer agarose solidified, 100 µl of media was dribbled on top of each well. Colonies formed in about 7 days. For a read-out of growth, 20 µl of Alamar Blue was added to each well and the plate was shaken for about 15 minutes. Fluorescence readings (530 nm excitation/590 nm emission) were taken after incubation for 6-24 hours.

Inhibition of colony formation in cancer cell lines using LIV-1 modulators indicates that LIV-1 is important in production and/or maintenance of the metastatic phenotype.

**Example 4: Regulation of Gene Expression**

The expression of the differentially expressed genes represented by the polynucleotides in the cancerous cells was analyzed using oligonucleotides to confirm the role and function of LIV-1 in tumorigenesis, e.g., in promoting a metastatic phenotype.

LIV-1 oligonucleotides were generated and tested for their ability to suppress expression of LIV-1. Once synthesized and quantitated, the oligomers were screened for efficiency of a transcript knock-out in a panel of cell lines. The efficiency of the knock-out was determined by analyzing mRNA levels using GeneAmp quantification.

The ability of each oligonucleotide to inhibit gene expression was tested through transfection into T47D1, T47D-T1 MCF7, ZR-75-1, MDA231, 184B5, or HMEC cells.

For each transfection mixture, a carrier molecule (such as a lipid, lipid derivative, lipid-like molecule, cholesterol, cholesterol derivative, or cholesterol-like molecule) was prepared ,to a working concentration of 0.5 mM in water, sonicated to yield a uniform solution, and filtered through a 0.45 µm PVDF membrane. The antisense and siRNA oligonucleotides were then prepared to a working concentration of about 100 µM in sterile Millipore water. The oligonucleotides were further diluted in OptiMEM™ (Gibco/BRL), in a microfuge tube, to 2 µM, or approximately 20 µg oligo/ml of OptiMEM™. In a separate microfuge tube, the carrier molecule, typically in the amount of about 1.5-2 nmol carrier/µg antisense oligonucleotide was diluted into the same volume of OptiMEM™ used to dilute the oligonucleotide. The diluted antisense oligonucleotide is immediately added to the diluted carrier and mixed by pipetting up and down. SiRNAs were added to the cells to a final concentration of about 67nM.

The level of target mRNA that corresponds to a target gene of interest in the transfected cells was quantitated in the cell lines using the ABI GeneAmp 7000™ real-time PCR machine. Values for the target mRNA were normalized versus an internal control. For each 20 µl reaction, extracted RNA (generally 0.2-1 µg total) was placed into a sterile 0.5 or 1.5 ml microcentrifuge tube, and water added to a total volume of 12.5 µl. To each tube was added 7.5 µl of a buffer/enzyme mixture, prepared by mixing (in the order listed) 2.5 µl H₂O, 2.0 µl 10X reaction buffer, 10 µl oligo dT (20 pmol), 1.0 µl dNTP mix (10 mM each), 0.5 µl RNAsin® (20u) (Ambion, Inc., Hialeah, FL), and 0.5 µl MMLV reverse transcriptase (50u) (Ambion, Inc.). The contents were mixed by pipetting up and down, and the reaction mixture was incubated at 42°C for 1 hour. The contents of each tube were centrifuged prior to amplification.

An amplification mixture was prepared using ABI sybr master mix, plus 0.175 pmol of each oligonucleotide. SYBR® Green (Molecular Probes, Eugene, OR) is a dye which fluoresces when bound to double-stranded DNA. As double stranded PCR product is produced during amplification, the fluorescence from SYBR® Green increases. To each 20 µl aliquot of amplification mixture, 2 µl of template RT was added, and amplification carried out according to standard protocols. The results were expressed as the percent decrease in expression of the corresponding gene product relative to non-transfected cells, vehicle-only transfected (mock-transfected) cells, or cells transfected with reverse control oligonucleotides.

Although LIV-1 oligonucleotides inhibited Liv-1 expression in all cell lines tested, LIV-1 oligonucleotides had functional consequences only in tumorigenic lines. No functional consequences of the LIV-1 oligonucleotides were observed in non-tumorigenic lines, indicating that cancer cells and "normal" cells have differing dependencies on Liv1 activity.

**Example 5: Effect of Expression on Proliferation**

The effect of gene expression on the inhibition of cell proliferation was assessed in several cell lines including T47D1, T47D-T1, MCF7, ZR-75-1, 184B5, MDA231 and HMEC cells using siRNA methodologies.

Cells were plated to a density that will be about 80-95% confluent after days in 96-well dishes. Oligonucleotides (antisense or siRNA) were diluted to 2 µM in OptiMEM™. The oligonucleotide-OptiMEM™ was then added to a delivery vehicle, selected so as to be optimized for the particular cell type to be used in the assay. The oligo/delivery vehicle mixture was then further diluted into medium with serum on the cells. The final concentration of antisense oligonucleotides was about 300 nM and the final concentration of siRNA oligonucleotides was 67-100 nM.

Oligonucleotides were prepared as described above. Cells were transfected from about 4 hours to overnight at 37°C and the transfection mixture was replaced with fresh medium. Transfection was carried out as described above.

LIV-1 oligonucleotides inhibited of proliferation cancer cells but did not inhibit proliferation of HMEC (primary breast epithelial cells) or 184B5 (non-tumorigenic breast epithelial cell line) cells, indicating that LIV-1 plays a role in production and/or maintenance of the cancerous phenotype in cancer cells.

**Example 6: Survival assay**

To assess the effect of depletion of a target message upon cell death, T47D-T1, MCF7, Zr-75-1, and MDA-MB231 cells were transfected for cytotoxicity assays. Cytotoxicity was monitored by measuring the amount of LDH enzyme released in the medium due to membrane damage. The activity of LDH was measured using the Cytotoxicity Detection Kit from Roche Molecular Biochemicals. The data is provided as a ratio of LDH released in the medium vs. the total LDH present in the well at the same time point and treatment (rLDH/tLDH). A positive control using antisense and reverse control oligonucleotides for BCL2 (a known anti-apoptotic gene) is included; loss of message for BCL2 leads to an increase in cell death compared with treatment with the control oligonucleotide (background cytotoxicity due to transfection).

**Example 7: Caspase/M30 methodology**

Procaspase and M30 were assessed using western analyses on lysates from cells treated with siRNA. At various time points post-treatment, cells (both adherent & detached cells, which are spun down) were lysed and subjected to western analyses using antibodies to Procaspase and M30. Disappearance of procaspase corresponded to caspase activation. The appearance of the M30 epitope was reflective of caspase cleavage of cytokeratin 18. Antibodies used were Axxora/Alexis M30 (CytoDeath: CAT# ALX-804-590) and Procaspase Ab (R&D Systems, cat no. MAB707).

**Example 8: Cyclin D1 methodology**

Cells were transfected with siRNA to knockdown Liv1 or treated with Liv-1 specific Abs. For transected cells, lysates were collected 48 hours post-transfection. For Ab treatment, lysates were collected 6 hours post-treatment. Lysates were subjected to Western analyses, using anti-cyclin D1 Ab: cyclinD1 Abcam Cat#-ab24249. The siRNAs used were SEQ ID NOS:369 and 370. LIV-1 Abs tested were generated in-house against N-terminus & TM2-3 ECD. The antibody against the TM2-3 ECD appeared to show the greatest effect.

**Example 9: Zinc levels**

Cells were treated with siRNA 24, 48 or 72 hours prior to example G or with Abs 30 minutes prior to Example 10. The siRNAs used were SEQ ID NOS:369 and 370. LIV-1 Abs tested were generated in-house against N-terminus & TM2-3 ECD. The antibody against the TM2-3 ECD appeared to show the greatest effect.

**Example 10: Zinc Transport Assay**

Zinc transport was assayed using the cell-permeant zinc-selective fluorescent indicator, Newport Green. Upon cleavage by intracellular esterases, Newport Green binds to free zinc ions and acts as a fluorescent indicator for intracellular free zinc content.

A working solution of 10 µM Newport Green PDX dye (permeant ester form, Cat # N24191, Invitrogen- Molecular Probes™) and 0.02% Pluronic F-127 (20% Pluronic F-127 in DMSO; Cat # P3000, Invitrogen-Molecular Probes™) in Krebs-Ringer-HEPES Buffer (KRH) buffer (120 mM NaCl, 25 mM HEPES, pH 7.5, 4.8 mM KCL, 1.2 mM KH₂PO₄, 1.2 mM MgSO₄, 1.3 mM CaCl₂) was prepared as follows. Newport Green was obtained in 50 µg aliquots that were stored desiccated at -20°C, protected from light and thawed just before use. A 2.5 mM stock solution of Newport Green dye was made by reconstituting 50 µg of Newport Green in 16.8 µL of anhydrous dimethyl sulfoxide (DMSO) (Cat # 276855-100mL, Sigma-Aldrich). The 10 µM working solution of Newport Green dye was prepared by adding an equal volume (16.8 µL) of the dispersing agent, Pluronic F-127, to the 2.5 mM stock solution and then diluting the entire volume of dye into 8.366 mL KRH buffer.

Adherent cells were washed once in phosphate-buffered saline (PBS) without Ca²⁺ and Mg²⁺ and harvested with enzyme-free, Hanks-based dissociation buffer (Invitrogen, Cat # 13150-016). Both the PBS and the dissociation buffer were equilibrated to 37°C before use. The pH of the cell suspension was then neutralized by the addition of an equal volume of growth media. The cells were collected by centrifugation at 1000 RPM for 5 minutes; the resulting cell pellet was resuspended in PBS at a concentration of 1 x 10⁶ cell/mL. For analysis, 1.5 x 10⁶ cells were aliquotted into a 5 ml polystyrene round-bottom FACS tube (Becton Dickinson, Cat # 352054). The cells were pelleted again by centrifugation and the PBS was removed.

The cell pellet was resuspended by vortexing in 0.5 mL of 10 µM Newport Green dye solution; the FACS tube was capped and incubated in a 30°C waterbath for 45 minutes. One mL of dye-free KRH buffer was added to the cell suspension and the cells were pelleted by centrifugation at 1000 RPM for 5 minutes. The supernatant was aspirated, the cells were washed again in 1 mL dye-free KRH buffer, the cell pellet was resuspended in 0.5 mL dye-free KRH buffer and incubated in a 30°C waterbath for 30 minutes. One mL of dye-free KRH buffer was added to the cell suspension and the cells were pelleted by centrifugation at 1000 RPM for 5 minutes. The supernatant was aspirated, the cells were washed again in 1 mL dye-free KRH buffer, and the cell pellet was resuspended by vortexing in 2.0 mL dye-free KRH buffer.

Intracellular fluorescence was monitored by FACS (FACSCalibur) (BD Biosciences). Cells were collected in real time and the Newport Green dye fluorescence (FITC setting) was plotted over time (collection settings at 2 million cells over 10 minutes). Following the establishment of a fluorescence baseline within 1.5 to 2 minutes of collection, 100 mM zinc chloride (Cat # Z0152-100G Sigma-Aldrich, dissolved in 29 mM HCl.) was added to the cells to a final concentration of 1-100 µM. The tube of cells was immediately returned to the FACS machine; zinc uptake was manifested by an instantaneous increase in fluorescence. The FACS data were exported to Flowjo software (Tree Star Inc, Ashland, Oregon) and analyzed using the Kinetics Platform. The median of the data was displayed with the moving average smoothing option and a graphical overlay was created in Layout Editor.

**Example 11: LIV-1 Epitopes**

Linear epitopes of LIV-1 for antibody recognition and preparation can be identified by any of numerous methods known in the art. Some example methods include probing antibody-binding ability of peptides derived from the amino acid sequence of the antigen-Binding can be assessed by using BIACORE or ELISA methods. Other techniques include exposing peptide libraries on planar solid support ("chip") to antibodies and detecting binding through any of multiple methods used in solid-phase screening. Additionally, phage display can be used to screen a library of peptides with selection of epitopes after several rounds of biopanning.

Table 1 below provides regions of LIV-1 (SEQ ID NO:2) that have been identified as linear epitopes suitable for recognition by anti-LIV1 antibodies.

| **Table 1** | | | | |
|---|---|---|---|---|
| ECD name | Mapped AA seq loc | Mapped epitope location | epitope sequence | SEQ ID NO: |
| Antigenic region 1 ECD#'1 | 92-147 | 93-100 | HHDHDHHS | 6 |
| Antigenic region 1 ECD#'1 | 92-147 | 94-101 | HDHDHHSD | 7 |
| Antigenic region 1 ECD#'1 | 92-147 | 95-102 | DHDHHSDH | 8 |
| Antigenic region 1 ECD#'1 | 92-147 | 96-103 | HDHHSDHE | 9 |
| Antigenic region 1 ECD#'1 | 92-147 | 97-104 | DHHSDHEH | 10 |
| Antigenic region 1 ECD#'1 | 92-147 | 98-105 | HHSDHEHH | 11 |
| Antigenic region 1 ECD#'1 | 92-147 | 99-106 | HSDHEHHS | 12 |
| Antigenic region 1 ECD#'1 | 92-147 | 100-107 | SDHEHHSD | 13 |
| Antigenic region 1 ECD#'1 | 92-147 | 101-108 | DHEHHSDH | 14 |
| Antigenic region 1 ECD#'1 | 92-147 | 102-109 | HEHHSDHE | 15 |
| Antigenic region 1 ECD#'1 | 92-147 | 103-110 | EHHSDHER | 16 |
| Antigenic region 1 ECD#'1 | 92-147 | 104-111 | HHSDHERH | 17 |
| Antigenic region 1 ECD#'1 | 92-147 | 105-112 | HSDHERHS | 18 |
| Antigenic region 1 ECD#'1 | 92-147 | 106-113 | SDHERHSD | 19 |
| Antigenic region 1 ECD#'1 | 92-147 | 107-114 | DHERHSDH | 20 |
| Antigenic region 1 ECD#'1 | 92-147 | 108-115 | HERHSDHE | 21 |
| Antigenic region 1 ECD#'1 | 92-147 | 109-116 | ERHSDHEH | 22 |
| Antigenic region 1 ECD#'1 | 92-147 | 110-117 | RHSDHEHH | 23 |
| Antigenic region 1 ECD#'1 | 92-147 | 111-118 | HSDHEHHS | 24 |
| Antigenic region 1 ECD#'1 | 92-147 | 112-119 | SDHEHHSD | 25 |
| Antigenic region 1 ECD#'1 | 92-147 | 113-120 | DHEHHSDH | 26 |
| Antigenic region 1 ECD#'1 | 92-147 | 114-121 | HEHHSDHE | 27 |
| Antigenic region 1 ECD#'1 | 92-147 | 115-122 | EHHSDHEH | 28 |
| Antigenic region 1 ECD#'1 | 92-147 | 116-123 | HHSDHEHH | 29 |
| Antigenic region 1 ECD#'1 | 92-147 | 117-124 | HSDHEHHS | 30 |
| Antigenic region 1 ECD#'1 | 92-147 | 118-125 | SDHEHHSD | 31 |
| Antigenic region 1 ECD#'1 | 92-147 | 119-126 | DHEHHSDH | 32 |
| Antigenic region 1 ECD#'1 | 92-147 | 120-127 | HEHHSDHN | 33 |
| Antigenic region 1 ECD#'1 | 92-147 | 121-128 | EHHSDHNH | 34 |
| Antigenic region 1 ECD#'1 | 92-147 | 122-129 | HHSDHNHA | 35 |
| Antigenic region 1 ECD#'1 | 92-147 | 123-130 | HSDHNHAA | 36 |
| Antigenic region 1 ECD#'1 | 92-147 | 124-131 | SDHNHAAS | 37 |
| Antigenic region 1 ECD#'1 | 92-147 | 125-132 | DHNHAASG | 38 |
| Antigenic region 1 ECD#'1 | 92-147 | 126-133 | HNHAASGK | 39 |
| Antigenic region 1 ECD#'1 | 92-147 | 127-134 | NHAASGKN | 40 |
| Antigenic region 1 ECD#'1 | 92-147 | 128-135 | HAASGKNK | 41 |
| Antigenic region 1 ECD#' 1 | 92-147 | 129-136 | AASGKNKR | 42 |
| Antigenic region 1 ECD#'1 | 92-147 | 130-137 | ASGKNKRK | 43 |
| Antigenic region 1 ECD#'1 | 92-147 | 131-138 | SGKNKRKA | 44 |
| Antigenic region 1 ECD#'1 | 92-147 | 132-139 | GKNKRKAL | 45 |
| Antigenic region 1 ECD#'1 | 92-147 | 133-140 | KNKRKALC | 46 |
| Antigenic region 1 ECD#'1 | 92-147 | 134-141 | NKRKALCP | 47 |
| Antigenic region 1 ECD#'1 | 92-147 | 135-142 | KRKALCPD | 48 |
| Antigenic region 1 ECD#'1 | 92-147 | 136-143 | RKALCPDH | 49 |
| Antigenic region 1 ECD#'1 | 92-147 | 137-144 | KALCPDHD | 50 |
| Antigenic region 1 ECD#'1 | 92-147 | 138-145 | ALCPDHDS | 51 |
| Antigenic region 1 ECD#'1 | 92-147 | 139-146 | LCPDHDSD | 52 |
| Antigenic region 1 ECD#'1 | 92-147 | 140-147 | CPDHDSDS | 53 |
| Antigenic region 1 ECD#'1 | 92-147 | 93-101 | HHDHDHHSD | 54 |
| Antigenic region 1 ECD#'1 | 92-147 | 94-102 | HDHDHHSDH | 55 |
| Antigenic region 1 ECD#'1 | 92-147 | 95-103 | DHDHHSDHE | 56 |
| Antigenic region 1 ECD#'1 | 92-147 | 96-104 | HDHHSDHEH | 57 |
| Antigenic region 1 ECD#'1 | 92-147 | 97-105 | DHHSDHEHH | 58' |
| Antigenic region 1 ECD#'1 | 92-147. | 98-106 | HHSDHEHHS | 59 |
| Antigenic region 1 ECD#'1 | 92-147 | 99-107 | HSDHEHHSD | 60 |
| Antigenic region 1 ECD#'1 | 92-147 | 100-108 | SDHEHHSDH | 61 |
| Antigenic region 1 ECD#'1 | 92-147 | 101-109 | DHEHHSDHE | 62 |
| Antigenic region 1 ECD#'1 | 92-147 | 102-110 | HEHHSDHER | 63 |
| Antigenic region 1 ECD#'1 | 92-147 | 103-111 | EHHSDHERH | 64 |
| Antigenic region 1 ECD#'1 | 92-147 | 104-112 | HHSDHERHS | 65 |
| Antigenic region 1 ECD#'1 | 92-147 | 105-113 | HSDHERHSD | 66 |
| Antigenic region 1 ECD#'1 | 92-147 | 106-114 | SDHERHSDH | 67 |
| Antigenic region 1 ECD#'1 | 92-147 | 107-115 | DHERHSDHE | 68 |
| Antigenic region 1 ECD#'1 | 92-147 | 108-116 | HERHSDHEH | 69 |
| Antigenic region 1 ECD#'1 | 92-147 | 109-117 | ERHSDHEHH | 70 |
| Antigenic region 1 ECD#'1 | 92-147 | 110-118 | RHSDHEHHS | 71 |
| Antigenic region 1 ECD#'1 | 92-147 | 111-119 | HSDHEHHSD | 72 |
| Antigenic region 1 ECD#'1 | 92-147 | 112-120 | SDHEHHSDH | 73 |
| Antigenic region 1 ECD#'1 | 92-147 | 113-121 | DHEHHSDHE | 74 |
| Antigenic region 1 ECD#'1 | 92-147 | 114-122 | HEHHSDHEH | 75 |
| Antigenic reqion 1 ECD#'1 | 92-147 | 115-123 | EHHSDHEHH | 76 |
| Antigenic region 1 ECD#'1 | 92-147 | 116-124 | HHSDHEHHS | 77 |
| Antigenic region 1 ECD#'1 | 92-147 | 117-125 | . HSDHEHHSD | 78 |
| Antigenic region 1 ECD#'1 | 92-147 | 118-126 | SDHEHHSDH | 79 |
| Antigenic region 1 ECD#'1 | 92-147 | 1.19-127 | DHEHHSDHN | 80 |
| Antigenic region 1 ECD#'1 | 92-147 | 120-128 | HEHHSDHNH | 81 |
| Antigenic region 1 ECD#'1 | 92-147 | 121-129 | EHHSDHNHA | 82 |
| Antigenic region 1 ECD#'1 | 92-147 | 122-130 | HHSDHNHAA | 83 |
| Antigenic region 1 ECD#'1 | 92-147 | 123-131 | HSDHNHAAS | 84 |
| Antigenic region 1 ECD#'1 | 92-147 | 124-132 | SDHNHAASG | 85 |
| Antigenic region 1 ECD#'1 | 92-147 | 125-133 | DHNHAASGK | 86 |
| Antigenic region 1 ECD#'1 | 92-147 | 126-134 | HNHAASGKN | 87 |
| Antigenic region 1 ECD#'1 | 92-147 | 127-135 | NHAASGKNK | 88 |
| Antigenic region 1 ECD#'1 | 92-147 | 128-136 | HAASGKNKR | 89 |
| Antigenic region 1 ECD#'1 | 92-147 | 129-137 | AASGKNKRK | 90 |
| Antigenic region 1 ECD#'1 | 92-147 | 130-138 | ASGKNKRKA | 91 |
| Antigenic region 1 ECD#'1 | 92-147 | 131-139 | SGKNKRKAL | 92 |
| Antigenic region 1 ECD#'1 | 92-147 | 132-140 | GKNKRKALC | 93 |
| Antigenic region 1 ECD#'1 | 92-147 | 133-141 | KNKRKALCP | 94 |
| Antigenic region 1 ECD#'1 | 92-147 | 134-142 | NKRKALCPD | 95 |
| Antigenic region 1 ECD#'1 | 92-147 | 135-143 | KRKALCPDH | 96 |
| Antigenic region 1 ECD#'1 | 92-147 | 136-144 | RKALCPDHD | 97 |
| Antigenic region 1 ECD#' 1 | 92-147 | 137-145 | KALCPDHDS | 98 |
| Antigenic region 1 ECD#'1 | 92-147 | 138-146 | ALCPDHDSD | 99 |
| Antigenic region 1 ECD#'1 | 92-147 | 139-147 | LCPDHDSDS | 100 |
| Antigenic region 1 ECD#'1 | 92-147 | 93-102 | HHDHDHHSDH | 101 |
| Antigenic region 1 ECD#'1 | 92-147 | 94-103 | HDHDHHSDHE | 102 |
| Antigenic region 1 ECD#'1 | 92-147 | 95-104 | DHDHHSDHEH | 103 |
| Antigenic region 1 ECD#'1 | 92-147 | 96-105 | HDHHSDHEHH | 104 |
| Antigenic region 1 ECD#'1 | 92-147 | 97-106 | DHHSDHEHHS | 105 |
| Antigenic region 1 ECD#'1 | 92-147 | 98-107 | HHSDHEHHSD | 106 |
| Antigenic region 1 ECD#'1 | 92-147 | 99-108 | HSDHEHHSDH | 107 |
| Antigenic region 1 ECD#'1 | 92-147 | 100-109 | SDHEHHSDHE | . 108 |
| Antigenic region 1 ECD#'1 | 92-147 | 101-110 | DHEHHSDHER | 109 |
| Antigenic region 1 ECD#'1 | 92-147 | 102-111 | HEHHSDHERH | 110 |
| Antigenic region 1 ECD#'1 | 92-147 | 103-112 | EHHSDHERHS | 111 |
| Antigenic region 1 ECD# 1 | 92-147 | 104-113 | HHSDHERHSD | 112 |
| Antigenic region 1 ECD#'1 | 92-147 | 105-114 | HSDHERHSDH | 113 |
| Antigenic region 1 ECD#'1 | 92-147 | 106-115 | SDHERHSDHE | 114 |
| Antigenic region 1 ECD#'1 | 92-147 | 107-116 | DHERHSDHEH | 115 |
| Antigenic region 1 ECD#'1 | 92-147 | 108-117 | HERHSDHEHH | 116 |
| Antigenic region 1 ECD#'1 | 92-147 | 109-118 | ERHSDHEHHS | 117 |
| Antigenic region 1 ECD#'1 | 92-147 | 110-119 | RHSDHEHHSD | 118 |
| Antigenic region 1 ECD#'1 | 92-147 | 111-120 | HSDHEHHSDH | 119 |
| Antigenic region 1 ECD#'1 | 92-147 | 112-121 | SDHEHHSDHE | 120 |
| Antigenic region 1 ECD#'1 | 92-147 | 113-122 | DHEHHSDHEH | 121 |
| Antigenic region 1 ECD#'1 | 92-147 | 114-123 | HEHHSDHEHH | 122 |
| Antigenic region 1 ECD#'1 | 92-147 | 115-124 | EHHSDHEHHS | 123 |
| Antigenic region 1 ECD#'1 | 92-147 | 116-125 | HHSDHEHHSD | 124 |
| Antigenic region 1 ECD#'1 | 92-147 | 117-126 | HSDHEHHSDH | 125 |
| Antigenic region 1 ECD#'1 | 92-147 | 118-127 | SDHEHHSDHN | 126 |
| Antigenic region 1 ECD#'1 | 92-147 | 119-128 | DHEHHSDHNH | 127 |
| Antigenic region 1 ECD#'1 | 92-147 | 120-129 | HEHHSDHNHA | 128 |
| Antigenic region 1 ECD#'1 | 92-147 | 121-130 | EHHSDHNHAA | 129 |
| Antigenic region 1 ECD#'1 | 92-147 | 122-131 | HHSDHNHAAS | 130 |
| Antigenic region 1 ECD#'1 | 92-147 | 123-132 | HSDHNHAASG | 131 |
| Antigenic region 1 ECD#'1 | 92-147 | 124-133 | SDHNHAASGK | 132 |
| Antigenic region 1 ECD#'1 | 92-147 | 125-134 | DHNHAASGKN | 133 |
| Antigenic region 1 ECD#'1 | 92-147 | 126-135 | HNHAASGKNK | 134 |
| Antigenic region 1 ECD#'1 | 92-147 | 127-136 | NHAASGKNKR | 135 |
| Antigenic region 1 ECD#'1 | 92-147 | 128-137 | HAASGKNKRK | 136 |
| Antigenic region 1 ECD#'1 | 92-147 | 129-138 | AASGKNKRKA | 137 |
| Antigenic region 1 ECD#'1 | 92-147 | 130-139 | ASGKNKRKAL | 138 |
| Antigenic region 1 ECD#'1 | 92-147 | 131-140 | SGKNKRKALC | 139 |
| Antigenic region 1 ECD#'1 | 92-147 | 132-141 | GKNKRKALCP | 140 |
| Antigenic region 1 ECD#'1 | 92-147 | 133-142 | KNKRKALCPD | 141 |
| Antigenic region 1 ECD#'1 | 92-147 | 134-143 | NKRKALCPDH | 142 |
| Antigenic region 1 ECD#'1 | 92-147 | 135-144 | KRKALCPDHD | 143 |
| Antigenic region 1 ECD#'1 | 92-147 | 136-145 | RKALCPDHDS | 144 |
| Antigenic region 1 ECD#'1 | 92-147 | 137-146 | KALCPDHDSD | 145 |
| Antigenic region 1 ECD#'1 | 92-147 | 138-147 | ALCPDHDSDS | 146 |
| Antigenic region 1 ECD#'1 | 92-147 | 93-103 | HHDHDHHSDHE | 147 |
| Antigenic region 1 ECD#'1 | 92-147 | 94-104 | HDHDHHSDHEH | 148 |
| Antigenic region 1 ECD#'1 | 92-147 | 95-105 | DHDHHSDHEHH | 149 |
| Antigenic region 1 ECD#'1 | 92-147 | 96-106 | HDHHSDHEHHS | 150 |
| Antigenic region 1 ECD#'1 | 92-147 | 97-107 | DHHSDHEHHSD | 151 |
| Antigenic region 1 ECD#'1 | 92-147 | 98-108 | HHSDHEHHSDH | 152 |
| Antigenic region 1 ECD#'1 | 92-147 | 99-109 | HSDHEHHSDHE | 153 |
| Antigenic region 1 ECD#'1 | 92-147 | 100-110 | SDHEHHSDHER | 154 |
| Antigenic region 1 ECD#'1 | 92-147 | 101-111 | DHEHHSDHERH | 155 |
| Antigenic region 1 ECD#'1 | 92-147 | 102-112 | HEHHSDHERHS | 156 |
| Antigenic region 1 ECD#'1 | 92-147 | 103-113 | EHHSDHERHSD | 157 |
| Antigenic region 1 ECD#'1 | 92-147 | 104-114 | HHSDHERHSDH | 158 |
| Antigenic region 1 ECD#'1 | 92-147 | 105-115 | HSDHERHSDHE | 159 |
| Antigenic region 1 ECD#'1 | 92-147 | 106-116 | SDHERHSDHEH | 160 |
| Antigenic region 1 ECD#'1 | 92-147 | 107-117 | DHERHSDHEHH | 161 |
| antigenic region 1 ECD#'1 | 92-147 | 108-118 | HERHSDHEHHS | 162 |
| Antigenic region 1 ECD#'1 | 92-147 | 109-119 | ERHSDHEHHSD | 163 |
| Antigenic region 1 ECD#'1 | 92-147 | 110-120 | RHSDHEHHSDH | 164 |
| Antigenic region 1 ECD#'1 | 92-147 | 111-121 | HSDHEHHSDHE | 165 |
| Antigenic region 1 ECD#'1 | 92-147 | 112-122 | SDHEHHSDHEH | 166 |
| Antigenic region 1 ECD#'1 | 92-147 | 113-123 | DHEHHSDHEHH | 167 |
| Antigenic region 1 ECD#'1 | 92-147 | 114-124 | HEHHSDHEHHS | 168 |
| Antigenic region 1 ECD#'1 | 92-147 | 115-125 | EHHSDHEHHSD | 169 |
| Antigenic region 1 ECD#'1 | 92-147 | 116-126 | HHSDHEHHSDH | 170 |
| Antigenic region 1 ECD#'1 | 92-147 | 117-127 | HSDHEHHSDHN | 171 |
| Antigenic region 1 ECD#'1 | 92-147 | 118-128 | SDHEHHSDHNH | 172 |
| Antigenic region 1 ECD#'1 | 92-147 | 119-129 | DHEHHSDHNHA | 173 |
| Antigenic region 1 ECD#'1 | 92-147 | 120-130 | HEHHSDHNHAA | 174 |
| Antigenic region 1 ECD#'1 | 92-147 | 121-131 | EHHSDHNHAAS | 175 |
| Antigenic region 1 ECD#'1 | 92-147 | 122-132 | HHSDHNHAASG | 176 |
| Antigenic region 1 ECD#'1 | 92-147 | 123-133 | HSDHNHAASGK | 177 |
| Antigenic region 1 ECD#'1 | 92-147 | 124-134 | SDHNHAASGKN | 178 |
| Antigenic region 1 ECD#'1 | 92-147 | 125-135 | DHNHAASGKNK | 179 |
| Antigenic region 1 ECD#'1 | 92-147 | 126-136 | HNHAASGKNKR | 180 |
| Antigenic region 1 ECD#'1 | 92-147 | 127-137 | NHAASGKNKRK | 181 |
| Antigenic region 1 ECD#'1 | 92-147 | 128-138 | HAASGKNKRKA | 182 |
| Antigenic region 1 ECD#'1 | 92-147 | 129-139 | AASGKNKRKAL | 183 |
| Antigenic region 1 ECD#'1 | 92-147 | 130-140 | ASGKNKRKALC | 184 |
| Antigenic region 1 ECD#'1 | 92-147 | 131-141 | SGKNKRKALCP | 185 |
| Antigenic region 1 ECD#'1 | 92-147 | 132-142 | GKNKRKALCPD | 186 |
| Antigenic region 1 ECD#'1 | 92-147 | 133-143 | KNKRKALCPDH | 187 |
| Antigenic region 1 ECD#'1 | 92-147 | 134-144 | NKRKALCPDHD | 188 |
| Antigenic region 1 ECD#'1 | 92-147 | 135-145 | KRKALCPDHDS | 189 |
| Antigenic region 1 ECD#'1 | 92-147 | 136-146 | RKALCPDHDSD | 190 |
| Antigenic region 1 ECD#'1 | 92-147 | 137-147 | KALCPDHDSDS | 191 |
| Antigenic region 1 ECD#'1 | 92-147 | 93-104 | HHDHDHHSDHEH | 192 |
| Antigenic region 1 ECD#'1 | 92-147 | 94-105 | HDHDHHSDHEHH | 193 |
| Antigenic region 1 ECD#'1 | 92-147 | 95-106 | DHDHHSDHEHHS | 194 |
| Antigenic region 1 ECD#'1 | 92-147 | 96-107 | HDHHSDHEHHSD | 195 |
| Antigenic region 1 ECD#'1 | 92-147 | 97-108 | DHHSDHEHHSDH | 196 |
| Antigenic region 1 ECD#'1 | 92-147 | 98-109 | HHSDHEHHSDHE | 197 |
| Antigenic region 1 ECD#'1 | 92-147 | 99-110 | HSDHEHHSDHER | 198 |
| Antigenic region 1 ECD#'1 | 92-147 | 100-111 | SDHEHHSDHERH | 199 |
| Antigenic region 1 ECD#'1 | 92-147 | 101-112 | DHEHHSDHERHS | 200 |
| Antigenic region 1 ECD#'1 | 92-147 | 102-113 | HEHHSDHERHSD | 201 |
| Antigenic region 1 ECD#'1 | 92-147 | 103-114 | EHHSDHERHSDH | 202 |
| Antigenic region 1 ECD#'1 | 92-147 | 104-115 | HHSDHERHSDHE | 203 |
| Antigenic region 1 ECD#'1 | 92-147 | 105-116 | HSDHERHSDHEH | 204 |
| Antigenic region 1 ECD#'1 | 92-147 | 106-117 | SDHERHSDHEHH | 205 |
| Antigenic region 1 ECD#'1 | 92-147 | 107-118 | DHERHSDHEHHS | 206 |
| Antigenic region 1 ECD#'1 | 92-147 | 108-119 | HERHSDHEHHSD | 207 |
| Antigenic region 1 ECD#'1 | 92-147 | 109-120 | ERHSDHEHHSDH | 208 |
| Antigenic region 1 ECD#'1 | 92-147 | 110-121 | RHSDHEHHSDHE | 209 |
| Antigenic region 1 ECD#'1 | 92-147 | 111-122 | HSDHEHHSDHEH | 210 |
| Antigenic region 1 ECD#'1 | 92-147 | 112-123 | SDHEHHSDHEHH | 211 |
| Antigenic region 1 ECD#'1 | 92-147 | 113-124 | DHEHHSDHEHHS | 212 |
| Antigenic region 1 ECD#'1 | 92-147 | 114-125 | HEHHSDHEHHSD | 213 |
| Antigenic region 1 ECD#'1 | 92-147 | 115-126 | EHHSDHEHHSDH | 214 |
| Antigenic region 1 ECD#'1 | 92-147 | 116-127 | HHSDHEHHSDHN | 215 |
| Antigenic region 1 ECD#'1 | 92-147 | 117-128 | HSDHEHHSDHNH | 216 |
| Antigenic region 1 ECD#'1 | 92-147 | 118-129 | SDHEHHSDHNHA | 217 |
| Antigenic region 1 ECD#'1 | 92-147 | 119-130 | DHEHHSDHNHAA | 218 |
| Antigenic region 1 ECD#'1 | 92-147 | 120-131 | HEHHSDHNHAAS | 219 |
| Antigenic region 1 ECD#'1 | 92-147 | 121-132 | EHHSDHNHAASG | 220 |
| Antigenic region 1 ECD#'1 | 92-147 | 122-133 | HHSDHNHAASGK | 221 |
| Antigenic region 1 ECD#'1 | 92-147 | 123-134 | HSDHNHAASGKN | 222 |
| Antigenic region 1 ECD#'1 | 92-147 | 124-135 | SDHNHAASGKNK | 223 |
| Antigenic region 1 ECD#'1 | 92-147 | 125-136 | DHNHAASGKNKR | 224 |
| Antigenic region 1 ECD#'1 | 92-147 | 126-137 | HNHAASGKNKRK | 225 |
| Antigenic region 1 ECD#'1 | 92-147 | 127-138 | NHAASGKNKRKA | 226 |
| Antigenic region 1 ECD#'1 | 92-147 | 128-139 | HAASGKNKRKAL | 227 |
| Antigenic region 1 ECD#'1 | 92-147 | 129-140 | AASGKNKRKALC | 228 |
| Antigenic region 1 ECD#'1 | 92-147 | 130-141 | ASGKNKRKALCP | 229 |
| Antigenic region 1 ECD#'1 | 92-147 | 131-142 | SGKNKRKALCPD | 230 |
| Antigenic region 1 ECD#'1 | 92-147 | 132-143 | GKNKRKALCPDH | 231 |
| Antigenic region 1 ECD#'1 | 92-147 | 133-144 | KNKRKALCPDHD | 232 |
| Antigenic region 1 ECD#'1 | 92-147 | 134-145 | NKRKALCPDHDS | 233 |
| Antigenic region 1 ECD#'1 | 92-147 | 135-146 | .KRKALCPDHDSD | 234 |
| Antigenic region 1 ECD#'1 | 92-147 | 136-147 | RKALCPDHDSDS | 235 |
| Antigenic region 2 ECD#'1 | 158-180 | 158-165 | KGAHRPEH | 236 |
| Antigenic region 2 ECD#'1 | 158-180 | 159-166 | GAHRPEHA | 237 |
| Antigenic region 2 ECD#'1 | 158-180 | 160-167 | AHRPEHAS | 238 |
| Antigenic region 2 ECD#'1 | 158-180 | '161-168 | HRPEHASG | 239 |
| Antigenic region 2 ECD#'1 | 158-180 | 162-169 | RPEHASGR | 240 |
| Antigenic region 2 ECD#'1 | 158-180 | 163-170 | PEHASGRR | 241 |
| Antigenic region 2 ECD#'1 | 158-180 | 164-171 | EHASGRRN | 242 |
| Antigenic region 2 ECD#'1 | 158-180 | 165-172 | HASGRRNV | 243 |
| Antigenic region 2 ECD#'1 | 158-180 | 166-173 | ASGRRNVK | 244 |
| Antigenic region 2 ECD#'1 | 158-180 | 167-174 | SGRRNVKD | 245 |
| Antigenic region 2 ECD#'1 | 158-180 | 168-175 | GRRNVKDS | 246 |
| Antigenic region 2 ECD#'1 | 158-180 | 169-176 | RRNVKDSV | 247 |
| Antigenic region 2 ECD#'1 | 158-180 | 170-177 | RNVKDSVS | 248 |
| Antigenic region 2 ECD#'1 | 158-180 | 171-178 | NVKDSVSA | 249 |
| Antigenic region 2 ECD#'1 | 158-180 | 172-179 | VKDSVSAS | 250 |
| Antigenic region 2 ECD#'1 | 158-180 | 173-180 | KDSVSASE | 251 |
| Antigenic region 2 ECD#'1 | 158-180 | 158-166 | KGAHRPEHA | 252 |
| Antigenic region 2 ECD#'1 | 158-180 | 159-167 | GAHRPEHAS | 253 |
| Antigenic region 2 ECD#'1 | 158-180 | 160-168 | AHRPEHASG | 254 |
| Antigenic region 2 ECD#'1 | 158-180 | 161-169 | HRPEHASGR | 255 |
| Antigenic region 2 ECD#'1 | 158-180 | 162-170 | RPEHASGRR | 256 |
| Antigenic region 2 ECD#'1 | 158-180 | 163-171 | PEHASGRRN | 257 |
| Antigenic region 2 ECD#'1 | 158-180 | 164-172 | EHASGRRNV | 258 |
| Antigenic region 2 ECD#'1 | 158-180 | 165-173 | HASGRRNVK | 259 |
| Antigenic region 2 ECD#'1 | 158-180 | 166-174 | ASGRRNVKD | 260 |
| Antigenic region 2 ECD#'1 | 158-180 | 167-175 | SGRRNVKDS | 261 |
| Antigenic region 2 ECD#'1 | 158-180 | 168-176 | GRRNVKDSV | 262 |
| Antigenic region 2 ECD#'1 | 158-180 | 169-177 | RRNVKDSVS | 263 |
| Antigenic region 2 ECD#'1 | 158-180 | 170-178 | RNVKDSVSA | 264 |
| Antigenic region 2 ECD#'1 | 158-180 | 171-179 | NVKDSVSAS | 265 |
| Antigenic region 2 ECD#'1 | 158-180 | 172-180 | VKDSVSASE | 266 |
| Antigenic region 2 ECD#'1 | 158-180 | 158-167 | KGAHRPEHAS | 267 |
| Antigenic region 2 ECD#'1 | 158-180 | 159-168 | GAHRPEHASG | 268 |
| Antigenic region 2 ECD#'1 | 158-180 | 160-169 | AHRPEHASGR | 269 |
| Antigenic region 2 ECD#'1 | 158-180 | 161-170 | HRPEHASGRR | 270 |
| Antigenic region 2 ECD#'1 | 158-180 | 162-171 | RPEHASGRRN | 271 |
| Antigenic region 2 ECD#'1 | 158-180 | 163-172 | PEHASGRRNV | 272 |
| Antigenic region 2 ECD#'1 | 158-180 | 164-173 | EHASGRRNVK | 273 |
| Antigenic region 2 ECD#'1 | 158-180 | 165-174 | HASGRRNVKD | 274 |
| Antigenic region 2 ECD#'1 | 158-180 | 166-175 | ASGRRNVKDS | 275 |
| Antigenic region 2 ECD#'1 | 158-180 | 167-176 | SGRRNVKDSV | 276 |
| Antigenic region 2 ECD#'1 | 158-180 | 168-177 | GRRNVKDSVS | 277 |
| Antigenic region 2 ECD#'1 | 158-180 | 169-178 | RRNVKDSVSA | 278 |
| Antigenic region 2 ECD#'1 | 158-180 | 170-179 | RNVKDSVSAS | 279 |
| Antigenic region 2 ECD#'1 | 158-180 | 171-180 | NVKDSVSASE | 280 |
| Antigenic region 2 ECD#'1 | 158-180 | 158-168 | KGAHRPEHASG | 281 |
| Antigenic region 2 ECD#'1 | 158-180 | 159-169 | GAHRPEHASGR | 282 |
| Antigenic region 2 ECD#'1 | 158-180 | 160-170 | AHRPEHASGRR | 283 |
| Antigenic region 2 ECD#'1 | 158-180 | 161-171 | HRPEHASGRRN | 284 |
| Antigenic region 2 ECD#'1 | 158-180 | 162-172 | RPEHASGRRNV | 285 |
| Antigenic region 2 ECD#'1 | 158-180 | 163-173 | PEHASGRRNVK | 286 |
| Antigenic region 2 ECD#'1 | 158-180 | 164-174 | EHASGRRNVKD | 287 |
| Antigenic region 2 ECD#'1 | 158-180 | 165-175 | HASGRRNVKDS | 288 |
| Antigenic region 2 ECD#'1 | 158-180 | 166-176 | ASGRRNVKDSV | 289 |
| Antigenic region 2 ECD#'1 | 158-180 | 167-177 | SGRRNVKDSVS | 290 |
| Antigenic region 2 ECD#'1 | 158-180 | 168-178 | GRRNVKDSVSA | 291 |
| Antigenic region 2 ECD#'1 | 158-180 | 169-179 | RRNVKDSVSAS | 292 |
| Antigenic region 2 ECD#'1 | 158-180 | 170-180 | RNVKDSVSASE | 293 |
| Antigenic region 2 ECD#'1 | 158-180 | 158-169 | KGAHRPEHASGR | 294 |
| Antigenic region 2 ECD#'1 | 158-180 | 159-170 | GAHRPEHASGRR | 295 |
| Antigenic region 2 ECD#'1 | 158-180 | 160-171 | AHRPEHASGRRN | 296 |
| Antigenic region 2 ECD#'1 | 158-180 | 161-172 | HRPEHASGRRNV | 297 |
| Antigenic region 2 ECD#'1 | 158-180 | 162-173 | RPEHASGRRNVK | 298 |
| Antigenic region 2 ECD#'1 | 158-180 | 163-174 | PEHASGRRNVKD | 299 |
| Antigenic region 2 ECD#'1 | 158-180 | 164-175 | EHASGRRNVKDS | 300 |
| Antigenic region 2 ECD#'1 | 158-180 | 165-176 | HASGRRNVKDSV | 301 |
| Antigenic region 2 ECD#'1 | 158-180 | 166-177 | ASGRRNVKDSVS | 302 |
| Antigenic region 2 ECD#'1 | 158-180 | 167-178 | SGRRNVKDSVSA | 303 |
| Antigenic region 2 ECD#'1 | 158-180 | 168-179 | GRRNVKDSVSAS | 304 |
| Antigenic region 2 ECD#'1 | 158-180 | 169-180 | RRNVKDSVSASE | 305 |
| Antigenic region 3 ECD#'2 | 360-379 | 360-367 | HLLPHSHA | 306 |
| Antigenic region 3 ECD#'2 | 360-379 | 361-368 | LLPHSHAS | 307 |
| Antigenic region 3 ECD#'2 | 360-379 | 362-369 | LPHSHASH | 308 |
| Antigenic region 3 ECD#'2 | 360-379 | 363-370 | PHSHASHH | 309 |
| Antigenic region 3 ECD#'2 | 360-379 | 364-371 | HSHASHHH | 310 |
| Antigenic region 3 ECD#'2 | 360-379 | 365-372 | SHASHHHS | 311 |
| Antigenic region 3 ECD#'2 | 360-379 | 366-373 | HASHHHSH | 312 |
| Antigenic region 3 ECD#'2 | 360-379 | 367-374 | ASHHHSHS | 313 |
| Antigenic region 3 ECD#'2 | 360-379 | 368-375 | SHHHSHSH | 314 |
| Antigenic region 3 ECD#'2 | 360-379 | 369-376 | HHHSHSHE | 315 |
| Antigenic region 3 ECD#'2 | 360-379 | 370-377 | HHSHSHEE | 316 |
| Antigenic region 3 ECD#'2 | 360-379 | 371-378 | HSHSHEEP | 317 |
| Antigenic region 3 ECD#'2 | 360-379 | 372-379 | SHSHEEPA | 318 |
| Antigenic region 3 ECD#'2 | 360-379 | 360-368 | HLLPHSHAS | 319 |
| Antigenic region 3 ECD#'2 | 360-379 | 361-369 | LLPHSHASH | 320 |
| Antigenic region 3 ECD#'2 | 360-379 | 362-370 | LPHSHASHH | 321 |
| Antigenic region 3 ECD#'2 | 360-379 | 363-371 | PHSHASHHH | 322 |
| Antigenic region 3 ECD#'2 | 360-379 | 364-372 | HSHASHHHS | 323 |
| Antigenic region 3 ECD#'2 | 360-379 | 365-373 | SHASHHHSH | 324 |
| Antigenic region 3 ECD#'2 | 360-379 | 366-374 | HASHHHSHS | 325 |
| Antigenic region 3 ECD#'2 | 360-379 | 367-375 | ASHHHSHSH | 326 |
| Antigenic region 3 ECD#'2 | 360-379 | 368-376 | SHHHSHSHE | 327 |
| Antigenic region 3 ECD#'2 | 360-379 | 369-377 | HHHSHSHEE | 328 |
| Antigenic region 3 ECD#'2 | 360-379 | 370-378 | HHSHSHEEP | 329 |
| Antigenic region 3 ECD#'2 | 360-379 | 371-379 | HSHSHEEPA | 330 |
| Antigenic region 3 ECD#'2 | 360-379 | 360-369 | HLLPHSHASH | 331 |
| Antigenic region 3 ECD#'2 | 360-379 | 361-370 | LLPHSHASHH | 332 |
| Antigenic region 3 ECD#'2 | 360-379 | 362-371 | LPHSHASHHH | 333 |
| Antigenic region 3 ECD#'2 | 360-379 | 363-372 | PHSHASHHHS | 334 |
| Antigenic region 3 ECD#'2 | 360-379 | 364-373 | HSHASHHHSH | 335 |
| Antigenic region 3 ECD#'2 | 360-379 | 365-374 | SHASHHHSHS | 336 |
| Antigenic region 3 ECD#'2 | 360-379 | 366-375 | HASHHHSHSH | 337 |
| Antigenic region 3 ECD#'2 | 360-379 | 367-376 | ASHHHSHSHE | 338 |
| Antigenic region 3 ECD#'2 | 360-379 | 368-377 | SHHHSHSHEE | 339 |
| Antigenic region 3 ECD#'2 | 360-379 | 369-378 | HHHSHSHEEP | 340 |
| Antigenic region 3 ECD#'2 | 360-379 | 370-379 | HHSHSHEEPA | 341 |
| Antigenic region 3 ECD#'2 | 360-379 | 360-370 | HLLPHSHASHH | 342 |
| Antigenic region 3 ECD#'2 | 360-379 | 361-371 | LLPHSHASHHH | 343 |
| Antigenic region 3 ECD#'2 | 360-379 | 362-372 | LPHSHASHHHS | 344 |
| Antigenic region 3 ECD#'2 | 360-379 | 363-373 | PHSHASHHHSH | 345 |
| Antigenic region 3 ECD#'2 | 360-379 | 364-374 | HSHASHHHSHS | 346 |
| Antigenic region 3 ECD#'2 | 360-379 | 365-375 | SHASHHHSHSH | 347 |
| Antigenic region 3 ECD#'2 | 360-379 | 366-376 | HASHHHSHSHE | 348 |
| Antigenic region 3 ECD#'2 | 360-379 | 367-377 | ASHHHSHSHEE | 349 |
| Antigenic region 3 ECD#'2 | 360-379 | 368-378 | SHHHSHSHEEP | 350 |
| Antigenic region 3 ECD#'2 | 360-379 | 369-379 | HHHSHSHEEPA | 351 |
| Antigenic region 3 ECD#'2 | 360-379 | 360-371 | HLLPHSHASHHH | 352 |
| Antigenic region 3 ECD#'2 | 360-379 | 361-372 | LLPHSHASHHHS | 353 |
| Antigenic region 3 ECD#'2 | 360-379 | 362-373 | LPHSHASHHHSH | 354 |
| Antigenic region 3 ECD#'2 | 360-379 | 363-374 | PHSHASHHHSHS | 355 |
| Antigenic region 3 ECD#'2 | 360-379 | 364-375 | HSHASHHHSHSH | 356 |
| Antigenic region 3 ECD#'2 | 360-379 | 365-376 | SHASHHHSHSHE | 357 |
| Antigenic region 3 ECD#'2 | 360-379 | 366-377 | HASHHHSHSHEE | 358 |
| Antigenic region 3 ECD#'2 | 360-379 | 367-378 | ASHHHSHSHEEP | 359 |
| Antigenic region 3 ECD#'2 | 360-379 | 368-379 | SHHHSHSHEEPA | 360 |

**Example 12: Immunohistochemistry**

Commercially available paraffin embedded human tissue microarrays were used to evaluate the expression of LIV-1 by means of immunohistochemistry. (Zymed Laboratories Inc, San Francisco CA; Cybrdi, Gaithersburg, MD; Clinomics Biosciences Inc, Cambridge UK). Naïve and LIV-1-transfected 293T cells were used as controls to validate the expression.

Tissue section deparaffinization and antigen retrieval were performed on the Ventana Discovery using standard cell conditioning followed by a 60-minute incubation in the primary antibodies. A rabbit anti-human LIV-1 antibody (Chiron, Emeryville, CA) and rabbit IgG Prebleed control (Chiron, Emeryville, CA) were used at 10ug/ml. Ventana Universal Secondary Reagent (Ventana Medical Systems, Inc., Tucson, Az) followed by Ventana DAB Map Kit (Ventana Medical Systems Inc.) was used for detection. Ventana Hematoxylin and Bluing Reagents (Ventana Medical Systems, Inc) were used for counter stain and sections were dehydrated in graded alcohols, cleared in xylene and coverslipped using a synthetic mounting media.

**Example 13: Cancer versus normal tissue; Spotfire analysis**

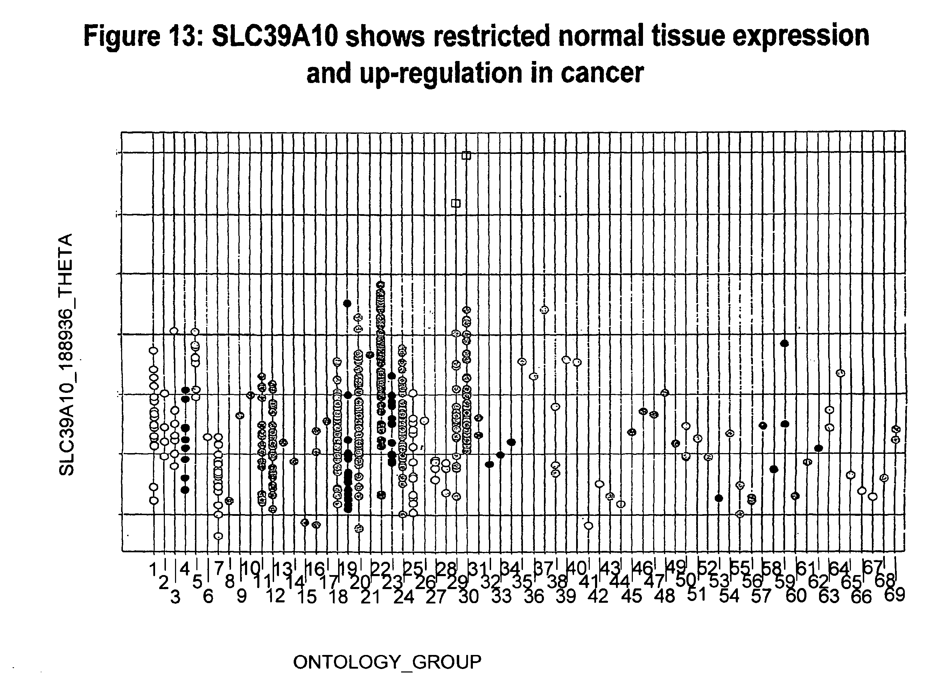

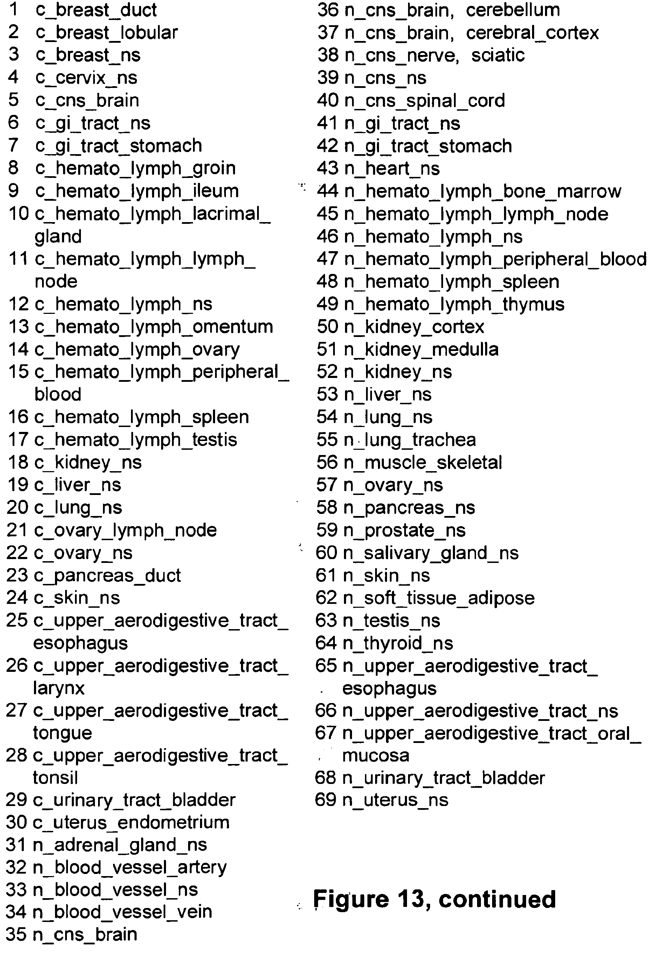

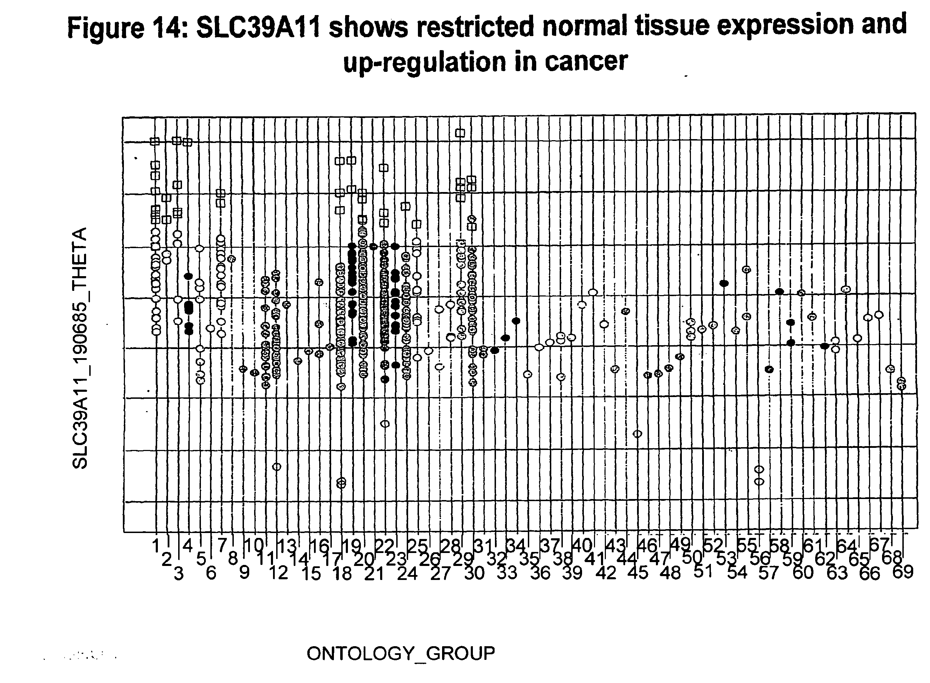

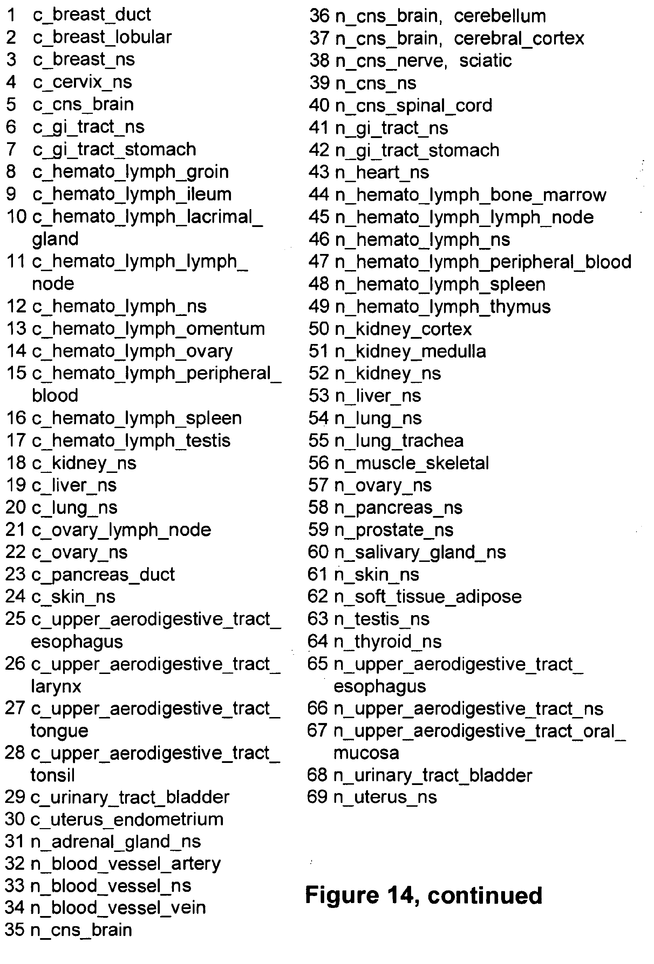

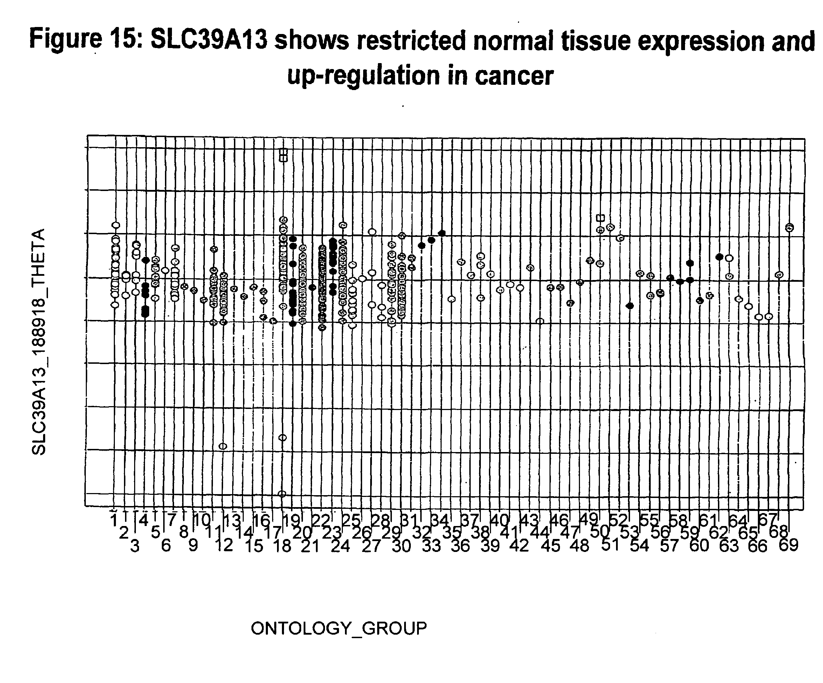

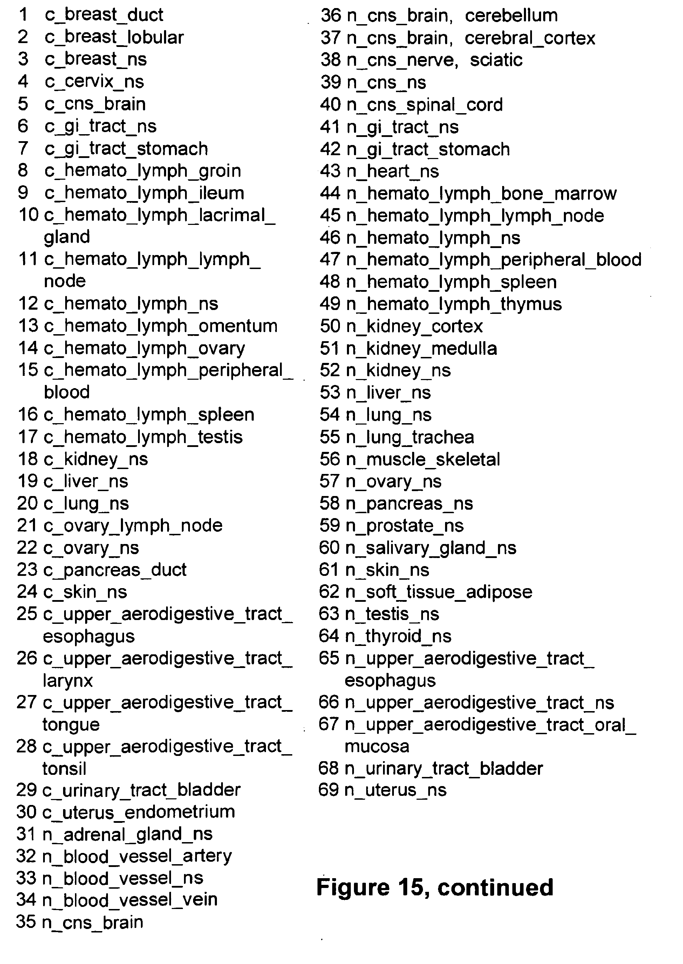

Microarray data was also used to determine expression of LIV-1, SLC39A10, SLC39A11, and SLC39A13 in a number of cancerous tumor types and a number of tissues from normal samples. DecisionSite software (Spotfire, Somerville, MA) and in-house developed Pipeline Pilot (SciTegic, San Diego, CA, in-house developer Josef Ringgenberg) protocols were used to generate the graphical depictions shown in Figures 12-15. The results show elevated expression for each of the tumor cell antigens of interest in a number of cancer types with a lower level of expression in a variety of normal tissue types.

**Example 14: Sequences**

LIV-1 nucleotide sequence; SEQ ID NO:1:

CTCGTGCCGAATTCGGCACGAGACCGCGTGTTCGCGCCTGGTAGAGATTTCTCGAAGACACCAGTGGCCCGTGT
GGAACCAAACCTGCGCGCGTGGCCGGCCGTGGACAACGAGGCCGCGGAGACGAAGGCGCAATGGCGAGGAAGT
TATCTGTAATCTTGATCCTGACCTTTGCCCTCTCTGTCACAAATCCCCTTCATGAACTAAAGCAGCTGCTTTCC
CCCAGACCACTGAGAAAATTAGTCCGAATTGGAATCTGGCATTAATGTTGACTTGGCAATTTCCACACGGCAAT
ATCATCTACAACAGCTTTTCTACCGCTATGGAGAAAATAATTCTTTGTCAGTTGAAGGTTCAGAAAATTACTTC
AAAATATAGGCATAGATAAGATTAAAAGAATCCATATACACCATGACCACGACCATCACTCAGACCACGAGCATC
ACTCAGACCATGAGCGTCACTCAGACCATGAGCATCACTCAGACCACGAGCATCACTCTGACCATGATCATCACT
CTCACCATAATCATGCTGCTTCTGGTAAAAATAAGCGAAAAGCTCTTTGCCCAGACCATGACTCAGATAGTTCAG
GTAAAGATCCTAGAAACAGCCAGGGGAAAGGAGCTCACCGACCAGAACATGCCAGTGGTAGAAGGAATGTCAAGG
ACAGTGTTAGTGCTAGTGAAGTGACCTCAACTGTGTACAACACTGTCTCTGAAGGAACTCACTTTCTAGAGACAA
TAGAGACTCCAAGACCTGGAAAACTCTTCCCCAAAGATGTAAGCAGCTCCACTCCACCCAGTGTCACATCAAAGA
GCCGGGTGAGCCGGCTGGCTGGTAGGAAAACAAATGAATCTGTGAGTGAGCCCCGAAAAGGCTTTATGTATTCCA
GAAACACAAATGAAAATCCTCAGGAGTGTTTCAATGCATCAAAGCTACTGACATCTCATGGCATGGGCATCCAGG
TTCCGCTGAATGCAACAGAGTTCAACTATCTCTGTCCAGCCATCATCAACCAAATTGATGCTAGATCTTGTCTGA
TTCATACAAGTGAAAAGAAGGCTGAAATCCCTCCAAAGACCTATTCATTACAAATAGCCTGGGTTGGTGGTTTTA
TAGCCATTTCCATCATCAGTTTCCTGTCTCTGCTGGGGGTTATCTTAGTGCCTCTCATGAATCGGGTGTTTTTCA
AATTTCTCCTGAGTTTCCTTGTGGCACTGGCCGTTGGGACTTTGAGTGGTGATGCTTTTTTACACCTTCTTCCAC
ATTCTCATGCAAGTCACCACCATAGTCATAGCCATGAAGAACCAGCAATGGAAATGAAAAGAGGACCACTTTTCA
GTCATCTGTCTTCTCAAAACATAGAAGAAAGTGCCTATTTTGATTCCACGTGGAAGGGTCTAACAGCTCTAGGAG
GCCTGTATTTCATGTTTCTTGTTGAACATGTCCTCACATTGATCAAACAATTTAAAGATAAGAAGAAAAAGAATC
AGAAGAAACCTGAAAATGATGATGATGTGGAGATTAAGAAGCAGTTGTCCAAGTATGAATCTCAACTTTCAACAA
ATGAGGAGAAAGTAGATACAGATGATCGAACTGAAGGCTATTTACGAGCAGACTCACAAGAGCCCTCCCACTTTG
ATTCTCAGCAGCCTGCAGTCTTGGAAGAAGAAGAGGTCATGATAGCTCATGCTCATCCACAGGAAGTCTACAATG
AATATGTACCCAGAGGGTGCAAGAATAAATGCCATTCACATTTCCACGATACACTCGGCCAGTCAGACGATCTCA
TTCACCACCATCATGACTACCATCATATTCTCCATCATCACCACCACCAAAACCACCATCCTCACAGTCACAGCC
AGCGCTACTCTCGGGAGGAGCTGAAAGATGCCGGCGTCGCCACTTTGGCCTGGATGGTGATAATGGGTGATGGCC
TGCACAATTTCAGCGATGGCCTAGCAATTGGTGCTGCTTTTACTGAAGGCTTATCAAGTGGTTTAAGTACTTCTG

TTGCTGTGTTCTGTCATGAGTTGCCTCATGAATTAGGTGACTTTGCTGTTCTACTAAAGGCTGGCATGACCGTTA
AGCAGGCTGTCCTTTATAATGCATTGTCAGCCATGCTGGCGTATCTTGGAATGGCAACAGGAATTTTCATTGGTC
ATTATGCTGAAAATGTTTCTATGTGGATATTTGCACTTACTGCTGGCTTATTCATGTATGTTGCTCTGGTTGATA
TGGTACCTGAAATGCTGCACAATGATGCTAGTGACCATGGATGTAGCCGCTGGGGGTATTTCTTTTTACAGAATG
CTGGGATGCTTTTGGGTTTTGGAATTATGTTACTTATTTCCATATTTGAACATAAAATCGTGTTTCGTATAAATT
TCTAGTTAAGGTTTAAATGCTAGAGTAGCTTAAAAAGTTGTCATAGTTTCAGTAGGTCATAGGGAGATGAGTTTG
TATGCTGTACTATGCAGCGTTTAAAGTTAGTGGGTTTTGTGATTTTTGTATTGAATATTGCTGTCTGTTACAAAG
TCAGTTAAAGGTACGTTTTAATATTTAAGTTATTCTATCTTGGAGATAAAATCTGTATGTGCAATTCACCGGTAT
TACCAGTTTATTATGTAAACAAGAGATTTGGCATGACATGTTCTGTATGTTTCAGGGAAAAATGTCTTTAATGCT
TTTTCAAGAACTAACACAGTTATTCCTATACTGGATTTTAGGTCTCTGAAGAACTGCTGGTG

LIV-1 amino acid sequence; SEQ ID NO:2:

MARKLSVILILTFALSVTNPLHELKAAAFPQTTEKISPNWESGINVDLAISTRQYHLQQLFYRYGENNSLSVEGF
RKLLQNIGIDKIKRIHIHHDHDHHSDHEHHSDHERHSDHEHHSDHEHHSDHDHHSHHNHAASGKNKRKALCPDHD
SDSSGKDPRNSQGKGAHRPEHASGRRNVKDSVSASEVTSTVYNTVSEGTHFLETIETPRPGKLFPKDVSSSTPPS
VTSKSRVSRLAGRKTNESVSEPRKGFMYSRNTNENPQECFNASKLLTSHGMGIQVPLNATEFNYLCPAIINQIDA
RSCLIHTSEKKAEIPPKTYSLQIAWVGGFIAISIISFLSLLGVILVPLMNRVFFKFLLSFLVALAVGTLSGDAFL
HLLPHSHASHHHSHSHEEPAMEMKRGPLFSHLSSQNIEESAYFDSTWKGLTALGGLYFMFLVEHVLTLIKQFKDK
KKKNQKKPENDDDVEIKKQLSKYESQLSTNEEKVDTDDRTEGYLRADSQEPSHFDSQQPAVLEEEEVMIAHAHPQ
EVYNEYVPRGCKNKCHSHFHDTLGQSDDLIHHHHDYHHILHHHHHQNHHPHSHSQRYSREELKDAGVATLAWMVI
MGDGLHNFSDGLAIGAAFTEGLSSGLSTSVAVFCHELPHELGDFAVLLKAGMTVKQAVLYNALSAMLAYLGMATG
IFIGHYAENVSMWIFALTAGLFMYVALVDMVPEMLHNDASDHGCSRWGYFFLQNAGMLLGFGIMLLISIFEHKIV
FRINF

In some embodiments of the present invention, LIV-1 modulators comprise or are directed to antigenic regions of the LIV-1 polypeptide. Antigenic regions of LIV-1 include, without limitation, SEQ ID NO:3, SEQ ID NO:4, and SEQ ID NO:5, below:
HHDHDHHSDHEHHSDHERHSDHEHHSDHEHHSDHNHAASGKNKRKALCPDHDSDS (SEQ ID NO:3)
KGAHRPEHASGRRNVKDSVSASE (SEQ ID NO:4)
HLLPHSHASHHHSHSHEEPA (SEQ ID NO: 5)

In some embodiments of the present invention, LIV-1 modulators comprise and/or specifically bind to one or more sequences of SEQ ID NO:2. In some embodiments, LIV-1 modulators specifically bind to one or more LIV-1 epitopes having a sequence selected from the group consisting of SEQ ID NO:361-SEQ ID NO:364 or SEQ ID NO:387-SEQ ID NO:391, below;
HSHSHEEPAMEMKRGPLFSH; SEQ ID NO:361
CFNASKLLSHGM; SEQ ID NO: 362
GMGIQVPLNATEFNYL; SEQ ID NO:363
SVSASEVTSTVYNTVSEGT; SEQ ID NO:364 
LHELKAAAFPQTTEKISPNWESGINVDLAISTRQYHLQQLFYRYGENNSLSVEGFRKLLQNIGIDKIKRIHIHHD
HDHHSDHEHHSDHERHSDHEHHSDHEHHSDHNHAASGKNKRKALCPDHDSDSSGKDPRNSQGKGAHRPEHASGRR
NVKDSVSASEVTSTVYNTVSEGTHFLETIETPRPGKLFPKDVSSSTPPSVTSKSRVSRLAGRKTNESVSEPRKGF
MYSRNTNENPQECFNASKLLTSHGMGIQVPLNATEFNYLCPAIINQIDARSCLIHTSEKKAEIPPKTYSLQIAWV
GGFI; SEQ ID NO:387; N-terminus;
 HLLPHSHASHHHSHSHEEPAMEMKRGPLFSHLSSQNIEESAYFDST; SEQ ID NO:388; TM 2/3
TEGLSS; SEQ ID NO:389; TM 4/5
HYAENVSM; SEQ ID NO:390; TM 6/7
VFRINF; SEQ ID NO:391; C-terminus

In some embodiments LIV-1 modulators of the present invention have the following sequences:
CHIR296-4SI; AAAGGCTGGCATGACCGTTAA (SEQ ID NO:367)
CHIR296-3SI; GACAGTGTTAGTGCTAGTGAA (SEQ ID NO:368)
CHIR296-2SI; CTAGTTAAGGTTTAAATGCTA (SEQ ID NO:369)
CHIR296-1SI; AAGGCTTATCAAGTGGTTTAA (SEQ ID NO:370)
CHIR296-7RC; GCAACCCTGAAACTCACCACTACGA (SEQ ID NO:371)
CHIR296-5RC; TACCGTACCCGTAGGTCCAAGGCG (SEQ ID NO:372)
CHIR296-2RC; TGTGGTACTGGTGCTGGTAGTGAGT (SEQ ID NO:373)
CHIR296-9; TGGTGAATGAGATCGTCTGACTGGC (SEQ ID NO:374)
CHIR296-8; AGGGCTCTTGTGAGTCTGCTCGTAA (SEQ ID NO:375)
CHIR296-7; AGCATCACCACTCAAAGTCCCAACG (SEQ ID NO:376)
CHIR296-6;GCCAGTGCCACAAGGAAACTCAGG (SEQ ID NO:377)
CHIR296-5; GGGGAACCTGGATGCCCATGCCAT (SEQ ID NO:378)
CHIR296-4; ACTGGCATGTTCTGGTCGGTGAGC (SEQ ID NO:379)
CHIR296-3; ATGCTCATGGTCTGAGTGACGCTCA (SEQ ID NO:380)
CHIR296-2; TGAGTGATGGTCGTGGTCATGGTGT (SEQ ID NO:381)
CHIR296-1; GAGAGGGCAAAGGTCGGATCAAGA (SEQ ID NO:382).

SLC39A10; SEQ ID NO:383; NP_065075.1

MKVHMHTKFCLICLLTFIFHHCNHCHEEHDHGPEALHRQHRGMTELEPSKFSKQAAENEKKYY
IEKLFERYGENGRLSFFGLEKLLTNLGLGERKVVEINHEDLGHDHVSHLDILAVQEGKHFHSH
NHQHSHNHLNSENQTVTSVSTKRNHKCDPEKETVEVSVKSDDKHMHDHNHRLRHHHRLHHHLD
HNNTHHFHNDSITPSERGEPSNEPSTETNKTQEQSDVKLPKGKRKKKGRKSNENSEVITPGFP
PNHDQGEQYEHNRVHKPDRVHNPGHSHVHLPERNGHDPGRGHQDLDPDNEGELRHTRKREAPH
VKNNAIISLRKDLNEDDHHHECLNVTQLLKYYGHGANSPISTDLFTYLCPALLYQIDSRLCIE
HFDKLLVEDINKDKNLVPEDEANIGASAWICGIISITVISLLSLLGVILVPIINQGCFKFLLT
FLVALAVGTMSGDALLHLLPHSQGGHDHSHQAHGHGHSHGHESNKFLEEYDAVLKGLVALGG
IYLLFIIEHCIRMFKHYKQQRGKQKWFMKQNTEESTIGRKLSDHKLNNTPDSDWLQLKPLAGT
DDSVVSEDRLNETELTDLEGQQESPPKNYLCIEEEKIIDHSHSDGLHTIHEHDLHAAAHNHHG
ENKTVLRKHNHQWHHKHSHHSHGPCHSGSDLKETGIANIAWMVIMGDGIHNFSDGLAIGAAFS
AGLTGGISTSIAVFCHELPHELGDFAVLLKAGMTVKQAIVYNLLSAMMAYIGMLIGTAVGQYA
NNITLWIFAVTAGMFLYVALVDMLPEMLHGDGDNEEHGFCPVGQFILQNLGLLFGFAIMLVIA
LYEDKIVFDIQF

SLC39A11; SEQ ID NO:384; NP_631916

MLQGHSSVFQALLGTFFTWGMTAAGAALVFVFSSGQRRILDGSLGFAAGVMLAASYWSLLAPAVEMATSSGGFGA
FAFFPVAVGFTLGAAFVYLADLLMPHLGAAEDPQTALALNFGSTLMKKKSDPEGPALLFPESELSIRIDKSENGE
AYQRKKAAATGLPEGPAVPVPSRGNLAQPGGSSWRRIALLILAITIHNVPEGLAVGVGFGAIEKTASATFESARN
LAIGIGIQNFPEGLAVSLPLRGAGFSTWRAFWYGQLSGMVEPLAGVFGAFAVVLAEPILPYALAFAAGAMVYVVM
DDIIPEAQISGNGKLASWASILGFVVMMSLDVGLG

SLC39A13; SEQ ID NO:385; NP_689477.2

MPGCPCPGCGMAGPRLLFLTALALELLGRAGGSQPALRSRGTATACRLDNKESESWGALLSGE
RLDTWICSLLGSLMVGLSGVFPLLVIPLEMGTMLRSEAGAWRLKQLLSFALGGLLGNVFLHLL
PEAWAYTCSASPGGEGQSLQQQQQLGLWVIAGILTFLALEKMFLDSKEEGTSQAPNKDPTAAA
AALNGGHCLAQPAAEPGLGAVVRSIKVSGYLNLLANTIDNFTHGLAVAASFLVSKKIGLLTTM
AILLHEIPHEVGDFAILLRAGFDRWSAAKLQLSTALGGLLGAGFAICTQSPKGVEETAAWVLP
FTSGGFLYIALVNVLPDLLEEEDPWRSLQQLLLLCAGIVVMVLFSLFVD

SEQ ID NO:386

LIV-1-delta; SEQ ID NO:365

MGIQVPLNATEFNYLCPAIINQIDARSCLIHTSEKKAEIPPKTYSLQIAWVGGFIAISIISFL
SLLGVILVPLMNRVFFKFLLSFLVALAVGTLSGDAFLHLLPHSHASHHHSHSHEEPAMEMKRG
PLFSHLSSQNIEESAYFDSTWKGLTALGGLYFMFLVEHVLTLIKQFKDKKKKNQKKPENDDDV
EIKKQLSKYESQLSTNEEKVDTDDRTEGYLRADSQEPSHFDSQQPAVLEEEEVMIAHAHPQEV
YNEYVPRGCKNKCHSHFHDTLGQSDDLIHHHHDYHHILHHHHQNHHPHSHSQRYSREELKDA
GVATLAWMVIMGDGLHNFSDGLAIGAAFTEGLSSGLSTSVAVFCHELPHELGDFAVLLKAGMT
VKQAVLYNALSAMLAYLGMATGIFIGHYAENVSMWIFALTAGLFMYVALVDMVSF

SEQ ID NO:366

MARKLSVILILTFALSVTNPLHELKAAAFPQTTEKISPNWESGINVDLAISTRQYHLQQLFYR
YGENNSLSVEGFRKLLQNIGIDKIKRIHIHHDHDHHSDHEHHSDHERHSDHEHHSDHEHHSDH
DHHSHHNHAASGKNKRKALCPDHDSDSSGKDPRNSQGKGAHRPEHASGRRNVKDSVSASEVTS
TVYNTVSEGTHFLETIETPRPGKLFPKDVSSSTPPSVTSKSRVSRLAGRKTNESVSEPRKGFM
YSRNTNENPQECFNASKLLTSHGMGIQVPLNATEFNYLCPAIINQIDARSCLIHTSEKKAEIP
PKTYSLQIAWVGGFIAISIISFLSLLGVILVPLMNRVFFKFLLSFLVALAVGTLSGDAFLHLL

PHSHASHHHSHSHEEPAMEMKRGPLFSHLSSQNIEESAYFDSTWKGLTALGGLYFMFLVEHVL
TLIKQFKDKKKKNQKKPENDDDVEIKKQLSKYESQESTNEEKVDTDDRTEGYLRADSQEPSHF
DSQQPAVLEEEEVMIAHAHPQEVYNEYVPRGCKNKCHSHFHDTLGQSDDLIHHHHDYHHILHH
HHHQNHHPHSHSQRYSREELKDAGVATLAWMVIMGDGLHNFSDGLAIGAAFTEGLSSGLSTSV
AVFCHELPHELGDFAVLLKADMTVKQAVLYNALSAMLAYLGMATGIFIGHYAENVSMWIFALT
AGLFMYVALVDMVPEMLHNDASDHGCSRWGYFFLQNAGMLLGFGIMLLISIFEHKIVFRINF

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the present invention.

**The following represent further embodiments of the present invention:**
1. A composition comprising a LIV-1 modulator and one or more pharmaceutically acceptable carriers, wherein the LIV-1 modulator is selected from the group consisting of: an isolated double-stranded RNA (dsRNA); an isolated oligonucleotide comprising at least 10 consecutive nucleotides of a sequence of SEQ ID NO:1; and an antibody that binds an epitope in a domain of LIV-1 selected from the group consisting of the N-terminal extracellular domain of LIV-1, the extracellular domain of LIV-1 between transmembrane domains (TM) 2&3, the extracellular domain of LIV-1 between TM 4&5, the extracellular domain of LIV-1 I between TM 6&7, and the C-terminal extracellular domain of LIV-1.
2. The composition of embodiment 1 wherein the oligonucleotide is a dsRNA, a siRNA, a shRNA or an antisense oligonucleotide.
3. The composition of embodiment 1 having one or more activities selected from the group consisting of increasing cancer cell apoptosis, inhibiting cancer cell growth, inhibiting tumor formation, inhibiting cancer cell survival, inhibiting cancer cell proliferation, inhibiting cancer cell metastasis, inhibiting cell migration, inhibiting angiogenesis, inhibiting LIV-1 signaling, inhibiting LIV-1-mediated cell-cell adhesion, inhibiting LIV-1-mediated cell-cell membrane interaction, inhibiting LIV-1-mediated cell-extracellular matrix interaction, inhibiting LIV-1-mediated cell-extracellular matrix degradation, and inhibiting LIV-1 expression.
4. The composition of embodiment 1 wherein the composition is a sterile injectable.
5. The composition of embodiment 1 wherein the LIV-1 modulator inhibits one or more of cancer cell growth, cancer cell survival, tumor formation, and cancer cell proliferation.
6. The composition of embodiment 1 wherein said LIV-1 modulator inhibits one or more of cyclin D1, fibronectin, RhoB, MT1-MMP, FGF, CDK4, VEGF, EGFR, and EGFR phosphorylation.
7. The composition of embodiment 1 wherein said LIV-1 modulator modulates integrin mediated activities.
8. The composition of embodiment 1 wherein the LIV-1 modulator inhibits one or more genes in the SNAIL pathway.
9. The composition of embodiment 1 wherein the LIV-1 modulator inhibits Snail nuclear localization.
10. The composition of embodiment 1 wherein said LIV-1 modulator up-regulates one or more of E-cadherin, VE-cadherin, Muc-1.
11. The composition of embodiment 1 wherein said LIV-1 modulator up-regulates one or more of claudin, occludin, desmoplakin, caspase, p21, p53, BID (bcl-interacting death agonist), DFF40 (DNA fragmentation factor), and cytokeratin.
12. The composition of embodiment 1 wherein the LIV-1 modulator inhibits zinc transport
13. The composition of embodiment 1 wherein the LIV-1 modulator reduces cytoplasmic zinc levels.
14. The composition of embodiment 1 wherein the LIV-1 modulator is a monoclonal antibody which binds to a LIV-1 polypeptide with an affinity of at least 1x10⁸Ka.
15. The composition of embodiment 14 wherein the LIV-1 polypeptide has a sequence of SEQ ID NO:2.
16. The composition of embodiment 14 wherein the monoclonal antibody modulates one or more LIV-1 related biological activities.
17. The composition of embodiment 14 wherein the monoclonal antibody inhibits one or more of cancer cell growth, cancer cell survival, tumor formation, and cancer cell proliferation.
18. The composition of embodiment 1 wherein the monoclonal antibody is a monoclonal antibody, a polyclonal antibody, a chimeric antibody, a human antibody, a humanized antibody, a single-chain antibody, a bi-specific antibody, a multi-specific antibody, or a Fab fragment.
19. The composition of embodiment 14 wherein the monoclonal antibody binds to one or more epitopes of LIV-1, said epitope having a sequence selected from the group consisting of SEQ ID NOS:3-364 and 388-391.
20. The composition of embodiment 14 wherein the monoclonal antibody binds to an LIV-1 epitope in the extracellular domain of LIV-1 between TM 2&3.
21. The composition of embodiment 19 wherein the monoclonal antibody binds to one or more epitopes of SEQ ID NO:388.
22. The composition of embodiment 14 wherein the monoclonal antibody binds to an LIV-1 epitope in the N-terminal extracellular domain of LIV-1.
23. The composition of embodiment 22 wherein the monoclonal antibody binds to one or more epitopes of SEQ ID NO:387.
24. The composition of embodiment 1 wherein the LIV-1 modulator is an oligonucleotide having a sequence selected from the group consisting of SEQ ID NO:367-382.
25. An isolated cell that produces the antibody of embodiment 14.
26. A hybridoma that produces the antibody of embodiment 14.
27. A non-human transgenic animal that produces an antibody of embodiment 14.
28. An isolated epitope-bearing polypeptide comprising one or more epitopes of SEQ ID NO:2, said epitopes selected from the group consisting of SEQ ID NOS:6-364 and 387-391.
29. An isolated epitope-bearing polypeptide of embodiment 28 further limited by a proviso that the polypeptide does not compise the entire amino acid sequence of SEQ ID NO:2.
30. A polynucleotide that encodes an isolated epitope-bearing polypeptide of embodiment 28.
31. The epitope-bearing polypeptide of embodiment 28, wherein each of said epitopes consists of between about 6 and about 20 contiguous amino acids of the epitope selected from the group consisting of SEQ ID NOS:6-364 and 387-391.
32. The epitope-bearing polypeptide of embodiment 28, which comprises at least two epitopes of SEQ ID NO:2 and wherein each of said epitopes consists of between about 6 and 20 contiguous amino acids of the epitope selected from the group consisting of SEQ ID NOS:6-364 and 387-391.
33. The epitope-bearing polypeptide of embodiment 28, wherein at least one of said epitopes consists of at least 21 contiguous amino acids of SEQ ID NO:2.
34. An isolated LIV-1 antibody which is obtained by immunization of a subject with the epitope-bearing polypeptide of embodiment 28.
35. A method of treating cancer or a cancer symptom in a patient in need thereof comprising administering to the patient a therapeutically effective amount of the LIV-1 modulator of embodiment 1.
36. The method of embodiment 35 wherein the LIV-1 modulator inhibits growth of cancer cells that express LIV-1 by at least 25% in an *in vitro* assay to measure cell growth.
37. The method of embodiment 35 wherein the LIV-1 modulator is effective at a concentration of less than about 1 mM to induce apoptosis in at least 25% of contacted cells in an *in vitro* assay to measure apoptosis.
38. The method of embodiment 35 wherein the LIV-1 modulator reduces cytoplasmic zinc levels by at least 25% as compared to a control.
39. The method of embodiment 35 wherein said LIV-1 modulator inhibits one or more of cyclin D1, fibronectin, RhoB, MT1-MMP, FGF, CDK4, VEGF, EGFR, and EGFR phosphorylation.
40. The method of embodiment 35 wherein the LIV-1 modulator inhibits LIV-1 expression by at least 25% as compared to a control.
41. The method of embodiment 35 wherein the LIV-1 modulator is a siRNA, a shRNA, or an antisense oligonucleotide.
42. The method of embodiment 41 wherein the LIV-1 modulator is an oligonucleotide having a sequence selected from the group consisting of SEQ ID NO: 367-382.
43. The method of embodiment 35 wherein the LIV-1 modulator is a monoclonal antibody.
44. The method of embodiment 43 wherein the monoclonal antibody binds one or more epitopes, said epitope having a sequence selected from the group consisting of SEQ ID NOS:3-364 and 388-391.
45. The method of embodiment 43 wherein the monoclonal antibody binds to an LIV-1 epitope in the extracellular domain of LIV-1 between transmembrane domains (TM) 2&3.
46. The method of embodiment 43 wherein the monoclonal antibody binds to one or more epitopes of SEQ ID NO:388.
47. The method of embodiment 43 wherein the monoclonal antibody binds to an LIV-1 epitope in the N-terminal extracellular domain of LIV-1.
48. The method of embodiment 43 wherein the monoclonal antibody binds to one or more epitopes of SEQ ID NO:387.
49. The method of embodiment 35 wherein the cancer is breast cancer, skin cancer, esophageal cancer, liver cancer, pancreatic cancer, prostatic cancer, uterine cancer, cervical cancer, lung cancer, bladder cancer, ovarian cancer, multiple myeloma and melanoma.
50. The method of embodiment 35 wherein the cancer is in a non-hormonally regulated tissue.
51. The method of embodiment 49 wherein the breast cancer is estrogen receptor positive breast cancer, estrogen receptor negative breast cancer, and metastatic breast cancer.
52. The method of embodiment 49 wherein the breast cancer is selected from the group consisting of ductal adenocarcinoma, lobular adenocarcinoma, and metastatic adenocarcinoma.
53. The method of embodiment 35 wherein the cancer is squamous cell carcinoma.
54. The method of embodiment 35 further comprising the administration of a conventional cancer therapeutic to the patient.
55. The method of embodiment 35 further comprising the treatment of the patient with one or more of chemotherapy, radiation therapy or surgery.
56. The method of embodiment 35 wherein the cancer symptom is selected from the group consisting of breast lumps, nipple changes, breast cysts, breast pain, death, weight loss, weakness, excessive fatigue, difficulty eating, loss of appetite, chronic cough, worsening breathlessness, coughing up blood, blood in the urine, blood in stool, nausea, vomiting, liver metastases, lung metastases, bone metastases, abdominal fullness, bloating, fluid in peritoneal cavity, vaginal bleeding, constipation, abdominal distension, perforation of colon, acute peritonitis, pain, vomiting blood, heavy sweating, fever, high blood pressure, anemia, diarrhea, jaundice, dizziness, chills, muscle spasms, colon metastases, lung metastases, bladder metastases, liver metastases, bone metastases, kidney metastases, and pancreas metastases, difficulty swallowing, and the like.
57. A method of modulating a LIV-1-related biological activity in a patient, the method comprising administering to the patient an amount of the LIV-1 modulator of embodiment 1 effective to modulate the LIV-1-related biological activity.
58. The method of embodiment 57 wherein the LIV-1 modulator is a monoclonal antibody which selectively binds to LIV-1.
59. The method of embodiment 57 wherein the patient has or is predisposed to one or more of breast cancer, skin cancer, esophageal cancer, liver cancer, pancreatic cancer, prostatic cancer, uterine cancer, cervical cancer, lung cancer, bladder cancer, ovarian cancer, multiple myeloma and melanoma.
60. The method of embodiment 35 wherein the LIV-1 modulator is an antibody and is administered to the subject via *in vivo* therapeutic antibody gene transfer.
61. A method of identifying a patient susceptible to LIV-1 therapy comprising:
   (a) detecting the presence or absence of LIV-1 differential expression in a patient sample, wherein the presence of LIV-1 differential expression in said sample is indicative of a patient who is a candidate for LIV-1 therapy and the absence LIV-1 differential expression in said sample is indicative of a patient who is not a candidate for LIV-1 therapy;
   (b) administering a therapeutically effective amount of the composition of embodiment 1 to the patient if the patient is a candidate for LIV-1 therapy; and
   c) administering a conventional cancer therapeutic to the patient if the patient is not a candidate for LIV-1 therapy.
62. The method of embodiment 61 wherein the expression of LIV-1 in the patient is increased at least 50% compared to a control.
63. The method of embodiment 61 wherein LIV-1 differential expression is detected by measuring LIV-1 RNA.
64. The method of embodiment 61 wherein LIV-1 differential expression is detected by measuring LIV-1 expression products.
65. The method of embodiment 61 further comprising the administration of a conventional cancer therapeutic to the patient.
66. A method of inhibiting growth of cancer cells that express LIV-1 comprising contacting an amount of the LIV-1 modulator of embodiment 1 effective to inhibit growth of the cells with the cells.
67. The method of embodiment 66 wherein the LIV-1 modulator reduces cytoplasmic zinc levels by at least 25% as compared to a control.
68. The method of embodiment 66 wherein the LIV-1 modulator reduces cyclin D1 levels by at least 25% as compared to a control.
69. A method of inhibiting a cancer cell phenotype in a population of cells expressing LIV-1, said method comprising administering to said population an amount of the LIV-1 modulator of embodiment 1 effective to inhibit the cancer cell phenotype.
70. The method of embodiment 69 wherein the cancer cells are selected from the group consisting of breast cancer, skin cancer, esophageal cancer, liver cancer, pancreatic cancer, prostatic cancer, uterine cancer, cervical cancer, lung cancer, bladder cancer, ovarian cancer, multiple myeloma and melanoma.
71. A method for detecting one or more cancer cells expressing LIV-1 in a sample comprising contacting the sample with a composition comprising the LIV-1 modulator of embodiment 1 linked to an imaging agent and detecting the localization of the imaging agent in the sample.
72. The method of embodiment 71 wherein the composition comprises an LIV-1 antibody conjugated to an imaging agent.
73. The method of embodiment 72 wherein the imaging agent is ¹⁸F, ⁴³K, ⁵²Fe , ⁵⁷Co, ⁶⁷Cu, ⁶⁷Ga , ⁷⁷Br, ⁸⁷MSr, ⁸⁶Y, ⁹⁰Y, ⁹⁹MTc, ¹¹¹In, ¹²³I, ¹²⁵I, ¹²⁷Cs, ¹²⁹CS, ¹³¹I, ¹³²I ¹⁹⁷Hg, ²⁰³Pb, or ²⁰⁶Bi_{.}
74. A method for inhibiting the interaction of two or more cells, wherein at least one of said cells expresses LIV-1, comprising administering an effective amount of the LIV-1 modulator of embodiment 1 to a sample comprising the cells.
75. The method of embodiment 74 wherein the interaction is *in vivo.*
76. The method of embodiment 74 wherein the LIV-1 modulator is a monoclonal antibody which reduces cytoplasmic zinc levels by at least 25% as compared to a control.
77. The method of embodiment 74 wherein the LIV-1 modulator is a monoclonal antibody which reduces cyclin D1 levels by at least 25% as compared to a control.
78. A method of expressing an anti-LIV-1 antibody in a CHO or myeloma cell wherein the anti-LIV-1 antibody inhibits one or more LIV-1-related biological activities, the method comprising expressing a nucleic acid encoding the anti-LIV-1 antibody in said CHO or myeloma cell.
79. A method of identifying a cancer inhibitor, said cancer characterized by overexpression of LIV-1 compared to a control, said method comprising contacting a cell expressing LIV-1 with a candidate compound and a LIV-1 ligand, and determining whether a LIV-1-related zinc transport activity is inhibited, wherein inhibition of the LIV-1-related zinc transport activity is indicative of a cancer inhibitor.
80. A method of identifying a cancer inhibitor, said cancer characterized by overexpression of LIV-1 compared to a control, said method comprising contacting a cell expressing LIV-1 with a candidate compound and a LIV-1 ligand, and determining whether a downstream marker of LIV-1 is inhibited, wherein inhibition of the downstream marker is indicative of a cancer inhibitor.
81. The method of embodiment 80 wherein the downstream marker is cyclin D1 or cytoplasmic zinc levels.
82. A method for determining the susceptibility of a patient to a LIV-1 modulator comprising detecting evidence of differential expression of LIV-1 in said patient's cancer sample, wherein evidence of differential expression of LIV-1 is indicative of the patient's susceptibility to said LIV-1 modulator.
83. The method of embodiment 82 wherein said evidence of differential expression of LIV-1 is upregulation of LIV-1 in said patient's cancer sample.
84. A method of purifying LIV-1 protein from a sample comprising LIV-1 protein comprising:
   a) providing an affinity matrix comprising the antibody of embodiment 1 bound to a solid support;
   b) contacting the sample with the affinity matrix to form an affinity matrix-LIV-1 protein complex;
   c) separating the affinity matrix-LIV-1 protein complex from the remainder of the sample; and
   d) releasing LIV-1 protein from the affinity matrix.
85. A method of delivering a cytotoxic agent or a diagnostic agent to one or more cells that express LIV-1, said method comprising:
   a) providing the cytotoxic agent or the diagnostic agent conjugated to an antibody or fragment thereof of embodiment 1; and,
   b) exposing the cell to the antibody-agent or fragment-agent conjugate.
86. A method for determining the prognosis of a cancer patient comprising determining the ratio of LIV-1-delta to LIV-1 in a sample of said patient, wherein the ratio of LIV-1-delta to LIV-1 is used to determine the prognosis of the cancer patient.
87. A method for determining the prognosis of a cancer patient comprising the presence or absence of LIV-1 bound to the plasma membrane of a cell in a sample of said patient, wherein the absence of LIV-1 bound to the plasma membrane of a cell in a sample of said patient indicates a good prognosis for the patient.
88. The method of embodiment 86 or 87 wherein the LIV-1 is encoded for by a nucleic acid having a sequence of SEQ ID NO:1.
89. The method of embodiment 86 or 87 wherein the LIV-1 has a sequence of SEQ ID NO:2.
90. The method of embodiment 86 wherein the LIV-1-delta has a sequence of SEQ ID NO:365.
91. The method of embodiment 86 wherein a LIV-1-delta:LIV-1 ratio of at least 2:1 is indicative of a patient with a good prognosis.
92. A composition comprising a zinc transport protein modulator and one or more pharmaceutically acceptable carriers, wherein the zinc transport protein modulator is selected from the group consisting of: an isolated double-stranded RNA (dsRNA); an isolated oligonucleotide comprising at least 10 consecutive nucleotides of a sequence selected from the group consisting of SEQ ID NOS:383-385; and an antibody that binds an epitope in a domain of the zinc transport protein selected from the group consisting of the N-terminal extracellular domain, the extracellular domain between transmembrane domains (TM) 2&3, the extracellular domain between TM 4&5, the extracellular domain between TM 6&7, and the C-terminal extracellular domain.
93. The composition of embodiment 92 wherein the zinc transport protein is SLC39A10, SLC39A11 or SLC39A13.
94. The composition of embodiment 92 wherein said zinc transport protein modulator modulates integrin mediated activities, inhibits zinc transport, or reduces cytoplasmic zinc levels.
95. The composition of embodiment 92 wherein the oligonucleotide is a siRNA or an antisense oligonucleotide.
96. The composition of embodiment 92 wherein the zinc transport protein modulator is a monoclonal antibody which binds to a zinc transport protein with an affinity of at least 1x10⁸Ka_{.}
97. The composition of embodiment 97 wherein the monoclonal antibody binds to one or more epitopes of the N-terminal extracellular domain of the zinc transport protein.
98. The composition of embodiment 97 wherein the monoclonal antibody binds to one or more epitopes in the extracellular domain of the zinc transport protein between transmembrane domains (TM) 2&3.
99. The composition of embodiment 97 wherein the monoclonal antibody inhibits one or more of cancer cell growth, cancer cell survival, tumor formation, and cancer cell proliferation.
100. A method of treating cancer or a cancer symptom in a patient in need thereof comprising administering to the patient a therapeutically effective amount of the zinc transport protein modulator of embodiment 92.
101. The method of embodiment 100 wherein the zinc transport protein modulator has one or more activities selected from the group consisting of increasing cancer cell apoptosis, inhibiting cancer cell growth, inhibiting cancer cell survival, inhibiting tumor formation, inhibiting cancer cell proliferation, inhibiting cancer cell metastasis, inhibiting cell migration, inhibiting angiogenesis, inhibiting LIV-1 signaling, inhibiting LIV-1-mediated cell-cell adhesion, inhibiting LIV-1-mediated cell-cell membrane interaction, inhibiting LIV-1-mediated cell-extracellular matrix interaction, inhibiting LIV-1-mediated cell-extracellular matrix degradation, and inhibiting LIV-1 expression.
102. The method of embodiment 100 wherein the zinc transport protein modulator inhibits cancer cell growth of cancer cells that express the zinc transport protein by at least 30% in an *in vitro* assay to measure cell growth.
103. The method of embodiment 100 wherein the zinc transport protein modulator is effective at a concentration of less than about 1 mM to induce apoptosis in at least 20% of contacted cells in an *in vitro* assay to measure apoptosis.
104. The method of embodiment 100 wherein the zinc transport protein modulator inhibits zinc transport.
105. The method of embodiment 100 wherein the zinc transport protein modulator modulates cytoplasmic zinc levels.
106. The method of embodiment 100 wherein said zinc transport protein modulator modulates integrin mediated activities.
107. The method of embodiment 101 wherein the zinc transport protein modulator inhibits zinc transport protein expression by at least 25% as compared to a control.
108. The method of embodiment 100 wherein the zinc transport protein modulator is a monoclonal antibody.
109. The method of embodiment 108 wherein the antibody has a binding affinity less than about 1x10⁵Ka for a polypeptide other than the zinc transport protein.
110. The method of embodiment 109 wherein the monoclonal antibody induces cell apoptosis.
111. The method of embodiment 109 wherein the monoclonal antibody inhibits tumor formation.
112. A method of modulating a zinc transport protein-related biological activity in a patient, the method comprising administering to the patient an amount of the zinc transport protein modulator of embodiment 92 effective to modulate the zinc transport protein -related biological activity.
113. The method of embodiment 112 wherein the zinc transport protein modulator is a monoclonal antibody which selectively binds to the zinc transport protein.
114. The method of embodiment 100 or 112 wherein the patient has or is predisposed to one or more of breast cancer, skin cancer, esophageal cancer, liver cancer, pancreatic cancer, prostatic cancer, uterine cancer, cervical cancer, lung cancer, bladder cancer, ovarian cancer, multiple myeloma and melanoma.
115. A method of inhibiting growth of cancer cells that express zinc transport protein comprising contacting the cells with an amount of the zinc transport protein modulator of embodiment 92 effective to inhibit growth of the cells.
116. The method of embodiment 115 wherein the zinc transport protein modulator is a monoclonal antibody which selectively binds the zinc transport protein and modulates cytoplasmic zinc levels by at least 30% as compared to a control.
117. A method for detecting one or more cancer cells expressing a zinc transport protein in a sample comprising the sample with a composition comprising the zinc transport protein modulator of embodiment 92 linked to an imaging agent and detecting the localization of the imaging agent in the sample.
118. The method of embodiment 117 wherein the composition comprises a zinc transport protein antibody conjugated to an imaging agent.
119. The method of embodiment 118 wherein the imaging agent is ¹⁸F, ⁴³K, ⁵²Fe, ⁵⁷Co, ⁶⁷Cu, ⁶⁷Ga, ⁷⁷Br, ⁸⁷MSr, ⁸⁶Y, ⁹⁰Y, ⁹⁹MTc, ¹¹¹In, ¹²³I, ¹²⁵I, ¹²⁷Cs, ¹²⁹Cs, ¹³¹I ¹³²I, ¹⁹⁷Hg, ²⁰³Pb, or ²⁰⁶Bi.
120. A method for inhibiting the interaction of two or more cells, wherein at least one of said cells expresses zinc transport protein, comprising administering an effective amount of the zinc transport protein modulator of embodiment 92 to a sample comprising the cells.
121. The method of embodiment 120 wherein the interaction is *in vivo.*
122. A method of identifying a cancer inhibitor, said cancer characterized by overexpression of a zinc transport protein compared to a control, said method comprising contacting a cell expressing a zinc transport protein with a candidate compound and a zinc transport protein ligand, and determining whether zinc transport activity is inhibited, wherein inhibition of the zinc transport activity is indicative of a cancer inhibitor.
123. A method for identifying a cancer inhibitor, said cancer characterized by overexpression of a zinc transport protein compared to a control, said method comprising contacting a cell expressing zinc transport protein with a candidate compound and a zinc transport protein ligand, and determining whether a downstream marker of the zinc transport protein is inhibited, wherein inhibition of the downstream marker is indicative of a cancer inhibitor.
124. A method for determining the susceptibility of a patient to a zinc transport protein modulator comprising detecting evidence of differential expression of zinc transport protein in said patient's cancer sample, wherein evidence of differential expression of zinc transport protein is indicative of the patient's susceptibility to said zinc transport protein modulator.
125. The method of embodiment 124 wherein said evidence of differential expression of the zinc transport protein is upregulation of the zinc transport protein in said patient's cancer sample.
126. A method of purifying a zinc transport protein from a sample comprising one or more zinc transport proteins comprising:
   a) providing an affinity matrix comprising an antibody of embodiment 92 bound to a solid support;
   b) contacting the sample with the affinity matrix to form an affinity matrix-zinc transport protein complex;
   c) separating the affinity matrix-zinc transport protein complex from the remainder of the sample; and
   d) releasing zinc transport protein from the affinity matrix.
127. A method of delivering a cytotoxic agent or a diagnostic agent to one or more cells that express zinc transport protein, said method comprising:
   a) providing the cytotoxic agent or the diagnostic agent conjugated to an antibody or fragment thereof of embodiment 92; and,
   b) exposing the cell to the antibody-agent or fragment-agent conjugate.

## Claims

1. Method for detecting one or more cancer cells expressing LIV-1 in a sample comprising:
a) contacting the sample with a composition comprising an LIV-1 antibody that binds an epitope in the extracellular domain of LIV-1 between transmembrane domains 2 and 3, wherein said antibody is conjugated to an imaging agent; and
b) detecting the localization of the imaging agent in the sample.

2. Method of claim 1, wherein the cancer cells are skin cancer cells.

3. Method of claim 1, wherein the cancer cells are breast cancer cells.

4. Method of claim 3, wherein the breast cancer cells are estrogen receptor positive.

5. Method of claim 3, wherein the breast cancer cells are estrogen receptor negative.

6. Method of claim 4 or 5, wherein the breast cancer cells are ductal adenocarcinoma cells.

7. Method of claim 4 or 5, wherein the breast cancer cells are lobular adenocarcinoma cells.

8. Method of claim 4 or 5, wherein the breast cancer cells are ductal adenocarcinoma and lobular adenocarcinoma.

9. Method of claim 4 or 5, wherein the breast cancer cells are metastatic adenocarcinoma.

10. Method of claims 1-9, wherein the imaging agent is ¹⁸F, ⁴³K, ⁵²Fe, ⁵⁷Co, ⁶⁷Cu, ⁶⁷Ga, ⁷⁷Br ⁸⁷MSr, ⁸⁶Y, ⁹⁰Y, ⁹⁹MTc ¹¹¹In, ¹²³I, ¹²⁵I, ¹²⁷Cs, ¹²⁹Cs, ¹³¹I, ¹³²I, ¹⁹⁷Hg, ²⁰³Pb or ²⁰⁶Bi.

11. LIV-1 antibody that binds an epitope in the extracellular domain of LIV-1 between transmembrane domains 2 and 3 for use in a method of treating cancer or a cancer symptom in a patient in need thereof.

12. Composition comprising a LIV-1 antibody that binds an epitope in the extracellular domain of LIV-1 between transmembrane domains 2 and 3 for use in a method of treating cancer or a cancer symptom in a patient in need thereof.

13. LIV-1 antibody of claim 11 or composition of claim 12, wherein the cancer is breast cancer, skin cancer, esophageal cancer, liver cancer, pancreatic cancer, prostatic cancer, uterine cancer, cervical cancer, lung cancer, bladder cancer, ovarian cancer, multiple myeloma and melanoma.

14. LIV-1 antibody of claim 11 or composition of claim 12, wherein the treatment further comprises the administration of one or more of chemotherapy, radiation therapy or surgery to the patient.
